# EUROPÄISCHE PATENTANMELDUNG

(11) **EP 3 195 865 A1**
(43) Veröffentlichungstag der Anmeldung: **26.07.2017**
(21) Anmeldenummer: 16152499.6
(22) Anmeldetag: 25.01.2016
(51) Int. Cl.: A61K 31/4439, A61K 31/454, A61K 31/496, A61K 31/497, A61K 31/4985, A61K 31/505, A61K 31/506, A61K 31/519, A61K 31/5377, A61K 31/55, C07D 401/12, A61P 35/02, A61P 35/00

(54) **KOMBINATIONEN VON IRAK4 INHIBITOREN UND BTK INHIBITOREN**

(71) Anmelder: Bayer Pharma Aktiengesellschaft, 13353 Berlin (DE)
(72) Erfinder: Bothe, Ulrich, 13187 Berlin (DE); Siebeneicher, Holger, 10557 Berlin (DE); Schmidt, Nicole, San Francisco, 94103 CA (US); Nubbemeyer, Reinhard, 13509 Berlin (DE); Boemer, Ulf, 16548 Glienicke/Nordbahn (DE); Günther, Judith, 13187 Berlin (DE); Steuber, Holger, 10115 Berlin (DE); Lange, Martin, 10115 Berlin (DE); Wengner, Antje Margret, 13189 Berlin (DE); Stegmann, Christian, 10437 Berlin (DE); Sutter, Andreas, 13086 Berlin (DE); Neuhaus, Roland, 12157 Berlin (DE)
(74) Vertreter: BIP Patents

(57) **Zusammenfassung**

Die vorliegende Anmeldung betrifft neue Kombinationen von mindestens zwei Komponenten, Komponente A und Komponente B :
● Komponente A ist eine IRAK4-inhibierende Verbindung der Formel (I) wie hierin definiert, oder ein Diastereomer, ein Enantiomer, ein Metabolit, ein Salz, ein Solvat oder ein Solvat eines Salzes von dieser;
● Komponente B ist eine BTK-inhibierende Verbindung, oder ein pharmazeutisch verträgliches Salz von dieser ;
und, wahlweise,
● einer oder mehreren Komponenten C, die pharmazeutische Mittel sind;
in der gegebenenfalls eine oder beide der oben definierten Komponenten A und B in pharmazeutischen Formulierungen vorliegen, die zur simultanen, separaten oder sequenziellen Verabreichung bereit sind,
zur Behandlung und/oder Prophylaxe von Krankheiten sowie ihre Verwendung zur Herstellung von Arzneimitteln zur Behandlung und/oder Prophylaxe von Krankheiten, insbesondere zur Behandlung und/oder Prophylaxe von Endometriose, Lymphome, Makuladegeneration, COPD, Tumorerkrankungen und Psoriasis.

## Beschreibung

Die vorliegende Erfindung betrifft Kombinationen von mindestens zwei Komponenten, Komponente A und Komponente B:
- Komponente A ist eine IRAK4-inhibierende Verbindung der Formel (I) wie hierin definiert, oder ein Diastereomer, ein Enantiomer, ein Metabolit, ein Salz, ein Solvat oder ein Solvat eines Salzes von dieser;
- Komponente B ist eine BTK-Inhibierende Verbindung;
und, wahlweise,
- eine oder mehrere Komponenten C, die pharmazeutische Mittel sind;
in der gegebenenfalls eine oder beide der oben definierten Komponenten A und B in pharmazeutischen Formulierungen vorliegen, die zur simultanen, separaten oder sequenziellen Verabreichung bereit sind.

Ein weiterer Aspekt der vorliegenden Erfindung betrifft Kombinationen von mindestens zwei Komponenten, Komponente A und Komponente B:
- Komponente A ist eine IRAK4-inhibierende Verbindung der Formel (I) wie hierin definiert, oder ein Diastereomer, ein Enantiomer, ein Metabolit, ein Salz, ein Solvat oder ein Solvat eines Salzes von dieser;
- Komponente B ist eine BTK-inhibierende Verbindung die aus der folgenden Liste ausgewählt ist:
   o Ibrutinib, oder ein pharmazeutisch verträgliches Salz davon ;
   ∘ 4-tert-Butyl-N-[2-methyl-3-(4-methyl-6-{[4-(morpholin-4-ylcarbonyl)phenyl]amino}-5-oxo-4,5-dihydropyrazin-2-yl)phenyl]benzamid (CGI-1746, CAS 910232-84-7) ;
   o N-{3-[(5-Fluor-2-{[4-(2-methoxyethoxy)phenyl]amino}pyrimidin-4-yl)amino]phenyl}acrylamid (AVL-292, CAS 1202757-89-8) ;
   ∘ 6-Cyclopropyl-8-fluor-2-[2-(hydroxymethyl)-3-(1-methyl-5-{[5-(4-methylpiperazin-1-yl)pyridin-2-yl]amino}-6-oxo-1,6-dihydropyridin-3-yl)phenyl]isochinolin-1(2H)-on (RN486, CAS 1242156-23-5);
   o HM71224;
   o N-{3-[6-({4-[(2R)-1,4-Dimethyl-3-oxopiperazin-2-yl]phenyl}amino)-4-methyl-5-oxo-4,5-dihydropyrazin-2-yl]-2-methylphenyl}-4,5,6,7-tetrahydro-1-benzothiophen-2-carboxamid (GDC-0834, CAS 1133432-50-4)
   ∘ 5-Amino-1-[(3R)-1-cyanpiperidin-3-yl]-3-[4-(2,4-difluorphenoxy)phenyl]-1H-pyrazol-4-carboxamid (PF-06250112, J Immunol 2013; 191:4540-4550);
   o (2E)-4-(Dimethylamino)-N-{7-fluor-4-[(2-methylphenyl)amino]imidazo[1,5-a]chinoxalin-8-yl}-N-methylbut-2-enamid (CAS 1345250-62-5, Bioorg. Med. Chem. Lett. 21 (2011) 6258-6262) ;
   o N-[3-(8-Anilinoimidazo[1,2-a]pyrazin-6-yl)phenyl]-4-tert-butylbenzamid (CGI-560, CAS 845269-74-1);
   ∘ 4-{4-[(4-{[3-(Acryloylamino)phenyl]amino}-5-fluorpyrimidin-2-yl)amino]phenoxy}-N-methylpyridin-2-carboxamid (CNX-774, CAS1202759-32-7);
   o ONO-4059 (Athritis and rheumatism 2012, 64 Suppl 10:1660).

Ein weiterer Aspekt der vorliegenden Erfindung betrifft die Verwendung solcher Kombinationen wie hierin definiert zur Herstellung eines Medikaments zur Behandlung oder Vorbeugung einer Krankheit, im Besonderen zur Behandlung von Tumorerkrankungen.

Noch ein weiterer Aspekt der vorliegenden Erfindung betrifft Methoden zur Behandlung oder Vorbeugung von Tumorerkrankungen, bei denen eine therapeutisch effektive Menge einer Kombination wie hierin definiert verabreicht wird.

Weiterhin betrifft die vorliegende Erfindung einen Kit enthaltend Kombinationen von:
- einer oder mehrerer Komponenten A, die aus einer IRAK4-inhibierenden Verbindung der Formel (I) wie hierin definiert bestehen, oder ein Diastereomer, ein Enantiomer, ein Metabolit, ein Salz, ein Solvat oder ein Solvat eines Salzes von dieser;
- einer Komponente B, die eine BTK-inhibierende Verbindung ist, oder ein pharmazeutisch verträgliches Salz von dieser.

Ein weiterer Aspekt der vorliegenden Erfindung betrifft Kombinationen von mindestens zwei Komponenten, Komponente A und Komponente B:
- Komponente A ist eine IRAK4-inhibierende Verbindung der Formel (I) wie hierin definiert, oder ein Diastereomer, ein Enantiomer, ein Metabolit, ein Salz, ein Solvat oder ein Solvat eines Salzes von dieser;
- Komponente B ist eine BTK-inhibierende Verbindung, die aus der folgenden Liste ausgewählt ist:
   ▪ Ibrutinib, oder ein pharmazeutisch verträgliches Salz davon ;
   ▪ 4-tert-Butyl-N-[2-methyl-3-(4-methyl-6-{[4-(morpholin-4-ylcarbonyl)phenyl]amino}-5-oxo-4,5-dihydropyrazin-2-yl)phenyl]benzamid (CGI-1746, CAS 910232-84-7);
   ▪ N-{3-[(5-Fluor-2-{[4-(2-methoxyethoxy)phenyl]amino}pyrimidin-4-yl)amino]phenyl}acrylamid (AVL-292, CAS 1202757-89-8) ;
   ▪ 6-Cyclopropyl-8-fluor-2-[2-(hydroxymethyl)-3-(1-methyl-5-{[5-(4-methylpiperazin-1-yl)pyridin-2-yl]amino}-6-oxo-1,6-dihydropyridin-3-yl)phenyl]isochinolin-1(2H)-on (RN486, CAS 1242156-23-5) ;
   ▪ HM71224;
   ▪ N-{3-[6-({4-[(2R)-1,4-Dimethyl-3-oxopiperazin-2-yl]phenyl}amino)-4-methyl-5-oxo-4,5-dihydropyrazin-2-yl]-2-methylphenyl}-4,5,6,7-tetrahydro-1-benzothiophen-2-carboxamid (GDC-0834, CAS 1133432-50-4) ;
   ▪ 5-Amino-1-[(3R)-1-cyanpiperidin-3-yl]-3-[4-(2,4-difluorphenoxy)phenyl]-1H-pyrazol-4-carboxamid (PF-06250112, J Immunol 2013; 191:4540-4550) ;
   ▪ (2E)-4-(Dimethylamino)-N-{7-fluor-4-[(2-methylphenyl)amino]imidazo[1,5-a]chinoxalin-8-yl}-N-methylbut-2-enamid (CAS 1345250-62-5, Bioorg. Med. Chem. Lett. 21 (2011) 6258-6262) ;
   ▪ N-[3-(8-Anilinoimidazo[1,2-a]pyrazin-6-yl)phenyl]-4-tert-butylbenzamid (CGI-560, CAS 845269-74-1);
   ▪ 4-{4-[(4-{[3-(Acryloylamino)phenyl]amino}-5-fluorpyrimidin-2-yl)amino]phenoxy}-N-methylpyridin-2-carboxamid (CNX-774, CAS1202759-32-7);
   ▪ ONO-4059 (Athritis and rheumatism 2012, 64 Suppl 10:1660).
   und gegebenenfalls

- ein oder mehrere pharmazeutische Mittel;
in denen gegebenenfalls eine oder beide der oben definierten Komponenten A und B in pharmazeutischen Formulierungen vorliegen, die zur simultanen, separaten oder sequenziellen Verabreichung bereit sind.

Die Komponente A kann oral, intravenös, topisch, intraperitoneal, nasal, parenteral, pulmonal, sublingual, lingual, buccal, rectal, dermal, transdermal, conjunctival, über das Ohr oder als Implantat bzw. Stent, oder als Depot verabreicht werden.

Die Komponente B kann oral, intravenös, topisch, intraperitoneal, nasal, parenteral, pulmonal, sublingual, lingual, buccal, rectal, dermal, transdermal, conjunctival, über das Ohr oder als Implantat bzw. Stent, oder als Depot verabreicht werden.

### HINTERGRUND DER ERFINDUNG

### KOMPONENTE A : IRAK4 INHIBITOREN :

Humanes IRAK4 (Interleukin-1 receptor-associated kinase 4) spielt eine Schlüsselrolle bei der Aktivierung des Immunsystems. Deshalb ist diese Kinase ein wichtiges therapeutisches Zielmolekül für die Entwicklung von entzündungshemmenden Substanzen. IRAK4 wird von einer Vielzahl von Zellen exprimiert und vermittelt die Signaltransduktion von Toll-like Rezeptoren (TLR), außer TLR3, sowie Rezeptoren der Interleukin (IL)-1β Familie, bestehend aus dem IL-1R (Rezeptor), IL-18R, IL-33R und IL-36R (Janeway und Medzhitov, Annu. Rev. Immunol., 2002; Dinarello, Annu. Rev. Immunol., 2009; Flannery and Bowie, Biochemical Pharmacology, 2010).
Weder IRAK4 Knockout Mäuse noch humane Zellen von Patienten, denen IRAK4 fehlt, reagieren auf die Stimulation von TLRs (außer TLR3) und der IL-1β Familie (Suzuki, Suzuki, et al., Nature, 2002; Davidson, Currie, et al., The Journal of Immunology, 2006; Ku, von Bernuth, et al., JEM, 2007; Kim, Staschke, et al., JEM, 2007).

Die Bindung der TLR Liganden bzw. der Liganden der IL-1β Familie an den jeweiligen Rezeptor führt zur Rekrutierung und Bindung von MyD88 [Myeloid differentiation primary response gene (88)] an den Rezeptor. Infolge dessen tritt MyD88 mit IRAK4 in Interaktion und es kommt zur Bildung eines aktiven Komplexes, welcher mit den Kinasen IRAK1 oder IRAK2 interagiert und diese aktiviert (Kollewe, Mackensen, et al., Journal of Biological Chemistry, 2004; Precious et al., J. Biol. Chem., 2009). Infolge dessen wird der NF (nuclear factor)-kB Signalweg und der MAPK (Mitogen-activated protein kinase) Signalweg aktiviert (Wang, Deng, et al., Nature, 2001). Die Aktivierung des NF-kB Signalweges als auch des MAPK Signalweges führen zu Prozessen, die mit verschiedenen Immunprozessen assoziiert sind. So kommt es beispielsweise zu einer erhöhten Expression von unterschiedlichen inflammatorischen Signalmolekülen und Enzymen, wie z.B. Zytokine, Chemokine und COX-2 (Cyclooxygenase-2), und zu einer erhöhten mRNA Stabilität von Inflammations-assoziierten Genen wie beispielsweise COX-2, IL-6, IL-8 (Holtmann, Enninga, et al., Journal of Biological Chemistry, 2001; Datta, Novotny, et al., The Journal of Immunology, 2004). Des Weiteren können diese Prozesse mit der Proliferation und der Differenzierung von bestimmten Zelltypen, wie z.B. Monozyten, Makrophagen, Dendritische Zellen, T-Zellen und B-Zellen einhergehen (Wan, Chi, et al., Nat Immunol, 2006; McGettrick and J. O'Neill, British Journal of Haematology, 2007).

Dies gilt auch für einige onkologischen Erkrankungen. Bestimmte Lymphome, wie beispielsweise ABC-DLBCL (aktivierten B-Zellen ähnliches diffuses großzelliges B-Zell-Lymphom), Mantelzelllymphon und Morbus Waldenström, als auch chronisch lymphatische Leukämie, Melanoma und Leberzellkarzinom sind durch Mutationen in MyD88 oder Veränderungen in der MyD88-Aktivität charakterisiert, die durch einen IRAK4 Inhibitor behandelt werden können (Ngo, Young, et al., Nature, 2011; Puente, Pinyol, et al., Nature, 2011; Srivastava, Geng, et al., Cancer Research, 2012; Treon, Xu, et al., New England Journal of Medicine, 2012; Choi, Kim, et al., Human Pathology, 2013; (Liang, Chen, et al., Clinical Cancer Research, 2013). Des Weiteren spielt MyD88 eine wichtige Rolle in Ras-abhängigen Tumoren, so dass IRAK4 Inhibitoren auch zu deren Behandlung geeignet sind (Kfoury, A., K. L. Corf, et al., Journal of the National Cancer Institute, 2013).

Diffuses großzelliges B-Zell-Lymphom (DLBCL) ist ein aggressiver Tumor von B-Lymphozyten und das häufigste Non-Hodgkin Lymphom bei Erwachsenen (Morton LM, et al., Blood 2006). Morphologisch ist das DLBCL in zentroblastische, immunoblastische und anaplastische Lymphome eingeteilt, basierend auf Geneexpression in aktivierten-B-Zellen-ähnlich (ABC-DLBCL) oder keimzentrumsartig (GCB-DLBCL) und genetisch nach PRDM1 Mutationen und BCL2-, BCL6-, MYC-Translokationen unterteilt. Die StandardTherapie für DLBCL ist R-CHOP, eine Kombination der Chemotherapeutika Cyclophosphamide, Doxorubicin, Vincristine und Prednisone (CHOP) und Rituximab, einem chimären monoklonalen CD20-Rezeptor Antikörper (Roschewski M et al., Nature Reviews Clinical Oncology, 2014). Ca. ein Drittel der Erkrankten spricht nicht auf die Standardtherapie an oder erleidet einen Rückfall, was die Notwendigkeit der Entwicklung neuer Therapeutika verdeutlicht (Friedberg, J. W. Hematology Am. Soc. Hematol. Educ. Program 2011). Der ABC-DLBCL Subtyp repräsentiert ca. 30% aller DLBCLs und bedeutet die schlechteste Prognose für Patienten (Siegel, R., et al., CA Cancer J. Clin. 2013). Es wurde gezeigt, dass der für das Überleben von DLBCL Zellen wichtige NF-κB-Signalweg sowohl durch die Aktivierung des B-Zell-Rezeptors (BCR) als auch des Toll-like Rezeptors (TLR) reguliert wird. (Rawlings, D. J., et al. Nat. Rev. Immunol. 2012). In ABC-DLBCL ist der NF-κB-Signalweg oftmals durch Mutationen in diesen beiden Signalwegen konstitutiv aktiviert (Compagno, M. et al. Nature 2009). Aktivierende Mutationen in MYD88, einem Adapter-Protein des TLR-Signalwegs wurden in fast 30% aller ABC-DLBCL gefunden. Diese Mutationen führen zu einer Aktivierung von IRAK4 und anschließender Stimulation des NF-κB-Signalwegs, Interleukin-6/ Interleukin-10-Sekretion sowie der Aktivierung des JAK-STAT-Signalwegs. Eine essentielle Rolle für IRAK4 in der Regulation der Zellviabilität wurde gezeigt (Ngo, VN et al. Nature, 2011). Die aberrante Aktivierung der BCR- und MYD88-Signalwege deutet darauf hin, dass eine Blockade beider Signalwege therapeutisch wirksam sein könnte. Ibrutinib (PCI-32765) ist ein irreversibler Inhibitor von Bruton Tyrosine Kinase (BTK), einer Komponente des BCR-Signalwegs (Winer ES, et al., Expert Opin. Investig. Drugs, 2012). In einer Phase 2 Studie von Ibrutinib in rückfälligen DLBCL Patienten sprachen 40% der Patienten auf die Therapie mit Ibrutinib an, was dafür spricht, das weitere Signalwege in DLBCL relevant sind (Wilson WH et al. ASH Annu Meet Abstr 2012, 120(21):686.). Es wurde gezeigt, dass Kombinationen von Ibrutinib und verschiedenen Inhibitoren des PI3K-Signalwegs sowie Kombinationen von Ibrutinib und Inhibitoren der BCL-2 Familie eine additive bzw. synergistische Wirkung auf die Zellviabilität in ABC-DLBCL haben (Mathews Griner LA et al., Proc Natl Acad Sci U S A. 2014).

Aus dem Stand der Technik sind eine Vielzahl von IRAK4 Inhibitoren bekannt (siehe beispielsweise Annual Reports in Medicinal Chemistry (2014), 49, 117 -133).

US8293923 und US20130274241 offenbaren IRAK4 Inhibitoren mit einer an Position 3 substituierten Indazolstruktur. 2-substituierte Indazole werden nicht beschrieben.

In WO2013106254 und WO2011153588 werden 2,3-disubstituierte Indazolderivate offenbart.

In WO2007091107 werden 2-substituierte Indazolderivate für die Behandlung der Duchenne-Muskeldystrophie beschrieben. Die offenbarten Verbindungen weisen keine 6-Hydroxyalkyl-Substitution auf.

WO2015091426 beschreibt Indazole wie Beispiel 64, die an der Position 2 mit einer Carboxamidseitenkette substituiert sind.

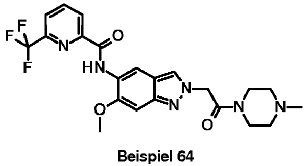

In WO2015104662 werden 2-substituierte Indazole der folgenden allgemeinen Formel offenbart:

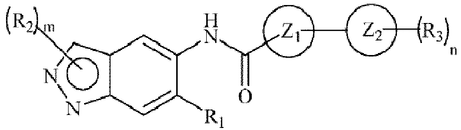

in denen R² eine Alkyl- oder Cycloalkylgruppe ist. Explizit beschrieben werden 2-substituierte Indazole mit einer Methyl, 2-Methoxyethyl und Cyclopentylgruppe an der 2-Position (Beispiele 1, 4 und 76). Außerdem wird mit Beispiel 117 ein Indazolderivat mit einem Hydroxyethyl-Subsituenten an der 1-Position beschrieben. Es werden jedoch keine Indazolderivate mit einem 3-Hydroxy-3-methylbutyl-Substituenten an der 1-Position oder 2-Position beschrieben.
Indazole, die an Position 2 eine Hydroxy-substituierte Alkylgruppe aufweisen, sind zwar generisch mit der allgemeinen Formel umfasst, werden aber in WO2015104662 nicht explizit offenbart.

Indazole mit einer Alkylgruppe an Position 2, wobei die Alkylgruppe zusätzlich mit einer Methylsulfonylgruppe substituiert ist, sind mit der allgemeinen Formel und den Definitionen für den Substituenten R² in WO2015104662 nicht umfasst.

Als Beispiele für Substituenten an der Position 6 werden in WO2015104662 für R¹ Cyclopropyl, Cyclohexyl, Cyano, 3-Fluorphenyl und gesättigte heterocyclische Substituenten beschrieben. Indazole mit einer Hydroxy-substituierten Alkylgruppe an Position 6 werden in WO2015104662 nicht explizit beschrieben.

In WO2015193846 werden 2-substituierte Indazole der folgenden allgemeinen Formel 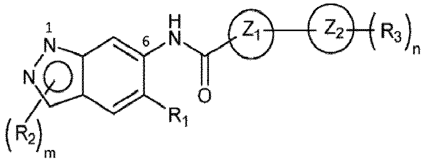 offenbart, in denen Z¹ und Z² jeweils eine gegebenenfalls substituierte Cycloalkyl-, Aryl- oder Heteroarylgruppe sind. R² kann ein Wasserstoff, Halogen, eine Aminogruppe, eine jeweils gegebenenfalls substitutierte Alkyl-, Cycloalkyl-, Aryl-, Heterocyclo-, Arylalkyl- oder Heterocycloalkylgruppe bedeuten. Explizit beschrieben sind Indazolderivate, in denen R² ein Methylrest sowie Z¹ und/ oder Z² Hetarylgruppen bedeuten, wobei der Substituent - NH(C=O)Z¹-Z²-(R³)ₙ, an der 6-Position des Indazolgerüstes gebunden ist. Indazolderivate mit einem Substituenten -NH(C=O)Z¹-Z²-(R³)ₙ gebunden an die 5-Postion werden nicht beschrieben.

### KOMPONENTE B: BTK Inhibitoren:

Bruton-Tyrosinkinase (BTK) ist ein Enzym in Wirbeltieren, das die Phosphorylierung bestimmter Proteine katalysiert. Sie gehört zu den Tyrosinkinasen der Tec-Familie, die vor allem in B-Zellen exprimiert ist. BTK übernimmt wichtige Funktionen bei der Vermittlung des B-Zell-Rezeptor-Signals ins Zellinnere. Eine Mutation im BTK-Gen des Menschen ist Ursache des so genannten Bruton-Syndroms (XLA).

Die chronische lymphatische Leukämie (CLL) ist bisher außer durch eine allogene Stammzelltransplantation nicht heilbar, und Patienten mit bestimmten Risikofaktoren (v. a. 17p-Deletion) profitieren auch von CD20-Antikörpern kaum. Der Signalweg des B-Zell-Rezeptors, der für B-Zell-Lymphome essenziell ist, ist mittlerweile gut erforscht und hat zu neuen therapeutischen Zugängen geführt. Bruton's Tyrosinkinase (BTK) ist eine zentrale Komponente dieses Signalwegs, und mit der Zulassung des BTK-Inhibitors Ibrutinib (Imbruvica^{®}) im Oktober 2014 zeichnet sich ein deutlicher Fortschritt in der Versorgung von Patienten mit CLL ebenso wie mit Mantelzell-Lymphom ab.

Komponente B ist ein BTK Inhibitor, der aus der folgenden Liste ausgewählt ist:
▪ Ibrutinib, oder ein pharmazeutisch verträgliches Salz davon ;
▪ 4-tert-Butyl-N-[2-methyl-3-(4-methyl-6-{[4-(morpholin-4-ylcarbonyl)phenyl]amino}-5-oxo-4,5-dihydropyrazin-2-yl)phenyl]benzamid (CGI-1746, CAS 910232-84-7);
▪ N-{3-[(5-Fluor-2-{[4-(2-methoxyethoxy)phenyl]amino}pyrimidin-4-yl)amino]phenyl}acrylamid (AVL-292, CAS 1202757-89-8) ;
▪ 6-Cyclopropyl-8-fluor-2-[2-(hydroxymethyl)-3-(1-methyl-5-{[5-(4-methylpiperazin-1-yl)pyridin-2-yl]amino}-6-oxo-1,6-dihydropyridin-3-yl)phenyl]isochinolin-1(2H)-on (RN486, CAS 1242156-23-5);
▪ HM71224;
▪ N-{3-[6-({4-[(2R)-1,4-Dimethyl-3-oxopiperazin-2-yl]phenyl}amino)-4-methyl-5-oxo-4,5-dihydropyrazin-2-yl]-2-methylphenyl}-4,5,6,7-tetrahydro-1-benzothiophen-2-carboxamid (GDC-0834, CAS 1133432-50-4) ;
▪ 5-Amino-1-[(3R)-1-cyanpiperidin-3-yl]-3-[4-(2,4-difluorphenoxy)phenyl]-1H-pyrazol-4-carboxamid (PF-06250112, J Immunol 2013; 191:4540-4550) ;
▪ (2E)-4-(Dimethylamino)-N-{7-fluor-4-[(2-methylphenyl)amino]imidazo[1,5-a]chinoxalin-8-yl}-N-methylbut-2-enamid (CAS 1345250-62-5, Bioorg. Med. Chem. Lett. 21 (2011) 6258-6262) ;
▪ N-[3-(8-Anilinoimidazo[1,2-a]pyrazin-6-yl)phenyl]-4-tert-butylbenzamid (CGI-560, CAS 845269-74-1);
▪ 4-{4-[(4-{[3-(Acryloylamino)phenyl]amino}-5-fluorpyrimidin-2-yl)amino]phenoxy}-N-methylpyridin-2-carboxamid (CNX-774, CAS1202759-32-7);
▪ ONO-4059 (Athritis and rheumatism 2012, 64 Suppl 10:1660).

### IBRUTINIB :

Ibrutinib (USAN ("United States Adopted Name")), auch bekannt als PCI-32765, ist 1-{(3R)-3-[4-Amino-3-(4-phenoxyphenyl)-1H-pyrazolo[3,4-d]pyrimidin-1-yl]piperidin-1-yl}prop-2-en-1-on
(CAS Registry Number 936563-96-1) der Formel (II): 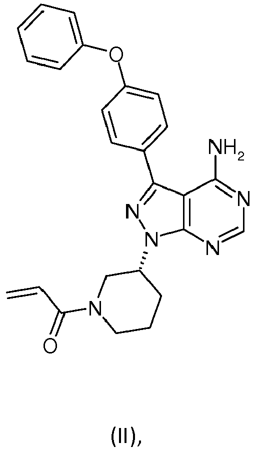 (IBRUTINIB, PCI-32765),
und wird im folgenden als "Ibrutinib" bezeichnet.

Ibrutinib (früher PCI-32765, Fa. Pharmacyclics und Janssen Pharmaceutica) ist ein Arzneistoff aus der Gruppe der Tyrosinkinase-Inhibitoren, der unter dem Handelsnamen *Imbruvica* zur Behandlung des Mantelzelllymphoms eingesetzt wird.

Ibrutinib ist ein oral einzunehmender Tyrosinkinase-Inhibitor, der die Bruton-Tyrosinkinase *(BTK)* hemmt. Diese spielt eine zentrale Rolle bei der intrazellulären Signalübertragung in B-Lymphozyten. Das anvisierte klinische Einsatzgebiet von Ibrutinib sind daher maligne B-Zell-Erkrankungen, im engeren Sinne B-Zell-Non-Hodgkin-Lymphome, aber auch Autoimmunerkrankungen, bei denen B-Zellen eine Rolle spielen, wie die Rheumatoide Arthritis.

Ibrutinib zeigte Wirksamkeit bei intensiv vorbehandelten Patienten mit therapierefraktärer chronischer lymphatischer Leukämie (CLL) bzw. mit Mantelzelllymphom (Ibrutinib: Kinase-Inhibitor gegen B-Zell-Malignome aktiv. Deutsches Ärzteblatt, 20. Juni 2013, abgerufen am 23. Juli 2013). Ibrutinib wurde am 13. November 2013 von der FDA für die Behandlung des Mantelzelllymphoms zugelassen. Der Handelsname in den Vereinigten Staaten lautet Imbruvica. Ibrutinib wurde im Februar 2014 von der FDA für die Behandlung der CLL zugelassen.

Im Juli 2014 empfahl der Ausschuss für Humanarzneimittel der Europäischen Arzneimittel-Agentur (EMA) Ibrutinib zur Zulassung für die Indikation Chronische Lymphatische Leukämie (CLL). Ibrutinib erhielt zudem eine Empfehlung zur Zulassung für die Indikation Mantelzelllymphom und Zydelig für die Indikation Follikuläres Lymphom (FL).

### Vergleiche zu Ibrutinib auch folgende Literaturstellen:

Ibrutinib: Kinase-Inhibitor gegen B-Zell-Malignome aktiv. Deutsches Ärzteblatt, 20. Juni 2013, abgerufen am 23. Juli 2013.

Ibrutinib Receives Two Oncology Breakthrough Therapy Designations from U.S. Food and Drug Administration. prnewswire.com, 12. Februar 2013, abgerufen am 29. Juli 2013 (englisch).

Ibrutinib wird als Verbindung per se als Compound 14 in dem Europäischen Patent EP 2,201,840 B1 und in dem US Patent US 7,514,444 B2 genannt.

Jedoch enthält der Stand der Technik keine Kombinationen wie in der vorliegenden Erfindung beschrieben, die eine IRAK4-inhibierende Verbindung der Formel (I) wie hierin definiert, oder ein Diastereomer, ein Enantiomer, einen Metabolit, ein Salz, ein Solvat oder ein Solvat eines Salzes von dieser, und Ibrutinib, oder ein pharmazeutisch verträgliches Salz von dieser enthalten.

### CGI-1746:

Über die Substanz CGI-1746 (CAS Registry Number 910232-84-7) wird in J. A. Di Paolo et al, Nature Chemical Biology, 2011, 7, 1, 41 - 50, DOI:10.1038/nchembio.481 als spezifischer BTK-Inhibitor berichtet (für die Herstellung vergleiche auch die Supplementary Information).

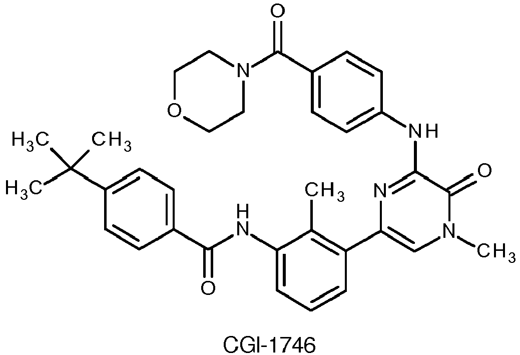

Jedoch enthält der Stand der Technik keine Kombinationen wie in der vorliegenden Erfindung beschrieben, die eine IRAK4-inhibierende Verbindung der Formel (I) wie hierin definiert, oder ein Diastereomer, ein Enantiomer, einen Metabolit, ein Salz, ein Solvat oder ein Solvat eines Salzes von dieser, und GCI-1746, oder ein pharmazeutisch verträgliches Salz von dieser enthalten.

### AVL-292:

Über die Substanz AVL-292 (CAS Registry Number 1202757-89-8) als Inhibitor von BTK wird in WO2009158571 berichtet. Außerdem wird die Herstellung von AVL-292 beschrieben.

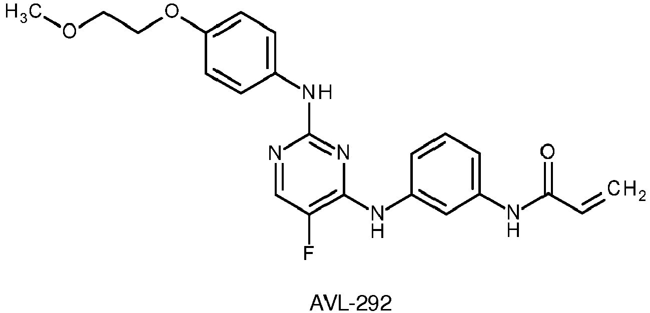

Jedoch enthält der Stand der Technik keine Kombinationen wie in der vorliegenden Erfindung beschrieben, die eine IRAK4-inhibierende Verbindung der Formel (I) wie hierin definiert, oder ein Diastereomer, ein Enantiomer, einen Metabolit, ein Salz, ein Solvat oder ein Solvat eines Salzes von dieser, und AVL-292, oder ein pharmazeutisch verträgliches Salz von dieser enthalten.

### RN486:

Der BTK-Inhibitor RN486 (CAS Registry Number 1242156-23-6, im vorliegenden Text wird auch die Bezeichnung RN-486 verwendet) wird in L. Yan et al, J. Med. Chem., 2015, 58, 512-516 als geeignet für die Behandlung der Rheumatoiden Arthritis beschrieben.

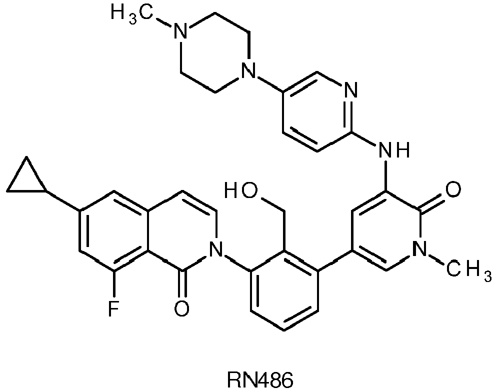

Jedoch enthält der Stand der Technik keine Kombinationen wie in der vorliegenden Erfindung beschrieben, die eine IRAK4-inhibierende Verbindung der Formel (I) wie hierin definiert, oder ein Diastereomer, ein Enantiomer, einen Metabolit, ein Salz, ein Solvat oder ein Solvat eines Salzes von dieser, und RN486, oder ein pharmazeutisch verträgliches Salz von dieser enthalten.

### ZUSAMMENFASSUNG DER ERFINDUNG

Es wurde überraschend gefunden, dass bei der Anwendung eines IRAK4 Inhibitors der Formel (I) wie hierin definiert in Kombination mit dem BTK Inhibitor Ibrutinib ein synergistischer anti-proliferativer Effekt in Tumorzelllinien auftritt.

Ein erster Aspekt der vorliegenden Erfindung betrifft Kombinationen von mindestens zwei Komponenten, Komponente A und Komponente B:
- Komponente A ist eine IRAK4-inhibierende Verbindung der Formel (I) wie hierin definiert, oder ein Diastereomer, ein Enantiomer, ein Metabolit, ein Salz, ein Solvat oder ein Solvat eines Salzes von dieser;
- Komponente B ist eine BTK-inhibierende Verbindung, z.B. Ibrutinib, oder ein pharmazeutisch verträgliches Salz von dieser.

Ein zweiter Aspekt der vorliegenden Erfindung betrifft Kombinationen von mindestens zwei Komponenten A und B:
- Komponente A ist eine IRAK4-inhibierende Verbindung der Formel (I) wie hierin definiert, oder ein Diastereomer, ein Enantiomer, ein Metabolit, ein Salz, ein Solvat oder ein Solvat eines Salzes von dieser;
- Komponente B ist eine BTK-inhibierende Verbindung, z.B. Ibrutinib.

Ein dritter Aspekt der vorliegenden Erfindung betrifft Kombinationen von mindestens zwei Komponenten A und B:
- Komponente A ist eine IRAK4-inhibierende Verbindung der Formel (I) wie hierin definiert;
- Komponente B ist eine BTK-inhibierende Verbindung, z.B. Ibrutinib, oder ein pharmazeutisch verträgliches Salz von dieser.

Ein vierter Aspekt der vorliegenden Erfindung betrifft Kombinationen von mindestens zwei Komponenten A und B:
- Komponente A ist eine IRAK4-inhibierende Verbindung der Formel (I) wie hierin definiert;
- Komponente B ist Ibrutinib.

Die Kombinationen von mindestens zwei Komponenten A und B, wie hierin beschrieben und definiert, werden auch als "Kombinationen der vorliegenden Erfindung" bezeichnet.

Weiterhin betrifft die vorliegende Erfindung ein Kit enthaltend eine Kombination von :
- Komponente A, die aus einem IRAK4 Inhibitor der Formel (I) wie hierin definiert oder einem Diastereomer, einem Enantiomer, einem Metabolit, einem Salz, einem Solvat oder einem Solvat eines Salzes von dieser besteht;
- Komponente B, die eine BTK-inhibierende Verbindung, z.B. Ibrutinib ist, oder ein pharmazeutisch verträgliches Salz von dieser.
und gegebenenfalls
- Komponente C, die aus einem oder mehreren pharmazeutischen Mitteln besteht;
in dem gegebenenfalls eine oder beide der oben definierten Komponenten A und B in allen oben beschriebenen Kombinationen in einer pharmazeutischen Formulierung/Zusammensetzung vorliegen, die zur simultanen, separaten oder sequenziellen Verabreichung bereit sind. Die Komponenten können unabhängig voneinander oral, intravenös, topisch, intraperitoneal, nasal oder als Depot verabreicht werden.

Ein weiterer Aspekt der vorliegenden Erfindung betrifft die Kombinationen wie hierin definiert zur Behandlung oder Vorbeugung einer Krankheit.

Ein weiterer Aspekt der vorliegenden Erfindung betrifft die Verwendung solcher Kombinationen wie hierin definiert zur Herstellung eines Medikaments zur Behandlung oder Vorbeugung einer Krankheit.

Die Kombinationen sollen insbesondere zur Behandlung und zur Prävention von proliferativen und entzündlichen Erkrankungen geeignet sein, die durch ein überreagierendes Immunsystem charakterisiert sind. Besonders genannt seien hier entzündliche Hauterkrankungen, Herz-Kreislauf-Erkrankungen, Lungenerkrankungen, Augenerkrankungen, Autoimmunerkrankungen, gynäkologische Erkrankungen, insbesondere Endometriose und Krebserkrankungen.

Besonders geeignet sollen die Kombinationen für die Behandlung von Krebserkrankungen sein.

Ganz besonders geeignet sollen die Kombinationen für die Behandlung der folgenden Krebserkrankungen sein: Non-Hodgkin-Lymphome (abgekürzt als "NHL"), insbesondere Erstlinientherapie oder Zweitlinientherapie rezidiver oder refraktärer indolenter oder aggressiver Non-Hodgkin-Lymphome (NHL), insbesondere des Follikulären Lymphoms (abgekürzt als "FL"), der chronischen lymphatischen Leukämie (abgekürzt als "CLL"), des Marginalzonen-Lymphoms (abgekürzt als "MZL"), des Diffusen großzelligen B-Zell-Lymphoms (abgekürzt als "DLBCL"), insbesondere des aktivierten B-Zellen ähnlichen Diffusen großzelligen B-Zell-Lymphoms (abgekürzt als "ABC-DLBCL"), des Mantelzelllymphoms (abgekürzt als "MCL"), des transformierten Lymphoms (abgekürzt als "TL"), peripherer T-Zell-Lymphome (abgekürzt als "PTCL") oder der lymphoplasmazytischen Lymphome (Waldenströms Makroglobulinämie (abgekürzt als "WM")).

### KOMPONENTE A DER KOMBINATION

Komponente A ist eine Verbindung der allgemeinen Formel (I) 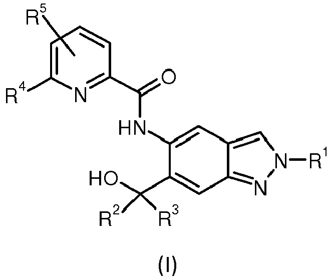 in der:
- R¹: für C₁-C₆-Alkyl steht, wobei der C₁-C₆-Alkylrest unsubstituiert oder ein- oder mehrfach, gleich oder verschieden substituiert ist mit
Halogen, Hydroxy, einem unsubstituierten oder ein- oder mehrfach mit Halogen substituierten C₃-C₆-Cycloalkyl, einem Rest R⁶, R⁷SO₂, R⁷SO oder R⁸O
oder für eine Gruppe steht, ausgewählt aus: 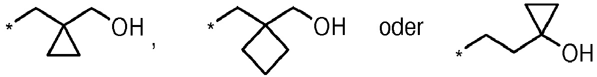 wobei * für die Verknüpfungsstelle der Gruppe mit dem Rest des Moleküls steht;
- R² und R³: immer dieselbe Bedeutung haben und gleichzeitig entweder für Wasserstoff oder C₁-C₆-Alkyl stehen;

- R⁴: für Halogen, Cyano, ein unsubstituiertes oder ein- oder mehrfach, gleich oder verschieden voneinander substituiertes C₁-C₆-Alkyl oder ein unsubstituiertes oder ein- oder mehrfach, gleich oder verschieden voneinander substituiertes C₃-C₆-Cycloalkyl steht, und die Substituenten ausgewählt sind aus der Gruppe Halogen und Hydroxy;

- R⁵: für Wasserstoff, Halogen oder ein unsubstituiertes oder ein- oder mehrfach mit Halogen substituiertes C₁-C₆-Alkyl steht,

- R⁶: für einen unsubstituierten oder ein- oder zweifach mit Methyl substituierten monocyclischen, gesättigten Heterocyclus mit 4 bis 6 Ringatomen steht, der ein Heteroatom oder eine Heterogruppe aus der Reihe O, S, SO oder SO₂ enthält.
- R⁷: für C₁-C₆-Alkyl steht, wobei der C₁-C₆-Alkylrest unsubstituiert oder ein- oder mehrfach, gleich oder verschieden mit Halogen, Hydroxy oder C₃-C₆-Cycloalkyl substituiert ist; oder R⁷ steht für C₃-C₆-Cycloalkyl,

- R⁸: für C₁-C₆-Alkyl steht, wobei der C₁-C₆-Alkylrest unsubstituiert oder ein- oder mehrfach, gleich oder verschieden mit Halogen, substituiert ist;
und ihre Diastereomere, Enantiomere, ihre Metabolite, ihre Salze, ihre Solvate oder die Solvate ihrer Salze.

Wenn bei den im Folgenden beschriebenen Synthese-Intermediaten und Ausführungsbeispielen der Erfindung eine Verbindung in der Form eines Salzes der korrespondierenden Base bzw. Säure aufgeführt ist, so ist die exakte stöchiometrische Zusammensetzung eines solchen Salzes, wie es nach dem jeweiligen Herstell- und/oder Reinigungsverfahren erhalten wurde, in der Regel nicht bekannt. Sofern nicht genauer spezifiziert, sind daher Namens- und Strukturformel-Zusätze wie beispielsweise "Hydrochlorid", "Trifluoracetat", "Natrium-Salz" bzw. "x HCl", "x CF₃COOH", "x Na⁺" bei solchen Salzen nicht stöchiometrisch zu verstehen, sondern haben allein deskriptiven Charakter bezüglich der enthaltenen salzbildenden Komponenten.

Sinngemäß gleiches gilt für den Fall, dass Synthese-Intermediate oder Ausführungsbeispiele oder Salze hiervon nach den beschriebenen Herstell- und/oder Reinigungsverfahren in Form von Solvaten, wie beispielsweise Hydraten, erhalten wurden, deren stöchiometrische Zusammensetzung (sofern definierter Art) nicht bekannt ist.

Bestandteile der erfindungsgemäßen Kombinationen sind die Verbindungen der Formel (I) und deren Salze, Solvate und Solvate der Salze, die von Formel (I) umfassten Verbindungen der nachfolgend genannten Formeln und deren Salze, Solvate und Solvate der Salze sowie die von Formel (I) umfassten, nachfolgend als Ausführungsbeispiele genannten Verbindungen und deren Salze, Solvate und Solvate der Salze, soweit es sich bei den von Formel (I) umfassten, nachfolgend genannten Verbindungen nicht bereits um Salze, Solvate und Solvate der Salze handelt.

Als Salze sind im Rahmen der vorliegenden Erfindung physiologisch unbedenkliche Salze der Verbindungen bevorzugt. Umfasst sind aber auch Salze, die für pharmazeutische Anwendungen selbst nicht geeignet sind, aber beispielsweise für die Isolierung oder Reinigung der Verbindungen verwendet werden können.

Physiologisch unbedenkliche Salze der Verbindungen umfassen Säure-additionssalze von Mineralsäuren, Carbonsäuren und Sulfonsäuren, z.B. Salze der Chlorwasserstoffsäure, Bromwasserstoffsäure, Schwefelsäure, Phosphorsäure, Methansulfonsäure, Ethansulfonsäure, Toluolsulfonsäure, Benzolsulfonsäure, Naphthalindisulfonsäure, Essigsäure, Trifluoressigsäure, Propionsäure, Milchsäure, Weinsäure, Äpfelsäure, Zitronensäure, Fumarsäure, Maleinsäure und Benzoesäure.

Physiologisch unbedenkliche Salze der Verbindungen umfassen auch Salze üblicher Basen, wie beispielhaft und vorzugsweise Alkalimetallsalze (z.B. Natrium- und Kaliumsalze), Erdalkalisalze (z.B. Calcium- und Magnesiumsalze) und Ammoniumsalze, abgeleitet von Ammoniak oder organischen Aminen mit 1 bis 16 C-Atomen, wie beispielhaft und vorzugsweise Ethylamin, Diethylamin, Triethylamin, Ethyldiisopropylamin, Monoethanolamin, Diethanolamin, Triethanolamin, Dicyclo-hexylamin, Dimethylaminoethanol, Prokain, Dibenzylamin, N-Methylmorpholin, Arginin, Lysin, Ethylendiamin und N-Methylpiperidin.

Als Solvate werden im Rahmen der Erfindung solche Formen der Verbindungen bezeichnet, welche in festem oder flüssigem Zustand durch Koordination mit Lösungsmittelmolekülen einen Komplex bilden. Hydrate sind eine spezielle Form der Solvate, bei denen die Koordination mit Wasser erfolgt.

Die Verbindungen der Formel (I) als Bestandteile der erfindungsgemäßen Kombinationen können in Abhängigkeit von ihrer Struktur in unter-schiedlichen stereoisomeren Formen existieren, d.h. in Gestalt von Konfigurationsisomeren oder gegebenenfalls auch als Konformationsisomere (Enantiomere und/oder Diastereomere, einschließlich solcher bei Atropisomeren). Die vorliegende Erfindung umfasst deshalb die Enantiomere und Diastereomere und ihre jeweiligen Mischungen. Aus solchen Mischungen von Enantiomeren und/ oder Diastereomeren lassen sich die stereoisomer einheitlichen Bestandteile in bekannter Weise isolieren; vorzugsweise werden hierfür chromatographische Verfahren verwendet, insbesondere die HPLC-Chromatographie an achiraler bzw. chiraler Phase.

Sofern die Verbindungen der Formel (I) als Bestandteile der erfindungsgemäßen Kombinationen in tautomeren Formen vorkommen können, umfasst die vorliegende Erfindung sämtliche tautomere Formen.

Die vorliegende Erfindung umfasst auch alle geeigneten isotopischen Varianten der erfindungsgemäßen Verbindungen. Unter einer isotopischen Variante einer erfindungsgemäßen Verbindung wird hierbei eine Verbindung verstanden, in welcher mindestens ein Atom innerhalb der erfindungsgemäßen Verbindung gegen ein anderes Atom der gleichen Ordnungszahl, jedoch mit einer anderen Atommasse als der gewöhnlich oder überwiegend in der Natur vorkommenden Atommasse ausgetauscht ist. Beispiele für Isotope, die in eine erfindungsgemäße Verbindung inkorporiert werden können, sind solche von Wasserstoff, Kohlenstoff, Stickstoff, Sauerstoff, Phosphor, Schwefel, Fluor, Chlor, Brom und lod, wie 2H (Deuterium), 3H (Tritium), 13C, 14C, 15N, 170, 180, 32P, 33P, 33S, 34S, 35S, 36S, 18F, 36Cl, 82Br, 1231, 1241, 1291 und 1311. Bestimmte isotopische Varianten einer erfindungsgemäßen Verbindung, wie insbesondere solche, bei denen ein oder mehrere radioaktive Isotope inkorporiert sind, können von Nutzen sein beispielsweise für die Untersuchung des Wirkmechanismus oder der Wirkstoffverteilung im Körper; aufgrund der vergleichsweise leichten Herstell- und Detektierbarkeit sind hierfür insbesondere mit 3H- oder 14C-Isotopen markierte Verbindungen geeignet. Darüber hinaus kann der Einbau von Isotopen, wie beispielsweise von Deuterium, zu bestimmten therapeutischen Vorteilen als Folge einer größeren metabolischen Stabilität der Verbindung führen, wie beispielsweise eine Verlängerung der Halbwertszeit im Körper oder eine Reduktion der erforderlichen Wirkdosis; solche Modifikationen der Verbindungen können daher gegebenenfalls auch eine bevorzugte Ausführungsform der vorliegenden Erfindung darstellen. Isotopische Varianten der Verbindungen können nach den dem Fachmann bekannten Verfahren hergestellt werden, so beispielsweise nach den weiter unten beschriebenen Methoden und den für die Ausführungsbeispiele angegebenen Vorschriften, indem entsprechende isotopische Modifikationen der jeweiligen Reagentien und/oder Ausgangsverbindungen eingesetzt werden.

Ein weiterer Gegenstand der vorliegenden Erfindung sind alle möglichen kristallinen und polymorphen Formen der Verbindungen der Formel (I) als Bestandteile der erfindungsgemäßen Kombinationen, wobei die Polymorphe entweder als einzelne Polymorphe oder als Gemisch mehrerer Polymorphe in allen Konzentrationsbereichen vorliegen können.

Außerdem umfasst die vorliegende Erfindung auch Prodrugs der Verbindungen der Formel (I) als Bestandteile der erfindungsgemäßen Kombinationen. Der Begriff "Prodrugs" bezeichnet hierbei Verbindungen, welche selbst biologisch aktiv oder inaktiv sein können, jedoch während ihrer Verweilzeit im Körper zu Verbindungen umgesetzt werden (beispielsweise metabolisch oder hydrolytisch).

Die Substituenten der Verbindungen der Formel (I) als Bestandteile der erfindungsgemäßen Kombinationen haben, soweit nicht anders spezifiziert, die folgenden Bedeutungen:
Alkyl steht im Rahmen der Erfindung für einen linearen oder verzweigten Alkylrest mit der jeweils angegebenen Anzahl an Kohlenstoffatomen. Beispielhaft seien Methyl, Ethyl, n-Propyl, Isopropyl, n-Butyl, iso-Butyl, 1-Methylpropyl, 2-Methylpropyl, tert.-Butyl, n-Pentyl, 1-Ethylpropyl, 1-Methylbutyl, 2-Methylbutyl, 3-Methylbutyl, 2,2-Dimethylpropyl, n-Hexyl, 1-Methylpentyl, 2-Methylpentyl, 3-Methylpentyl, 4-Methylpentyl, 1-Ethylbutyl und 2-Ethylbutyl genannt. Bevorzugt sind Methyl, Ethyl, n-Propyl, n-Butyl, 2-Methylbutyl, 3-Methylbutyl und 2,2-Dimethylpropyl.

Cycloalkyl steht im Rahmen der Erfindung für einen monocyclischen, gesättigten Alkylrest mit der jeweils angegebenen Anzahl an Kohlenstoffatomen. Beispielhaft und vorzugsweise seien Cyclopropyl, Cyclobutyl, Cyclopentyl und Cyclohexyl genannt.

Alkoxy steht im Rahmen der Erfindung für einen linearen oder verzweigten Alkoxyrest mit der jeweils angegebenen Anzahl an Kohlenstoffatomen. 1 bis 6 Kohlenstoffatome sind bevorzugt. Beispielhaft seien Methoxy, Ethoxy, n-Propoxy, Isopropoxy, 1-Methylpropoxy, n-Butoxy, iso-Butoxy, tert.-Butoxy, n-Pentoxy, iso-Pentoxy, 1-Ethylpropoxy, 1-Methylbutoxy, 2-Methylbutoxy, 3-Methylbutoxy und n-Hexoxy genannt. Besonders bevorzugt ist ein linearer oder verzweigter Alkoxyrest mit 1 bis 4 Kohlenstoffatomen. Beispielhaft und vorzugsweise seien Methoxy, Ethoxy, n-Propoxy, 1-Methylpropoxy, n-Butoxy und iso-Butoxy genannt.

Halogen steht im Rahmen der Erfindung für Fluor, Chlor und Brom. Bevorzugt ist Fluor.

Hydroxy steht im Rahmen der Erfindung für OH.

Ein monocyclischer, gesättigter Heterocyclus steht für einen monocyclischen, gesättigten Heterocyclus mit 4 bis 6 Ringatomen, der ein Heteroatom oder eine Heterogruppen aus der Reihe O, S, SO oder SO₂ enthält. Ein Heterocylcus mit einem Heteroatom oder einer Heterogruppe aus der Reihe O, SO oder SO₂ ist bevorzugt. Beispielsweise seien genannt: Oxetan, Tetrahydrofuran, Tetrahydro-2H-pyran-4-yl, 1,1-Dioxidotetrahydro-2H-thiopyran-3-yl, 1,1-Dioxidotetrahydro-2H-thiopyran-2-yl, 1,1-Dioxidotetrahydro-2H-thiopyran-4-yl, 1,1-Dioxidotetrahydrothiophen-3-yl, 1,1-Dioxidotetrahydrothiophen-2-yl, 1,1-Dioxidothietan-2-yl oder 1,1-Dioxidothietan-3-yl. Besonders bevorzugt sind hierbei Oxetan und Tetrahydrofuran. Ganz besonders bevorzugt ist Oxetan-3-yl.

Ein Symbol * an einer Bindung bedeutet die Verknüpfungsstelle im Molekül.

Wenn Reste in den Verbindungen der Formel (I) als Bestandteile der erfindungsgemäßen Kombinationen substituiert sind, können die Reste, soweit nicht anders spezifiziert, ein- oder mehrfach substituiert sein. Im Rahmen der vorliegenden Erfindung gilt, dass für alle Reste, die mehrfach auftreten, deren Bedeutung unabhängig voneinander ist. Eine Substitution mit ein, zwei oder drei gleichen oder verschiedenen Substituenten ist bevorzugt.

Eine bevorzugte Ausführungsform von R¹ ist ein mit 1, 2 oder 3 Fluoratomen substituierter C₂-C₆-Alkylrest. Besonders bevorzugt sind 2,2,2-Trifluorethyl, 3,3,3-Trifluorpropyl und 4,4,4-Trifluorbutyl. Ganz besonders bevorzugt ist 4,4,4-Trifluorbutyl. Eine weitere bevorzugte Ausführungsform von R¹ ist ein C₂-C₆-Alkylrest, der mit einer oder zwei Hydroxygruppe(n) oder einem C₁-C₃-Alkoxy oder einem dreifach mit Fluor substituierten C₁-C₃-Alkoxy substituiert ist. Besonders bevorzugt ist ein C₂-C₅-Alkylrest, der mit Hydroxy oder C₁-C₃-Alkoxy oder mit Trifluormethoxy oder 2,2,2-Trifluorethoxy substituiert ist. Ganz besonders bevorzugt sind 3-Hydroxy-3-methylbutyl, 3-Methoxypropyl, 3-Hydroxypropyl, 3-Trifluormethoxypropyl, 2-Methoxyethyl oder 2-Hydroxyethyl. Insbesondere ist 3-Hydroxy-3-methylbutylrest bevorzugt.

Weiterhin bevorzugt steht R¹ für einen mit einer C₁-C₆-Alkyl-SO₂-Gruppe substituierten C₂-C₆-Alkylrest. Ein mit Methyl-SO₂ substituierter C₂-C₄-Alkylrest ist besonders bevorzugt. Insbesondere sind für R¹ 2-(Methylsulfonyl)ethyl oder 3-(Methylsulfonyl)propyl bevorzugt. Aus der letztgenannten Gruppe ist 2-(Methylsulfonyl)ethyl besonders bevorzugt.

Weiterhin bevorzugt steht R¹ für ein mit Oxetanyl, Tetrahydrofuranyl, Tetrahydro-2H-pyran-4-yl, 1,1-Dioxidotetrahydro-2H-thiopyran-3-yl, 1,1-Dioxidotetrahydro-2H-thiopyran-2-yl, 1,1-Dioxidotetrahydro-2H-thiopyran-4-yl, 1,1-Dioxidotetrahydrothiophen-3-yl, 1,1-Dioxidotetrahydrothiophen-2-yl, 1,1-Dioxidothietan-2-yl oder 1,1-Dioxidothietan-3-yl substituierter C₁-C₃ Alkylrest. Besonders bevorzugt ist ein mit einer Oxetangruppe subsituierter C₁-C₃ Alkylrest. Insbesondere ist eine Oxetan-3-ylmethylgruppe für R¹ bevorzugt.

Für R² und R³, die immer dieselbe Bedeutung haben, sind Wasserstoff oder Methyl bevorzugt. Dabei ist Methyl besonders bevorzugt.
Im Fall von R⁴ ist ein unsubstituierter oder ein ein- oder mehrfach mit Halogen substituierter C₁-C₃-Alkylrest oder ein mit einer Hydroxygruppe substituierter C₁-C₃-Alkylrest oder ein mit einer Hydroxygruppe und drei Fluoratomen substituierter C₁-C₃-Alkylrest bevorzugt.
Besonders bevorzugt für R⁴ sind folgende Reste: Methyl, Ethyl, Trifluor-C₁-C₃-alkyl, Difluor-C₁-C₃-Alkyl, Hydroxymethyl, 1-Hydroxyethyl, 2-Hydroxypropan-2-yl und 2,2,2-Trifluor-1-hydroxyethyl. Besonders bevorzugt für R⁴ sind die Reste Methyl, Trifluormethyl und Difluormethyl. Hierbei ist ein Trifluormethylrest besonders bevorzugt.

Eine bevorzugte Ausführungsform von R⁵ ist Wasserstoff, Fluor, Chlor oder C₁-C₃-Alkyl. Besonders bevorzugt ist R⁵ Wasserstoff, Fluor oder Methyl. Ganz besonders bevorzugt ist R⁵ Wasserstoff oder Fluor.

Besonders bevorzugt sind außerdem Verbindungen der Formel (I) als Bestandteile der erfindungsgemäßen Kombinationen, in denen R⁴ Methyl oder Trifluormethyl bedeutet und R⁵ Fluor. Ganz besonders bevorzugt sind Verbindungen bei denen R⁴ für Methyl steht und R⁵ für Fluor steht, wobei R⁵ in ortho-Position zu R⁴ steht.

Für R⁶ sind als bevorzugte Ausführungsformen Oxetanyl, Tetrahydrofuranyl, Tetrahydro-2H-pyran-4-yl, 1,1-Dioxidotetrahydro-2H-thiopyran-3-yl, 1,1-Dioxidotetrahydro-2H-thiopyran-2-yl, 1,1-Dioxidotetrahydro-2H-thiopyran-4-yl, 1,1-Dioxidotetrahydrothiophen-3-yl, 1,1-Dioxidotetrahydrothiophen-2-yl, 1,1-Dioxidothietan-2-yl oder 1,1-Dioxidothietan-3-yl zu nennen. Besonders bevorzugt ist hierbei Oxetanyl. Ganz besonders bevorzugt ist Oxetan-3-yl.

R⁷ steht ausschließlich im Zusammenhang mit den funktionellen Gruppen -SO₂- und -SO-, d.h. für eine mit R⁷ substituierte -SO₂- oder SO-Gruppe. In diesem Zusammenhang ist für R⁷ C₁-C₄-Alkyl bevorzugt, wobei der C₁-C₄-Alkylrest unsubstituiert oder einfach mit Hydroxy oder mit Cyclopropyl oder mit drei Fluoratomen substituiert ist. Weiterhin bevorzugt ist für R⁷ ein Cyclopropylrest. Besonders bevorzugt für R⁷ sind Methyl, Ethyl oder Hydroxyethyl. Ganz besonders ist Methyl für R⁷ bevorzugt.

Das bedeutet, dass im Falle eines mit R⁷SO₂- oder R⁷SO- susbtituierten C₁-C₆-Alkyl im Sinne von R¹ ein mit einem C₁-C₆-Alkyl-SO₂ oder einem C₁-C₆-Alkyl-SO substituiertes C₁-C₆-Alkyl bevorzugt ist. Hierbei sind sind für R¹ insbesondere Methylsulfonylethyl und Methylsulfonylpropyl bevorzugt. Ganz besonders bevorzugt ist hierbei Methylsulfonylethyl.

Für R⁸ ist ein unsubstituierter C₁-C₄-Alkylrest oder ein dreifach mit Fluor substituierter C₁-C₄-Alkylrest bevorzugt. Besonders bevorzugt sind Methyl, Ethyl, Trifluormethyl oder 2,2,2-Trifluorethyl. Ganz besonders bevorzugt sind Methyl, Trifluormethyl oder 2,2,2-Trifluorethyl.

Bevorzugt sind Verbindungen der Formel (I) als Bestandteile der erfindungsgemäßen Kombinationen, in der
- R¹: für C₁-C₆-Alkyl steht, wobei der C₁-C₆-Alkylrest unsubstituiert oder ein- oder mehrfach, gleich oder verschieden substituiert ist mit Fluor, Hydroxy, einem Rest R⁶, R⁷SO₂, R⁷SO oder R⁸O,

- R² und R³: immer dieselbe Bedeutung haben und gleichzeitig entweder für Wasserstoff oder C₁-C₃-Alkyl stehen;

- R⁴: für Halogen, Cyano oder C₁-C₃-Alkyl steht, wobei der C₁-C₃-Alkylrest unsubstituiert oder ein- oder mehrfach, gleich oder verschieden substituiert ist mit Halogen oder Hydroxy;

- R⁵: für Wasserstoff, Fluor, Chlor oder C₁-C₃ Alkyl steht;
- R⁶: für Oxetanyl oder Tetrahydrofuranyl steht;
- R⁷: für C₁-C₄-Alkyl steht, wobei der C₁-C₄-Alkylrest unsubstituiert oder einfach mit Hydroxy oder mit Cyclopropyl oder mit drei Fluoratomen substituiert ist;
- R⁸: für unsubstituiertes C₁-C₄-Alkyl oder dreifach mit Fluor substituiertes C₁-C₄-Alkyl steht;
sowie ihre Diastereomere, Enantiomere, ihre Metabolite, ihre Salze, ihre Solvate oder die Solvate ihrer Salze.

Weiterhin bevorzugt sind Verbindungen der Formel (I) als Bestandteile der erfindungsgemäßen Kombinationen, in der
- R¹: für C₂-C₆-Alkyl steht, wobei C₂-C₆-Alkyl unsubstituiert ist, oder C₂-C₆-Alkyl ein-, zwei- oder dreifach mit Fluor substituiert ist oder C₂-C₆-Alkyl einfach mit Hydroxy, R⁶, R⁷SO₂, oder R⁸O substituiert ist, oder in der R¹ für ein mit Oxetanyl subsituiertes C₁-C₃ Alkyl steht;

- R² und R³: immer dieselbe Bedeutung haben und gleichzeitig entweder für Wasserstoff oder Methyl stehen;

- R⁴: für einen unsubstituierten oder einen ein- oder mehrfach mit Halogen substituierten C₁-C₃-Alkylrest oder einen mit einer Hydroxygruppe substituierten C₁-C₃-Alkylrest oder einen mit einer Hydroxygruppe und drei Fluoratomen substituierten C₁-C₃-Alkylrest steht

- R⁵: für Wasserstoff, Fluor oder C₁-C₃ Alkyl steht;

- R⁷: für C₁-C₃-Alkyl steht,

- R⁸: für C₁-C₄-Alkyl steht, wobei der C₁-C₄-Alkylrest unsubstituiert oder ein-, zwei- oder dreifach mit Fluor substituiert ist;
sowie ihre Diastereomere, Enantiomere, ihre Metabolite, ihre Salze, ihre Solvate oder die Solvate ihrer Salze.

Besonders bevorzugt sind außerdem Verbindungen der allgemeinen Formel (I) als Bestandteile der erfindungsgemäßen Kombinationen, in der
- R¹: für einen C₂-C₅-Alkylrest, der mit Hydroxy oder C₁-C₃-Alkoxy oder Trifluormethoxy oder 2,2,2-Trifluorethoxy oder Trifluormethyl substitutiert ist oder für einen mit Methyl-SO₂ substituierten C₂-C₄-Alkylrest oder für einen mit Oxetan-3-yl subsituierten C₁-C₂ Alkylrest steht;
- R² und R³: immer dieselbe Bedeutung haben und gleichzeitig für Wasserstoff oder Methyl stehen;

- R⁴: für Methyl, Ethyl, Trifluor-C₁-C₃-alkyl, Difluor-C₁-C₃-Alkyl, Hydroxymethyl, 1-Hydroxyethyl, 2-Hydroxypropan-2-yl und 2,2,2-Trifluor-1-hydroxyethyl steht;

- R⁵: für Wasserstoff, Fluor oder Methyl steht;
sowie ihre Diastereomere, Enantiomere, ihre Metabolite, ihre Salze, ihre Solvate oder die Solvate ihrer Salze.

Ganz besonders bevorzugt sind Verbindungen der Formel (I) als Bestandteile der erfindungsgemäßen Kombinationen, in denen
- R¹: für 4,4,4-Trifluorbutyl, 3-Hydroxy-3-methylbutyl, 3-Hydroxybutyl, 3-Methoxypropyl, 3-Hydroxypropyl, 3-Hydroxy-2-methylpropyl, 3-Hydroxy-2,2-dimethylpropyl, 3-Trifluormethoxypropyl, 2-Methoxyethyl, 2-Hydroxyethyl, 2-(Methylsulfonyl)ethyl oder 3-(Methylsulfonyl)propyl steht;

- R² und R³: gleichzeitig für Methyl oder Wasserstoff stehen; und

- R⁴: für Difluormethyl, Trifluormethyl oder Methyl steht; und

- R⁵: für Wasserstoff oder Fluor steht.
sowie ihre Diastereomere, Enantiomere, ihre Metabolite, ihre Salze, ihre Solvate oder die Solvate ihrer Salze.

Ganz besonders bevorzugt sind außerdem Verbindungen der Formel (I) als Bestandteile der erfindungsgemäßen Kombinationen, in denen
- R¹: für 3-Hydroxy-3-methylbutyl, 3-Hydroxybutyl, 3-Hydroxy-2-methylpropyl, 3-Hydroxy-2,2-dimethylpropyl, 3-(Methylsulfonyl)propyl oder 2-(Methylsulfonyl)ethyl steht;

- R² und R³: gleichzeitig für Methyl stehen;

- R⁴: für Difluormethyl oder Trifluormethyl steht; und

- R⁵: für Wasserstoff steht;
sowie ihre Diastereomere, Enantiomere, ihre Metabolite, ihre Salze, ihre Solvate oder die Solvate ihrer Salze.

Weiterhin besonders bevorzugt sind außerdem Verbindungen der Formel (I) als Bestandteile der erfindungsgemäßen Kombinationen, in denen
- R¹: für 3-Hydroxy-3-methylbutyl, 3-Hydroxybutyl, 3-Hydroxy-2-methylpropyl, 3-Hydroxy-2,2-dimethylpropyl, 3-(Methylsulfonyl)propyl oder 2-(Methylsulfonyl)ethyl steht;

- R² und R³: gleichzeitig für Methyl stehen;

- R⁴: für Methyl steht und

- R⁵: für Fluor steht, wobei R⁵ in ortho-Position zu R⁴ steht;
sowie ihre Diastereomere, Enantiomere, ihre Metabolite, ihre Salze, ihre Solvate oder die Solvate ihrer Salze.

Gegenstand der vorliegenden Erfindung sind insbesondere die folgenden Verbindungen der Formel (I) als Bestandteile der erfindungsgemäßen Kombinationen:
1) N-[6-(2-Hydroxypropan-2-yl)-2-(2-methoxyethyl)-2H-indazol-5-yl]-6-(trifluormethyl)pyridin-2-carboxamid
2) N-[6-(Hydroxymethyl)-2-(2-methoxyethyl)-2H-indazol-5-yl]-6-(trifluormethyl)pyridin-2-carboxamid
3) N-[6-(2-Hydroxypropan-2-yl)-2-(3-methoxypropyl)-2H-indazol-5-yl]-6-(trifluormethyl)pyridin-2-carboxamid
4) N-[6-(Hydroxymethyl)-2-(3-methoxypropyl)-2H-indazol-5-yl]-6-(trifluormethyl)pyridin-2-carboxamid
5) N-[2-(2-Hydroxyethyl)-6-(2-hydroxypropan-2-yl)-2H-indazol-5-yl]-6-(trifluormethyl)pyridin-2-carboxamid
6) N-[6-(2-Hydroxypropan-2-yl)-2-(3-hydroxypropyl)-2H-indazol-5-yl]-6-(trifluormethyl)pyridin-2-carboxamid
7) N-[2-(2-Hydroxyethyl)-6-(hydroxymethyl)-2H-indazol-5-yl]-6-(trifluormethyl)pyridin-2-carboxamid
8) N-[6-(2-Hydroxypropan-2-yl)-2-(oxetan-3-ylmethyl)-2H-indazol-5-yl]-6-(trifluormethyl)pyridin-2-carboxamid
9) N-[6-(Hydroxymethyl)-2-(oxetan-3-ylmethyl)-2H-indazol-5-yl]-6-(trifluormethyl)pyridin-2-carboxamid
10) N-{6-(2-Hydroxypropan-2-yl)-2-[3-(methylsulfonyl)propyl]-2H-indazol-5-yl}-6-(trifluormethyl)pyridin-2-carboxamid
11) N-[2-(3-Hydroxy-3-methylbutyl)-6-(2-hydroxypropan-2-yl)-2H-indazol-5-yl]-6-(trifluormethyl)pyridin-2-carboxamid
12) N-{6-(2-Hydroxypropan-2-yl)-2-[2-(methylsulfonyl)ethyl]-2H-indazol-5-yl}-6-(trifluormethyl)pyridin-2-carboxamid
13) 6-(Difluormethyl)-N-[2-(3-hydroxy-3-methylbutyl)-6-(2-hydroxypropan-2-yl)-2H-indazol-5-yl]pyridin-2-carboxamid
14) 6-(Difluormethyl)-N-{6-(2-hydroxypropan-2-yl)-2-[2-(methylsulfonyl)ethyl]-2H-indazol-5-yl}pyridin-2-carboxamid
15) 6-(Difluormethyl)-N-[6-(2-hydroxypropan-2-yl)-2-(3-hydroxypropyl)-2H-indazol-5-yl]pyridin-2-carboxamid
16) N-[6-(2-Hydroxypropan-2-yl)-2-(4,4,4-trifluorbutyl)-2H-indazol-5-yl]-6-(trifluormethyl)pyridin-2-carboxamid
17) N-{6-(2-Hydroxypropan-2-yl)-2-[3-(trifluormethoxy)propyl]-2H-indazol-5-yl}-6-(trifluormethyl)pyridin-2-carboxamid
18) N-{6-(2-Hydroxypropan-2-yl)-2-[3-(2,2,2-trifluorethoxy)propyl]-2H-indazol-5-yl}-6-(trifluormethyl)pyridin-2-carboxamid
19) 5-Fluor-N-[2-(3-hydroxy-3-methylbutyl)-6-(2-hydroxypropan-2-yl)-2H-indazol-5-yl]-6-methylpyridin-2-carboxamid
20) N-[2-(3-Hydroxy-3-methylbutyl)-6-(2-hydroxypropan-2-yl)-2H-indazol-5-yl]-6-methylpyridin-2-carboxamid
21) 6-(2-Hydroxypropan-2-yl)-N-[6-(2-hydroxypropan-2-yl)-2-(4,4,4-trifluorbutyl)-2H-indazol-5-yl]pyridin-2-carboxamid

Die Verbindungen der Formel (I) als Bestandteile der erfindungsgemäßen Kombinationen wirken als Inhibitoren der IRAK4 Kinase, und zeigen ein wertvolles pharmakologisches Wirkspektrum.

Daher ist neben den weiter oben genannten ein weiterer Gegenstand der vorliegenden Erfindung die Verwendung der erfindungsgemäßen Kombinationen zur Behandlung und/oder Prophylaxe von Krankheiten bei Menschen und Tieren.

Die Behandlung und/oder Prophylaxe von Krankheiten, die durch unkontrolliertes Zellwachstum, Zellvermehrung und/oder Zellüberleben, eine unangemessene zelluläre Immunantwort oder eine unangemessene zelluläre inflammatorische Reaktion verursacht werden, wie z.B. hämatologische Tumore, ein solider Tumor und/oder Metastasen davon, z.B. Leukämien und myelodysplastische Syndrome, maligne Lymphome, Kopf- und Genicktumore inklusive Gehirntumore und -metastasen, Tumore des Thorax inklusive nicht-kleinzellige und kleinzellige Lungentumore, Gastrointestinaltumore, endokrine Tumore, Mamma- und andere gynäkologische Tumore, urologische Tumore inklusive Nieren-, Blasen- und Prostatatumore, Hauttumore und Sarkome und/oder deren Metastasen sind ganz besonders bevorzugt.

Im Weiteren ist Gegenstand der vorliegenden Erfindung auch ein Verfahren zur Behandlung und/oder Prävention von Erkrankungen, insbesondere der zuvor genannten Erkrankungen, unter Verwendung einer wirksamen Menge von mindestens einer der Verbindungen der Formel (I) als Bestandteil der erfindungsgemäßen Kombinationen.

Im Sinne der vorliegenden Erfindung umfasst der Begriff "Behandlung" oder "behandeln" ein Hemmen, Verzögern, Aufhalten, Lindern, Abschwächen, Einschränken, Verringern,

Unterdrücken, Zurückdrängen oder Heilen einer Krankheit, eines Leidens, einer Erkrankung, einer Verletzung oder einer gesundheitlichen Störung, der Entfaltung, des Verlaufs oder des Fortschreitens solcher Zustände und/oder der Symptome solcher Zustände. Der Begriff "Therapie" wird hierbei als Synonym mit dem Begriff "Behandlung" verstanden.

Die Begriffe "Prävention", "Prophylaxe" oder "Vorbeugung" werden im Rahmen der vorliegenden Erfindung synonym verwendet und bezeichnen das Vermeiden oder Vermindern des Risikos, eine Krankheit, ein Leiden, eine Erkrankung, eine Verletzung oder eine gesundheitliche Störung, eine Entfaltung oder ein Fortschreiten solcher Zustände und/oder die Symptome solcher Zustände zu bekommen, zu erfahren, zu erleiden oder zu haben.

Die Behandlung oder die Prävention einer Krankheit, eines Leidens, einer Erkrankung, einer Verletzung oder einer gesundheitlichen Störung können teilweise oder vollständig erfolgen.

Die erfindungsgemäßen Kombinationen können allein oder bei Bedarf in Kombination mit einem oder mehreren anderen pharmazeutischen Mitteln (hierin als "Komponente C" bezeichnet) eingesetzt werden, solange diese Kombination nicht zu unerwünschten und inakzeptablen Nebenwirkungen führt. Weiterer Gegenstand der vorliegenden Erfindung sind daher Arzneimittel, enthaltend eine erfindungsgemäße Kombination und einen oder mehrere weitere Wirkstoffe, insbesondere zur Prophylaxe und Therapie der zuvor genannten Erkrankungen.

Beispielsweise können die erfindungsgemäßen Kombinationen mit bekannten anti-hyperproliferativen, zytostatischen oder zytotoxischen Substanzen zur Behandlung von Krebserkrankungen kombiniert werden. Die Kombination der erfindungsgemäßen Kombinationen mit anderen für die Krebstherapie gebräuchlichen Substanzen oder auch mit der Strahlentherapie ist besonders angezeigt.

Als geeignete Komponente "C" seien die folgenden pharmazeutischen Mittel beispielhaft genannt, ohne dass diese Aufzählung abschliessend wäre:
131l-chTNT, Abarelix, Abirateron, Aclarubicin, Ado-Trastuzumab Emtansin, Afatinib, Aflibercept, Aldesleukin, Alemtuzumab, Alendronsäure, Alitretinoin, Altretamin, Amifostine, Aminoglutethimid, Hexyl-5-Aminolevulinat, Amrubicin, Amsacrin, Anastrozol, Ancestim, Anethole dithiolethione, Angiotensin II, Antithrombin III, Aprepitant, Arcitumomab, Arglabin, Arsentrioxid, Asparaginase, Axitinib, Azacitidin, Belotecan, Bendamustin, Belinostat, Bevacizumab, Bexaroten, Bicalutamid, Bisantren, Bleomycin, Bortezomib, Buserelin, Bosutinib, Brentuximab Vedotin, Busulfan, Cabazitaxel, Cabozantinib, Calciumfolinat, Calciumlevofolinat, Capecitabin, Capromab, Carboplatin, Carfilzomib, Carmofur, Carmustin, Catumaxomab, Celecoxib, Celmoleukin, Ceritinib, Cetuximab, Chlorambucil, Chlormadinon, Chlormethin, Cidofovir, Cinacalcet, Cisplatin, Cladribin, Clodronsäure, Clofarabin, Copanlisib(BAY 80-6946), Crisantaspase, Crizotinib, Cyclophosphamid, Cyproteron, Cytarabin, Dacarbazin, Dactinomycin, Dabrafenib, Dasatinib, Daunorubicin, Decitabin, Degarelix, Denileukin-Diftitox, Denosumab, Depreotid, Deslorelin, Dexrazoxane, Dibrospidiumchlorid, Dianhydrogalactitol, Diclofenac, Docetaxel, Dolasetron, Doxifluridin, Doxorubicin, Doxorubicin + Estron, Dronabinol, Edrecolomab, Elliptiniumacetat, Endostatin, Enocitabin, Enzalutamid, Epirubicin, Epitiostanol, Epoetin-alfa, Epoetin-beta, Epoetin-zeta, Eptaplatin, Eribulin, Erlotinib, Esomeprazol, Estramustin, Etoposid, Everolimus, Exemestan, Fadrozol, Fentanyl, Fluoxymesteron, Floxuridin, Fludarabin, Fluoruracil, Flutamid, Folinsäure, Formestan, Fosaprepitant, Fotemustin, Fulvestrant, Gadobutrol, Gadoteridol, Gadotersäure-Megluminsalz, Gadoversetamid, Gadoxetsäure Dinatriumsalz (Gd-EOB-DTPA Dinatriumsalz), Galliumnitrat, Ganirelix, Gefitinib, Gemcitabin, Gemtuzumab, Glucarpidase, Glutoxim, Goserelin, Granisetron, Granulocyten Kolonie stimulierender Factor (G-CSF), Granulocyten Macrophagen Kolonie stimulierender Factor (GM-CSF), Histamindihydrochlorid, Histrelin, Hydroxycarbamid, I-125-Seeds, Ibandronsäure, Ibritumomab-Tiuxetan, Idarubicin, Ifosfamid, Imatinib, Imiquimod, Improsulfan, Indisetron, Incadronic acid, Ingenolmebutat, Interferon-alfa, Interferon-beta, Interferongamma, lobitridol, lobenguane (1231), lomeprol, Ipilimumab, Irinotecan, Itraconazole, Ixabepilon, Lanreotid, Lansoprazole, Lapatinib, Lasocholine, Lenalidomid, Lentinan, Letrozol, Leuprorelin, Levamisol, Levonorgestrel, Levothyroxin-Natrium, Lipegfilgrastim, Lisurid, Lobaplatin, Lomustin, Lonidamin, Masoprocol, Medroxyprogesteron, Megestrol, Melarsoprol, Melphalan, Mepitiostan, Mercaptopurin, Mesna, Methadon, Methotrexat, Methoxsalen, Methylaminolevulinat, Methylprednisolon, Methyltestosteron, Metirosin, Mifamurtid, Miltefosin, Miriplatin, Mitobronitol, Mitoguazon, Mitolactol, Mitomycin, Mitotan, Mitoxantron, Mogamulizumab, Molgramostim, Mopidamol, Morphinhydrochlorid, Morphinsulfat, Nabilon, Nabiximols, Nafarelin, Naloxon + Pentazocin, Naltrexon, Nartograstim, Nedaplatin, Nelarabin, Neridronsäure, Nivolumabpentetreotid, Nilotinib, Nilutamid, Nimorazol, Nimotuzumab, Nimustin, Nitracrin, Nivolumab, Obinutuzumab, Octreotid, Ofatumumab, Omacetaxin-Mepesuccinat, Omeprazol, Ondansetron, Orgotein, Orilotimod, Oxaliplatin, Oxycodon, Oxymetholon, Ozogamicin, p53-Gentherapie, Paclitaxel, Palladium-103-Seed, Palonosetron, Pamidronsäure, Panitumumab, Pantoprazol, Pazopanib, Pegaspargase, Pembrolizumab, Peg-interferon-alfa-2b, Pemetrexed, Pentostatin, Peplomycin, Perflubutane, Perfosfamid, Pertuzumab, Picibanil, Pilocarpin, Pirarubicin, Pixantron, Plerixafor, Plicamycin, Poliglusam, Polyestradiolphosphat, Polyvinylpyrrolidone + Natriumhyaluronat, Polysaccharid-K, Pomalidomid, Ponatinib, Porfimer-Natrium, Pralatrexat, Prednimustin, Prednison, Procarbazin, Procodazole, Propranolol, Quinagolid, Rabeprazol, Racotumomab, Radium-223-chlorid, Radotinib, Raloxifen, Raltitrexed, Ramosetron, Ramucirumab, Ranimustin, Rasburicase, Razoxan, Refametinib (BAY 86-9766 (RDEA 119)), Regorafenib, Risedronsäure, Rhenium-186 Etidronat, Rituximab, Romidepsin, Romurtid, Roniciclib (BAY 1000394), Samarium (153Sm) lexidronam, Satumomab, Secretin, Sipuleucel-T, Sizofiran, Sobuzoxan, Natriumglycididazol, Sorafenib, Stanozolol, Streptozocin, Sunitinib, Talaporfin, Tamibaroten, Tamoxifen, Tapentadol, Tasonermin, Teceleukin, Technetium (99mTc) Nofetumomab Merpentan, 99mTc-HYNIC-[Tyr3]-octreotid, Tegafur, Tegafur + Gimeracil + Oteracil, Temoporfin, Temozolomid, Temsirolimus, Teniposid, Testosteron, Tetrofosmin, Thalidomid, Thiotepa, Thymalfasin, Thyrotropin alfa, Tioguanin, Tocilizumab, Topotecan, Toremifen, Tositumomab, Trabectedin, Tramadol, Trastuzumab, Treosulfan, Tretinoin, Trifluridine + Tipiracil, Trametinib, Trilostan, Triptorelin, Trofosfamid, Thrombopoietin, Ubenimex, Valrubicin, Vandetanib, Vapreotid, Vatalanib (PTK/ZK), Vemurafenib, Vinblastin, Vincristin, Vindesin, Vinflunin, Vinorelbin, Vismodegib, Vorinostat, Yttrium-90-Glasmikrokugeln, Zinostatin, Zinostatin-Stimalamer, Zoledronsäure, Zorubicin.

Als geeingnete Komponente C sind besonders die Kombinationen mit einem P-TEFb- oder CDK9- Inhibitor angezeigt.

In vielversprechender Weise können die erfindungsgemäßen Kombinationen auch biologische Therapeutika wie Antikörper (z.B. Aflibercept, Alemtuzumab, Bevacizumab, Brentuximumab, Catumaxomab, Cetuximab, Denosumab, Edrecolomab, Gemtuzumab, Ibritumomab, Ipilimumab, Ofatumumab, Panitumumab, Pertuzumab, Rituximab, Tositumumab, Trastuzumab) und rekombinante Proteine enthalten.

Die erfindungsgemäßen Kombinationen können auch in Kombination mit anderen, gegen die Angiogenese gerichteten Therapien positive Effekte erzielen, wie zum Beispiel mit Bevacizumab, Axitinib, Regorafenib, Cediranib, Sorafenib, Sunitinib oder Thalidomid. Kombinationen mit Antihormonen und steroidalen metabolischen Enzyminhibitoren sind wegen ihres günstigen Nebenwirkungsprofils besonders geeignet.

Generell können mit der Kombination der erfindungsgemäßen Kombinationen mit anderen, zytostatisch oder zytotoxisch wirksamen Agentien folgende Ziele verfolgt werden:
- eine verbesserte Wirksamkeit bei der Verlangsamung des Wachstums eines Tumors, bei der Reduktion seiner Größe oder sogar bei seiner völligen Eliminierung im Vergleich zu einer Behandlung mit einem einzelnen Wirkstoff;
- die Möglichkeit, die verwendeten Chemotherapeutika in geringerer Dosierung als bei der Monotherapie einzusetzen;
- die Möglichkeit einer verträglicheren Therapie mit weniger Nebeneffekten im Vergleich zur Einzelgabe;
- die Möglichkeit zur Behandlung eines breiteren Spektrums von Tumorerkrankungen;
- das Erreichen einer höheren Ansprechrate auf die Therapie;
- eine längere Überlebenszeit der Patienten im Vergleich zur heutigen Standardtherapie.

Darüber hinaus können die erfindungsgemäßen Kombinationen auch in Verbindung mit einer Strahlentherapie und/oder einer chirurgischen Intervention eingesetzt werden.

Die erfindungsgemäßen Kombinationen können systemisch und/oder lokal wirken. Zu diesem Zweck können sie auf geeignete Weise appliziert werden, wie z.B. oral, parenteral, pulmonal, nasal, sublingual, lingual, buccal, rectal, dermal, transdermal, conjunctival, über das Ohr oder als Implantat bzw. Stent.

Für diese Applikationswege können die erfindungsgemäßen Kombinationen in geeigneten Applikationsformen verabreicht werden.

Für die orale Applikation eignen sich nach dem Stand der Technik funktionierende, die erfindungsgemäßen Kombinationen schnell und/oder modifiziert abgebende Applikationsformen, die die erfindungsgemäßen Kombinationen in kristalliner und/oder amorphisierter und/oder gelöster Form enthalten, wie z.B. Tabletten (nicht-überzogene oder überzogene Tabletten, beispielsweise mit magensaftresistenten oder sich verzögert auflösenden oder unlöslichen Überzügen, die die Freisetzung der erfindungsgemäßen Kombinationen kontrollieren), in der Mundhöhle schnell zerfallende Tabletten oder Filme/Oblaten, Filme/Lyophylisate, Kapseln (beispielsweise Hart- oder Weichgelatinekapseln), Dragees, Granulate, Pellets, Pulver, Emulsionen, Suspensionen, Aerosole oder Lösungen.

Die parenterale Applikation kann unter Umgehung eines Resorptionsschrittes geschehen (z.B. intravenös, intraarteriell, intrakardial, intraspinal oder intralumbal) oder unter Einschaltung einer Resorption (z.B. intramuskulär, subcutan, intracutan, percutan oder intraperitoneal). Für die parenterale Applikation eignen sich als Applikationsformen u.a. Injektions- und Infusionszubereitungen in Form von Lösungen, Suspensionen, Emulsionen, Lyophilisaten oder sterilen Pulvern.

Für die sonstigen Applikationswege eignen sich z.B. Inhalationsarzneiformen (u.a. Pulverinhalatoren, Nebulizer), Nasentropfen, -lösungen oder -sprays, lingual, sublingual oder buccal zu applizierende Tabletten, Filme/Oblaten oder Kapseln, Suppositorien, Ohren- oder Augenpräparationen, Vaginalkapseln, wässrige Suspensionen (Lotionen, Schüttelmixturen), lipophile Suspensionen, Salben, Cremes, transdermale therapeutische Systeme (z.B. Pflaster), Milch, Pasten, Schäume, Streupuder, Implantate oder Stents.

Bevorzugt sind die orale oder parenterale Applikation, insbesondere die orale Applikation.

Die erfindungsgemäßen Kombinationen können in die angeführten Applikationsformen überführt werden. Dies kann in an sich bekannter Weise durch Mischen mit inerten, nichttoxischen, pharmazeutisch geeigneten Hilfsstoffen geschehen. Zu diesen Hilfsstoffen zählen u.a. Trägerstoffe (beispielsweise mikrokristalline Cellulose, Lactose, Mannitol), Lösungsmittel (z.B. flüssige Polyethylenglycole), Emulgatoren und Dispergier- oder Netzmittel (beispielsweise Natriumdodecylsulfat, Polyoxysorbitanoleat), Bindemittel (beispielsweise Polyvinylpyrrolidon), synthetische und natürliche Polymere (beispielsweise Albumin), Stabilisatoren (z.B. Antioxidantien wie beispielsweise Ascorbinsäure), Farbstoffe (z.B. anorganische Pigmente wie beispielsweise Eisenoxide) und Geschmacks- und/oder Geruchskorrigentien.

Ein weiterer Gegenstand der vorliegenden Erfindung sind Arzneimittel, die mindestens eine erfindungsgemäße Verbindung, üblicherweise zusammen mit einem oder mehreren inerten, nicht-toxischen, pharmazeutisch geeigneten Hilfsstoffen enthalten, sowie deren Verwendung zu den zuvor genannten Zwecken.

Im Allgemeinen hat es sich als vorteilhaft erwiesen, bei parenteraler Applikation Mengen von etwa 0.001 bis 1 mg/kg, vorzugsweise etwa 0.01 bis 0.5 mg/kg Körpergewicht zur Erzielung wirksamer Ergebnisse zu verabreichen. Bei oraler Applikation beträgt die Dosierung etwa 0.01 bis 100 mg/kg, vorzugsweise etwa 0.01 bis 20 mg/kg und ganz besonders bevorzugt 0.1 bis 10 mg/kg Körpergewicht.

Trotzdem kann es gegebenenfalls erforderlich sein, von den genannten Mengen abzuweichen, und zwar in Abhängigkeit von Körpergewicht, Applikationsweg, individuellem Verhalten gegenüber dem Wirkstoff, Art der Zubereitung und Zeitpunkt bzw. Intervall, zu welchem die Applikation erfolgt. So kann es in einigen Fällen ausreichend sein, mit weniger als der vorgenannten Mindestmenge auszukommen, während in anderen Fällen die genannte obere Grenze überschritten werden muss. Im Falle der Applikation größerer Mengen kann es empfehlenswert sein, diese in mehreren Einzelgaben über den Tag zu verteilen.

Die nachfolgenden Ausführungsbeispiele erläutern die Erfindung. Die Erfindung ist nicht auf die Beispiele beschränkt.

Die Prozentangaben in den folgenden Tests und Beispielen sind, sofern nicht anders angegeben, Gewichtsprozente; Teile sind Gewichtsteile. Lösungsmittelverhältnisse, Verdünnungsverhältnisse und Konzentrationsangaben von flüssig/flüssig-Lösungen beziehen sich jeweils auf das Volumen.

### Herstellung der Verbindungen der Formel (I) als Bestandteile der erfindungsgemäßen Kombinationen

Die Herstellung der Verbindungen der Formel (I) als Bestandteile der erfindungsgemäßen Kombinationen wird durch die folgenden Syntheseschemata verdeutlicht.

Als Ausgangsmaterial zur Synthese der Verbindungen der Formel (I) als Bestandteile der erfindungsgemäßen Kombinationen werden Carbonsäuren (Intermediat V3) verwendet, welche kommerziell erhältlich sind oder auf literaturbekannten oder analog zu literaturbekannten Wegen (siehe zum Beispiel European Journal of Organic Chemistry 2003, 8, 1559 - 1568, Chemical and Pharmaceutical Bulletin, 1990, 38, 9, 2446 - 2458, Synthetic Communications 2012, 42, 658 - 666, Tetrahedron, 2004 , 60, 51, 11869 - 11874) hergestellt werden können (siehe beispielsweise Syntheseschema 1). Einige Carbonsäuren V3 können ausgehend von Carbonsäureestern (Intermediat V2) durch Verseifung (vergleiche zum Beispiel die Umsetzung von Ethyl-6-(hydroxymethyl)pyridin-2-carboxylat mit wässriger Natriumhydroxidlösung in Methanol, WO200411328) oder - in dem Fall, dass es sich um einen *tert*-Butylester handelt - durch Reaktion mit einer Säure wie zum Beispiel Chlorwasserstoff oder Trifluoressigsäure (vergleiche zum Beispiel Dalton Transactions, 2014 , 43, 19, 7176 - 7190) hergestellt werden. Die Carbonsäuren V3 können auch in Form ihrer Alkalimetallsalze verwendet werden. Die Herstellung der Intermediate V2 kann gegebenenfalls aus den Intermediaten V1 erfolgen, welche als Substituent X¹ ein Chlor, Brom oder lod tragen, durch Umsetzung in einer Kohlenmonoxid-Atmosphäre gegebenenfalls unter Überdruck in Gegenwart eines Phosphinliganden wie zum Beispiel 1,3-Bis(diphenylphoshino)propan, einer Palladium-Verbindung wie zum Beispiel Palladium(II)acetat und einer Base wie zum Beispiel Triethylamin unter Zusatz von Ethanol oder Methanol in einem Lösemittel wie zum Beispiel Dimethylsulfoxid (für Herstellungsmethoden vergleiche zum Beispiel WO2012112743, WO 2005082866, Chemical Communications (Cambridge, England), 2003, 15, 1948 - 1949, WO200661715). Die Intermediate V1 sind entweder kommerziell erhältlich oder können auf literaturbekannten Wegen hergestellt werden. Beispielhafte Herstellungsmethoden sind in WO 2012061926, European Journal of Organic Chemistry, 2002, 2, 327 - 330, Synthesis, 2004, 10, 1619 - 1624, Journal of the American Chemical Society, 2013, 135, 32, 12122 - 12134, Bioorganic and Medicinal Chemistry Letters, 2014, 24, 16, 4039-4043, US2007185058, WO2009117421 aufgeführt. 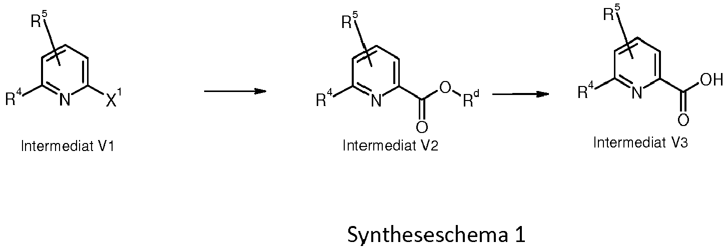 X¹ bedeutet Chlor, Brom oder lod.

R^{d} bedeutet Methyl, Ethyl, Benzyl oder tert-Butyl.
R⁴, R⁵ haben die in der allgemeinen Formel (I) beschriebenen Definitionen.

Methyl-5-amino-1H-indazol-6-carboxylat (Intermediat 2) kann ausgehend von Methyl-1H-indazol-6-carboxylat (Intermediat 0) gemäß Syntheseschema 2 durch Nitrierung und Reduktion der Nitrogruppe von Intermediat 1 durch Wasserstoff in Gegenwart von Palladium auf Kohle analog zu *Ohrai, Kazuhiko Chiba* WO 2008/001883 erhalten werden. Zur Herstellung der Intermediate 3 ausgehend vom Intermediat 2 können verschiedene in der Literatur bekannte Kupplungsreagenzien eingesetzt werden (Amino Acids, Peptides and Proteins in Organic Chemistry. Vol.3 - Building Blocks, Catalysis and Coupling Chemistry, Andrew B. Hughes, Wiley, Kapitel 12 - Peptide-Coupling Reagents, 407-442; Chem. Soc. Rev., 2009, 38, 606). Beispielsweise können 1-(3-Dimethylaminopropyl)-3-ethylcarbodiimidhydrochlorid in Kombination mit 1-Hydroxy-1H-benzotriazol Hydrat (HOBt, WO2012107475; Bioorg. Med. Chem. Lett., 2008 , 18, 2093), (1H-Benzotriazol-1-yloxy)(dimethylamino)-N,N-dimethylmethaniminiumtetrafluoroborat (TBTU, CAS 125700-67-6), (Dimethylamino)-N,N-dimethyl(3H-[1,2,3]triazolo[4,5-b]pyridin-3-yloxy)methane-iminiumhexafluorophosphat (HATU, CAS 148893-10-1), Propanphosphonsäureanhydrid (als Lösung in Ethylacetat oder DMF, CAS68957-94-8) oder Di-1H-imidazol-1-ylmethanon (CDI) als Kupplungsreagenzien verwendet werden, wobei jeweils zur Reaktionsmischung noch eine Base wie Triethylamin oder N-Ethyl-N-isopropylpropan-2-amin gegeben wird. Bevorzugt ist die Verwendung von TBTU und N-Ethyl-N-isopropylpropan-2-amin in THF.

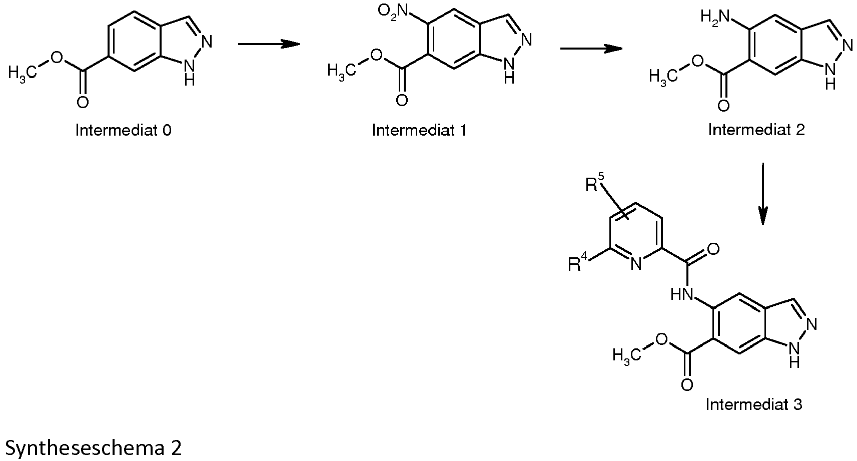

Die Substituenten R⁴, R⁵ haben die in der allgemeinen Formel (I) angegebenen Definitionen.

Ausgehend von den Intermediaten 3 können 2-substituierte Indazolderivate (Intermediate 4) hergestellt werden (siehe Syntheseschema 3). Hierfür kommen Reaktionen mit gegebenenfalls substituierten Alkylchloriden, Alkylbromiden, Alkyliodiden oder Alkyl-4-methylbenzolsulfonaten in Betracht. Die verwendeten Alkylhalogenide oder Alkyl-4-methylbenzolsulfonate sind kommerziell erhältlich oder können analog zu literaturbekannten Wegen hergestellt werden (für die Herstellung von Alkyl-4-methylbenzolsulfonaten sei zum Beispiel die Umsetzung eines entsprechenden Alkoholes mit 4-Methylbenzolsulfonylchlorid in Gegenwart von Triethylamin oder Pyridin genannt, siehe zum Beispiel Bioorganic and Medicinal Chemistry, 2006, 14, 12 4277 - 4294). Gegebenenfalls kann bei der Verwendung von Alkylchloriden oder Alkylbromiden noch ein Alkalimetalliodid zugegeben werden wie Kaliumiodid oder Natriumiodid. Als Basen können beispielsweise Kaliumcarbonat, Cäsiumcarbonat oder Natriumhydrid verwendet werden. Bei reaktiven Alkylhalogeniden kann in einigen Fällen auch N-Cyclohexyl-N-methylcyclohexanamin zur Verwendung kommen. Als Lösemittel kommen zum Beispiel 1-Methylpyrrolidin-2-on, DMF, DMSO oder THF in Betracht. Gegebenenfalls können die verwendeten Alkylhalogenide oder Alkyl-4-methylbenzolsulfonate funktionelle Gruppen aufweisen, die vorher gegebenenfalls mit einer Schutzgruppe geschützt wurden (vergleiche auch P. G. M. Wuts, T. W. Greene, Greene's Protective Groups in Organic Synthesis, Fourth Edition, ISBN: 9780471697541). Kommen beispielsweise Alkylhalogenide oder Alkyl-4-methylbenzolsulfonate zum Einsatz, die eine oder mehrere Hydroxygruppen aufweisen, so können diese Hydroxygruppen gegebenenfalls durch eine *tert*-Butyl(dimethyl)silyl-Gruppe oder eine ähnliche dem Fachmann geläufige Siliciumhaltige Schutzgruppe geschützt vorliegen. Alternativ können die Hydroxygruppen aber auch mit der Tetrahydro-2H-pyran (THP)-Gruppe oder mit der Acetyl oder Benzoylgruppe geschützt vorliegen. Die verwendeten Schutzgruppen können dann nachfolgend der Synthese von Intermediat 4, aber auch nach der Synthese von (I) abgespalten werden. Wird zum Beispiel eine *tert*-Butyl(dimethylsilyl)gruppe als Schutzgruppe verwendet, so kann diese unter Verwendung von Tetrabutylammoniumfluorid in einem Lösemittel wie zum Beispiel THF abgespalten werden. Eine THP-Schutzgruppe kann zum Beispiel unter Verwendung von 4-Methylbenzolsulfonsäure (ggf. als Monohydrat) abgespalten werden. Acetylgruppen oder Benzoylgruppen können durch Behandlung mit wässriger Natronlauge abgespalten werden.

Gegebenenfalls können die verwendeten Alkylhalogenide oder Alkyl-4-methylbenzolsulfonate funktionelle Gruppen enthalten, die durch dem Fachmann bekannte Oxidations- oder Reduktionsreaktionen umgesetzt werden können (vergleiche zum Beispiel Science of Synthesis, Georg Thieme Verlag). Handelt es sich beispielsweise bei der funktionellen Gruppe um eine Sulfidgruppe, so kann diese durch literaturbekannte Methoden zu einer Sulfoxid oder Sulfongruppe oxidiert werden. Handelt es sich um eine Sulfoxidgruppe so kann diese ebenfalls zu einer Sulfongruppe oxidiert werden. Beispielsweise kann für diese Oxidationsschritte 3-Chloroperbenzoesäure (CAS 937-14-4) verwendet werden (vergleiche hierzu zum Beispiel auch US201094000 für die Oxidation eines 2-(Methylsulfanyl)ethyl-1H-pyrazol-Derivates zu einem 2-(Methylsulfinyl)ethyl-1H-pyrazol-Derivat und die Oxidation eines weiteren 2-(Methylsulfanyl)ethyl-1H-pyrazol-Derivates zu einem 2-(Methylsulfonyl)ethyl-1H-pyrazol-Derivat). Enthalten die verwendeten Alkylhalogenide oder -tosylate eine Ketogruppe, so kann diese durch dem Fachmann bekannte Reduktionsmethoden zu einer Alkoholgruppe reduziert werden (vergleiche zum Beispiel Chemische Berichte, 1980, 113, 1907 - 1920 für die Verwendung von Natriumborhydrid). Diese Oxidations- oder Reduktionsschritte können nachfolgend der Synthese von Intermediat 4, aber auch nach der Synthese der Verbindungen der allgemeinen Formel (I) erfolgen. Alternativ kann die Herstellung von Intermediat 4 durch Mitsunobu Reaktion (siehe zum Beispiel K. C. K. Swamy et. al. Chem. Rev. 2009, 109, 2551 - 2651) von Intermediat 3 mit gegebenenfalls substituierten Alkylalkoholen erfolgen. Verschiedene Phosphine wie Triphenylphosphin, Tributylphosphin oder 1,2-Diphenylphosphinoethan in Kombination mit Azodicarbonsäurediisopropylester (CAS 2446-83-5) oder weiteren in der Literatur genannten Diazenderivaten (K. C. K. Swamy et. al. Chem. Rev. 2009, 109, 2551 - 2651) können benutzt werden. Bevorzugt ist die Verwendung von Triphenylphosphin und Azodicarbonsäurediisopropylester. Trägt der Alkylalkohol eine funktionelle Gruppe, so können - wie bei den oben genannten Reaktionen mit Alkylhalogeniden - bekannte Schutzgruppenstrategien verwendet werden (weitere Hinweise sind P. G. M. Wuts, T. W. Greene, Greene's Protective Groups in Organic Synthesis, Fourth Edition, ISBN: 9780471697541 zu entnehmen) sowie - wie bei den oben genannten Reaktionen mit Alkylhalogeniden - Oxidations- oder Reduktionsschritte nachfolgend der Synthese von Intermediat 4, aber auch nach der Synthese der Verbindungen der allgemeinen Formel (I) erfolgen. Ausgehend von Intermediat 4 können erfindungsgemäße Verbindungen der allgemeinen Formel (I) mit der Bedeutung R² und R³ = C₁-C₆-Alkyl (wobei R² und R³ dieselbe Bedeutung haben), durch eine Grignard-Reaktion erhalten werden (vergleiche zum Beispiel die Umsetzung eines Methyl-1H-indazol-6-carboxylat-Derivates mit Methylmagnesiumbromid in EP 2489663). Für die Grignard-Reaktion können Alkylmagnesiumhalogenide verwendet werden. Besonders bevorzugt sind Methylmagnesiumchlorid oder Methylmagnesiumbromid in THF oder Diethylether oder auch in Mischungen aus THF und Diethylether. Alternativ können ausgehend von Intermediat 4 erfindungsgemäße Verbindungen der allgemeinen Formel (I) mit der Bedeutung R² und R³ = C₁-C₆-Alkyl (wobei R² und R³ dieselbe Bedeutung haben), durch eine Reaktion mit einem Alkyllithiumreagenz erhalten werden (vergleiche zum Beispiel die Umsetzung eines Methyl-2-amino-4-chlor-1-methyl-1H-benzimidazol-7-carboxylat-Derivates mit Isopropyllithium oder tert-Butyllithiumin WO2006116412). Ausgehend von Intermediat 4 können erfindungsgemäße Verbindungen der allgemeinen Formel (I) mit der Bedeutung R² = H und R³ = H durch Reduktion mit Lithiumaluminiumhydrid in THF, Lithiumborhydrid in THF oder Natriumborhydrid in THF gegebenenfalls unter Zusatz von Methanol oder Mischungen aus Lithiumborhydrid und Natriumborhydrid hergestellt werden. 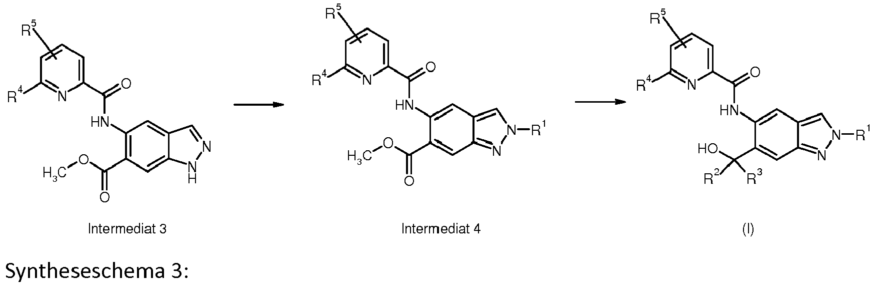 Die Substituenten R¹, R², R³, R⁴, R⁵ haben die in der allgemeinen Formel (I) angegebenen Definitionen.

Ausgehend von Intermediat 3 kann Intermediat 5 mit der Bedeutung R² und R³ = C₁-C₆-Alkyl (wobei R² und R³ dieselbe Bedeutung haben) durch eine Grignard-Reaktion erhalten werden (siehe Syntheseschema 4). Hierfür können geeignete Alkylmagnesiumhalogenide wie zum Beispiel Methylmagnesiumchlorid oder Methylmagnesiumbromid in THF oder in Diethylether oder auch in Mischungen aus THF und Diethylether verwendet werden.
Ausgehend von Intermediat 5 kann dann eine Teilmenge (I-a) mit der Bedeutung R² und R³ = C₁-C₆-Alkyl (wobei R² und R³ dieselbe Bedeutung haben) der Verbindungen (I) hergestellt werden. Hierfür kommen analog zu Syntheseschema 3 (Herstellung von Intermediat 3) Reaktionen von Intermediat 5 mit gegebenenfalls substituierten Alkylchloriden, Alkylbromiden, Alkyliodiden oder Alkyl-4-methylbenzolsulfonaten in Betracht. Es können Schutzgruppenstrategien analog zu den in Syntheseschema 3 beschriebenen verwendet werden.
Alternativ kann zur Herstellung einer Teilmenge (I-a) mit der Bedeutung R² und R³ = C₁-C₆-Alkyl (wobei R² und R³ dieselbe Bedeutung haben) der Verbindungen (I) die Mitsunobu Reaktion von Intermediat 5 mit gegebenenfalls substituierten Alkylalkoholen verwendet werden (analog zu Syntheseschema 3).
Beinhaltet R¹ der Verbindungen der Formel (I-a) eine geeignete funktionelle Gruppe, so können gegebenenfalls nachfolgend in Analogie zu Syntheseschema 3 Oxidations- bzw.

Reduktionsreaktionen verwendet werden zur Herstellung weiterer erfindungsgemäßer Verbindungen. 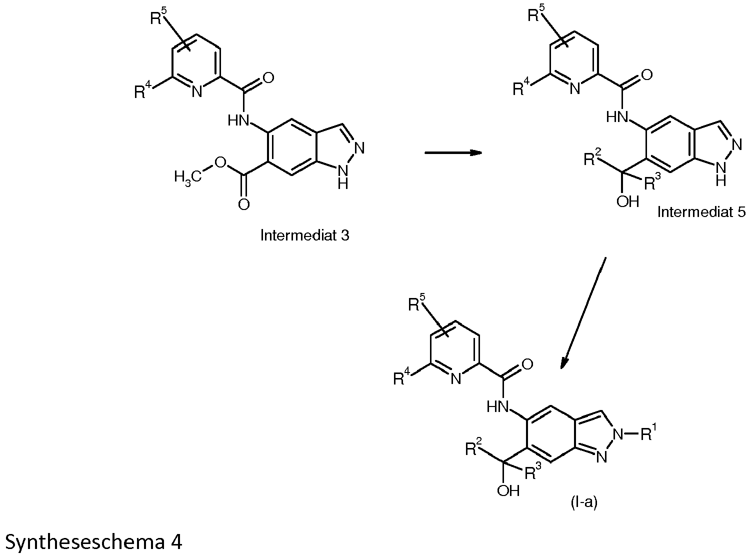 Die Substituenten R¹, R⁴, R⁵ haben die in der allgemeinen Formel (I) angegebenen Definitionen. R² und R³ haben immer dieselbe Bedeutung und stehen gleichzeitig für C₁-C₆-Alkyl.

Ausgehend von Intermediat 1 kann Intermediat 4 auf alternative Weise hergestellt werden (siehe Syntheseschema 5). Zuerst wird Intermediat 1 mit Methoden wie bei Syntheseschema 3 (Herstellung von Intermediat 4 aus Intermediat 3) beschrieben zu Intermediat 6 umgesetzt.
Das Intermediat 6 kann dann durch Reduktion der Nitrogruppe zu Intermediat 7 umgesetzt werden. Beispielsweise kann die Nitrogruppe mit Palladium auf Kohlenstoff unter einer Wasserstoffatmosphäre reduziert werden (vergleiche zum Beispiel WO2013174744 für die Reduktion von 6-Isopropoxy-5-nitro-1H-indazol zu 6-Isopropoxy-1H-indazol-5-amin) oder durch die Verwendung von Eisen und Ammoniumchlorid in Wasser und Ethanol (siehe zum Beispiel auch Journal of the Chemical Society, 1955, 2412 -2419), oder durch die Verwendung von Zinn(II)-chlorid (CAS 7772-99-8). Die Verwendung von Eisen und Ammoniumchlorid in Wasser und Ethanol ist bevorzugt. Die Herstellung von Intermediat 4 aus Intermediat 7 kann analog zu Syntheseschema 2 (Herstellung von Intermediat 3 aus Intermediat 2) erfolgen.

Wie bei Syntheseschema 3 beschrieben, können gegebenenfalls auch bei Syntheseschema 5 Schutzgruppenstrategien verwendet werden. Gegebenenfalls können zusätzlich ausgehend von Intermediat 6 bzw. Intermediat 7 wie bei Syntheseschema 3 beschrieben dem Fachmann bekannte Oxidation- oder Reduktionsreaktionen durchgeführt werden (vergleiche zum Beispiel Science of Synthesis, Georg Thieme Verlag). 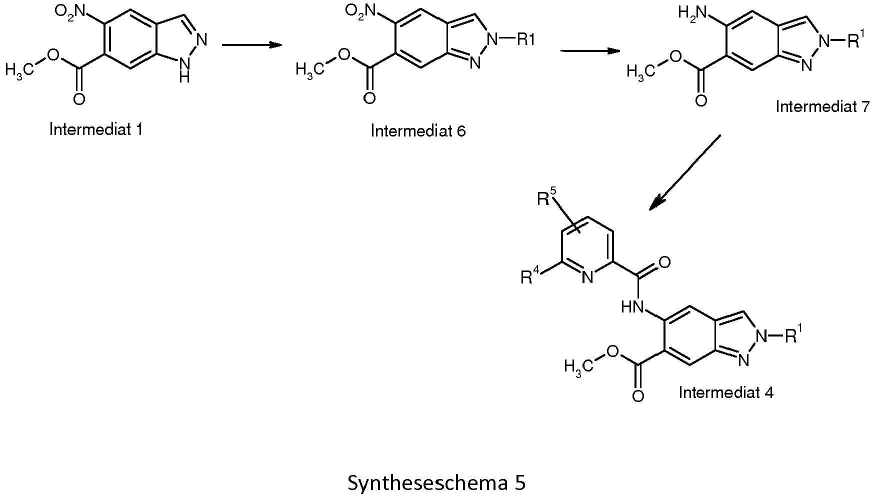 Die Substituenten R1, R4, R5 haben die in der allgemeinen Formel (I) angegebenen Definitionen.

### Synthese der Beispielverbindungen der Formel (I) als Bestandteile der erfindungsgemäßen Kombinationen

### Abkürzungen und Erläuterungen

| | |
|---|---|
| DMF | *N,N*-Dimethylformamid |
| DMSO | Dimethylsulfoxid |
| THF | Tetrahydrofuran |
| RT | Raumtemperatur |
| d. Th. | Der Theorie |
| HPLC | Hochleistungsflüssigkeitschromatographie |
| H | Stunde(n) |
| Min | Minute(n) |
| UPLC | Ultra-Hochleistungsflüssigchromatographie |
| DAD | Diodenarraydetektor |
| ELSD | Verdampfungslichtstreudetektor |
| ESI | Elektronenspray-lonisation |
| SQD | Single Quadrupole Detektor |
| KPG | Kerngezogenes Präzisions-Glasgerät |

Der Begriff *Kochsalzlösung* bedeutet eine gesättigte wässrige Natriumchloridlösung

Die chemische Bezeichnung der Intermediate und Beispiele wurde unter Verwendung der Software von ACD / LABS (Batch Version 12.01.) erstellt.

### Methoden

In einigen Fällen wurden die Verbindungen sowie deren Vor- und/ oder Zwischenstufen durch LC-MS analysiert. :
Methode A1: UPLC (MeCN-HCOOH):
   Instrument: Waters Acquity UPLC-MS SQD 3001; Säule: Acquity UPLC BEH C18 1.7 50x2.1mm; Eluent A: Wasser + 0.1% Vol. Ameisensäure (99%), Eluent B: Acetonitril; Gradient: 0-1.6 min 1-99% B, 1.6-2.0 min 99% B; Fluss 0.8 ml/min; Temperatur: 60 °C.; Injektion: 2 µl; DAD scan: 210-400 nm; MS ESI+, ESI-, scan range 160-1000 m/z; ELSD.
Methode A2: UPLC (MeCN-NH₃):
   Instrument: Waters Acquity UPLC-MS SQD 3001; Säule: Acquity UPLC BEH C18 1.7 50x2.1mm; Eluent A: Wasser + 0.2% Vol. Ammoniak (32%), Eluent B: Acetonitril; Gradient: 0-1.6 min 1-99% B, 1.6-2.0 min 99% B; Fluss 0.8 ml/min; Temperatur: 60 °C; Injektion: 2 µl; DAD scan: 210-400 nm; MS ESI+, ESI-, scan range 160-1000 m/z; ELSD.
Methode A3: (LC-MS)
   Instrument: Agilent 1290 Infinity LC; Säule: Acquity UPLC BEH C18 1.7 50x2.1mm; Eluent A: Wasser + 0.05% Vol. Ameisensäure, Eluent B: Acetonitril + 0.05% Vol. Ameisensäure; Gradient: 0-1.7 min 2-90% B, 1.7-2.0 min 90% B; Fluss 1.2 ml/min; Temperatur: 60 °C; Injektion: 2 µl; DAD scan: 190-390 nm; MS: Agilent TOF 6230.
Methode A4: (LC-MS)
   Instrument: Waters Acquity; Säule: Kinetex (Phenomenex), 50x2 mm; Eluent A: Wasser + 0.05% Vol. Ameisensäure, Eluent B: Acetonitril + 0.05% Vol. Ameisensäure; Gradient: 0-1.9 min 1-99% B, 1.9-2.1 min 99% B; Fluss 1.5 ml/min; Temperatur: 60 °C; Injektion: 0.5 µl; DAD scan: 200 -400 nm.

In einigen Fällen wurden die Verbindungen der Formel (I) als Bestandteile der erfindungsgemäßen Kombinationen sowie deren Vor- und/ oder Zwischenstufen mit folgenden präparativen HPLC-Methoden gereinigt:
Methode P1: System: Waters Autopurificationsystem: Pump 2545, Sample Manager 2767, CFO, DAD 2996, ELSD 2424, SQD; Säule: XBrigde C18 5µm 100x30 mm; Eluent: A: Wasser + 0.1% Vol. Ameisensäure, Eluent B: Acetonitril; Gradient: 0-8 min 10-100% B, 8-10 min 100% B; Flow: 50 mL/min; Temperatur: Raumtemperatur; Lösung: Max. 250 mg / max. 2.5 mL DMSO o. DMF; Injektion: 1 x 2.5 mL; Detection: DAD scan range 210-400 nm; MS ESI+, ESI-, scan range 160-1000 m/z.
Methode P2: System: Waters Autopurificationsystem: Pump 254, Sample Manager 2767, CFO, DAD 2996, ELSD 2424, SQD 3100; Säule: XBrigde C18 5µm 100x30 mm; Eluent: A: Wasser + 0.2% Vol. Ammoniak (32%), Eluent B: Methanol; Gradient: 0-8 min 30-70% B; Fluss: 50 mL/min; Temperatur: Raumtemperatur; Detektion: DAD scan range 210-400 nm; MS ESI+, ESI-, scan range 160-1000 m/z; ELSD.
Methode P3: System: Labomatic, Pump: HD-5000, Fraction Collector: LABOCOL Vario-4000, UV-Detektor: Knauer UVD 2.1S; Säule: XBrigde C18 5µm 100x30 mm; Eluent A: Wasser+ 0.2% Vol. Ammoniak (25%), Eluent B: Acetonitril; Gradient: 0-1 min 15% B, 1-6,3 min 15-55% B, 6,3-6,4min 55-100% B, 6,4-7,4min 100% B; Flow: 60 mL/min; Temperatur: Raumtemperatur; Lösung: Max. 250 mg / 2mL DMSO; Injektion: 2 x 2mL; Detection: UV 218 nm; Software: SCPA PrepCon5.
Methode P4: System: Labomatic, Pump: HD-5000, Fraction Collector: LABOCOL Vario-4000, UV-Detektor: Knauer UVD 2.1S; Säule: Chromatorex RP C18 10µm 125x30 mm, Eluent: A: Wasser + 0.1% Vol. Ameisensäure, Eluent B: Acetonitril; Gradient: 0-15 min 65 - 100% B; Fluss: 60 mL/min; Temperatur: Raumtemperatur; Lösung: Max. 250 mg / 2mL DMSO; Injektion: 2 x 2mL; Detection: UV 254 nm; Software: SCPA PrepCon5.
Methode P5: System: Sepiatec: Prep SFC100, Säule: Chiralpak IA 5µm 250x20 mm; Eluent A: Kohlendioxid, Eluent B: Ethanol; Gradient: isokratisch 20% B; Fluss: 80 mL/min; Temperatur: 40°C; Lösung: Max. 250 mg / 2mL DMSO; Injektion: 5 x 0.4 mL Detektion: UV 254 nm
Methode P6: System: Agilent: Prep 1200, 2xPrep Pump, DLA, MWD, Gilson: Liquid Handler 215; Säule: Chiralcel OJ-H 5µm 250x20 mm; Eluent A: Hexan, Eluent B: Ethanol; Gradient: isokratisch 30% B; Fluss: 25 mL/min; Temperatur: 25°C; Lösung: 187 mg / 8 mL Ethanol/Methanol; Injektion: 8 x 1.0 mL Detektion: UV 280 nm
Methode P7: System: Labomatic, Pump: HD-5000, Fraction Collector: LABOCOL Vario-4000, UV-Detektor: Knauer UVD 2.1S; Säule: XBrigde C18 5µm 100x30 mm; Eluent A: Wasser + 0.1% Vol. Ameisensäure, Eluent B: Acetonitril; Gradient: 0-3min: 65%B isokratisch, 3-13min: 65-100% B; Fluss: 60ml/min; Temperatur: Raumtemperatur; Lösung: Max. 250 mg / 2mL DMSO; Injektion: 2 x 2mL; Detektion: UV 254 nm.
Methode P8: System: Agilent: Prep 1200, 2xPrep Pump, DLA, MWD, Gilson: Liquid Handler 215; Säule: Chiralpak IF 5µm 250x20 mm; Eluent A: Ethanol, Eluent B: Methanol; Gradient: isokratisch 50% B; Fluss: 25 mL/min; Temperatur: 25°C; Lösung: 600 mg / 7 mL N,N-Dimethylformamid; Injektion: 10 x 0.7 mL Detektion: UV 254 nm

In einigen Fällen erfolgte die Aufreinigung von Substanzgemischen durch Säulenchromatographie an Kieselgel.

Zur Herstellung der Verbindungen der Formel (I) als Bestandteile der erfindungsgemäßen Kombinationen sowie deren Vorstufen und/ oder Zwischenstufen wurde eine säulenchromatographische Reinigung ("Flash-Chromatographie") an Kieselgel durchgeführt unter Verwendung von Geräten Isolera^{®} der Firma Biotage. Hierbei kamen Kartuschen der Firma Biotage wie zum Beispiel die Kartusche "SNAP Cartridge, KP_SIL" unterschiedlicher Größe sowie Kartuschen "Interchim Puriflash Silica HP 15UM flash column" der Firma Interchim unterschiedlicher Größe zum Einsatz.

### Ausgangsverbindungen

### Intermediat V2-1

### Methyl-6-(2-hydroxypropan-2-yl)pyridin-2-carboxylat

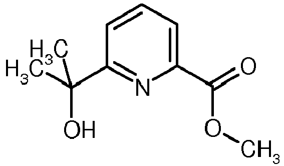

2.00 g (9.26 mmol) 2-(6-Bromopyridin-2-yl)propan-2-ol (CAS 638218-78-7) wurden in 20 ml Methanol und 20 ml DMSO gelöst. Anschließend wurden 250 mg 1,3-Bis(diphenyl-phoshino)propan, 130 mg Palladium(II)acetat und 3 ml Triethylamin zugegeben. Das Reaktionsgemisch wurde bei Raumtemperatur dreimal mit Kohlenmonoxid gespült und unter 13 bar Kohlenmonoxidatmosphäre 30 min gerührt. Man entfernte die Kohlenmonoxidatmosphäre durch Anlegen eines Vakuums und rührte unter 14 bar Kohlenmonoxidatmosphäre bei 100°C 24 h. Man entspannte den Autoklaven, versetzte die Reaktionsmischung mit Wasser, extrahierte dreimal mit Ethylacetat, wusch mit gesättigter wässriger Natriumhydrogencarbonatlösung, Kochsalzlösung, filtrierte über einen wasserabweisenden Filter und engte ein. Man erhielt 1.60 g eines Rohproduktes.

UPLC-MS (Methode A1): Rₜ = 0.76 min (UV Detektor: TIC), gefundene Masse 195.00.

### Intermediat V3-1

### Kalium-6-(2-hydroxypropan-2-yl)pyridin-2-carboxylat

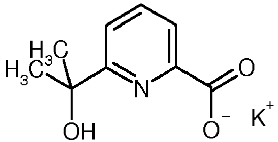

1.60 g des Rohproduktes Intermediat 0-1 wurden in 15 ml Methanol vorgelegt, man addierte 0.74 g Kaliumhydroxid und rührte 16.5 h bei 50°C. Nach Einengen erhielt man 2.1 g eines Rückstandes, welcher ohne weitere Reinigung eingesetzt wurde.
UPLC-MS (Methode A1): Rₜ = 0.47 min (UV Detektor: TIC), gefundene Masse 181.00.

### Intermediat 1-1

### Methyl-5-nitro-1H-indazol-6-carboxylat

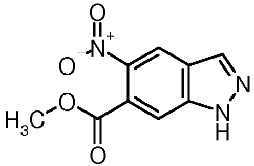

4.60 g (26.1 mmol) Methyl-1H-indazol-6-carboxylat (CAS Nr: 170487-40-8) wurden in 120 ml Schwefelsäure (96%ig) gelöst und in einem Dreihalskolben mit KPG-Rührer, Tropftrichter und Innenthermometer auf -15°C gekühlt. Zu dieser Lösung wurde innerhalb von 15 min die vorher hergestellte und gekühlte Nitriersäure (10 ml Schwefelsäure 96%ig in 5 ml Salpetersäure 65%ig) zugetropft. Nach Beendigung des Zutropfens wurde 1 h nachgerührt (Innentemperatur bei -13°C). Die Reaktionsmischung wurde auf Eis gegeben, der Niederschlag abgesaugt, mit Wasser gewaschen und im Trockenschrank bei 50°C im Vakuum getrocknet. Es wurden 5.49 g der Titelverbindung erhalten.
UPLC-MS (Methode A2): Rₜ = 0.75 min
MS (ESIpos): m/z = 222(M+H)⁺
¹H NMR (400 MHz, DMSO-d6): 6 [ppm] = 3.87 (s, 3 H), 7.96 (s, 1 H), 8.44 (s, 1 H), 8.70 (s, 1 H), 13.98 (br. s., 1 H).

### Intermediat 2-1

### Methyl-5-amino-1H-indazol-6-carboxylat

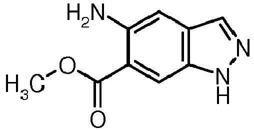

4.40 g (19.8 mmol) Methyl-5-nitro-1H-indazol-6-carboxylat (Intermediat 1-1) wurden in 236 ml Methanol gelöst und mit 1.06 g (0.99 mmol) Palladium auf Aktivkohle 3 h bei 25°C unter Normaldruck Wasserstoff hydriert. Das Reaktionsgemisch wurde über Celite filtriert, der Filter mit Methanol gewaschen und das Filtrat eingeengt. Es wurden 3.53 g der Titelverbindung erhalten.
¹H NMR (300 MHz, DMSO-d6): 6 [ppm] = 3.85 (s, 3 H) 6.01 (s, 2 H) 6.98 (s, 1 H) 7.79 - 7.91 (m, 1 H) 7.99 (s, 1 H) 12.84 (br. s., 1 H).

### Intermediat 3-1

### Methyl-5-({[6-(trifluormethyl)pyridin-2-yl]carbonyl}amino)-1H-indazol-6-carboxylat

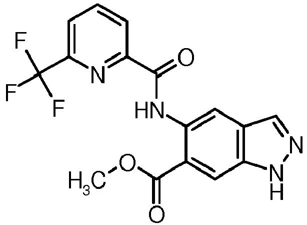

4.95 g (25.9 mmol) 6-(Trifluormethyl)pyridin-2-carbonsäure wurden in 45 ml THF vorgelegt. Man addierte 9.07 g (28.2 mmol) O-(Benzotriazol-1-yl)-N,N,N',N'-tetramethyluroniumtetrafluoroborat und 4.92 ml (28.2 mmol) N-Ethyl-N-isopropylpropan-2-amin und rührte 30 min bei 25°C. Anschließend wurden 4.50 g (23.5 mmol) Methyl-5-amino-1H-indazol-6-carboxylat (Intermediat 2-1) hinzugegeben und 24 h bei 25°C gerührt. Die Reaktionsmischung wurde über einen Membranfilter abgesaugt und mit THF und mit Wasser gewaschen und über Nacht im Trockenschrank getrocknet. Es wurden 7.60 g der Titelverbindung erhalten.
UPLC-MS (Methode A2): Rₜ = 1.16 min
MS (ESIpos): m/z = 365 (M+H)⁺
¹H NMR (400 MHz, DMSO-d6): 6 [ppm] = 3.97 (s, 3 H), 8.13 - 8.27 (m, 2 H), 8.30 (s, 1 H), 8.33 - 8.45 (m, 1 H), 8.45 - 8.51 (m, 1 H), 9.15 (s, 1 H), 12.57 (s, 1 H), 13.44 (s, 1 H).

### Intermediat 3-2

### Methyl-5-({[6-(difluormethyl)pyridin-2-yl]carbonyl}amino)-1H-indazol-6-carboxylat

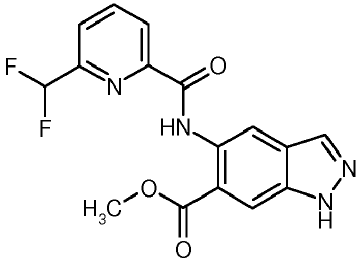

2.85 g (23.5 mmol) 6-(Difluormethyl)pyridin-2-carbonsäure wurden in 30 ml THF vorgelegt. Man addierte 6.05 g (18.8 mmol) O-(Benzotriazol-1-yl)-N,N,N',N'-tetramethyluroniumtetrafluoroborat und 3.3 ml N-Ethyl-N-isopropylpropan-2-amin und rührte 10 Minuten bei Raumtemperatur. Anschließend wurden 3.00 g (15.7 mmol) Methyl-5-amino-1H-indazol-6-carboxylat hinzugegeben und über Nacht bei Raumtemperatur gerührt. Das Reaktionsgemisch wurde mit Wasser versetzt, der Niederschlag wurde abgesaugt und mit Wasser und Dichlormethan mehrfach gewaschen. Man erhielt 1.53 g (27% d. Th.) der Titelverbindung. Man trennte die Phasen des Filtrates, engte die organische Phase ein, versetzte mit wenig Dichlormethan, suspendierte im Ultraschallbad und saugte den Niederschlag ab. Man erhielt weitere 1.03 g der Titelverbindung.
1H-NMR (erste Produktfraktion, 300MHz, DMSO-d6): 6 [ppm]= 3.99 (s, 3H), 7.09 (t, 1H), 8.00 (d, 1H), 8.21 - 8.40 (m, 4H), 9.14 (s, 1H), 12.53 (s, 1H), 13.44 (s, 1H).

### Intermediat 3-3

### Methyl-5-({[6-(2-hydroxypropan-2-yl)pyridin-2-yl]carbonyl}amino)-1H-indazol-6-carboxylat

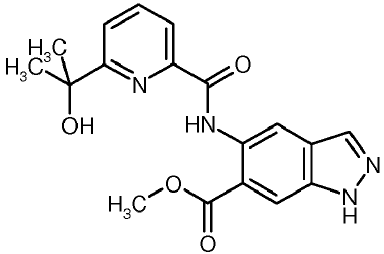

2.10 g Kalium-6-(2-hydroxypropan-2-yl)pyridin-2-carboxylat (Intermediat V3-1) wurden in 15 ml THF vorgelegt. Man addierte 3.69 g (11.5 mmol) O-(Benzotriazol-1-yl)-N,N,N',N'-tetramethyluroniumtetrafluoroborat und 2.00 ml N-Ethyl-N-isopropylpropan-2-amin und rührte 15 min bei Raumtemperatur. Anschließend wurden 1.83 g (9.58 mmol) Methyl-5-amino-1H-indazol-6-carboxylat (Intermediat 2-1) hinzugegeben und 19 h bei Raumtemperatur gerührt. Man versetzte mit Wasser und Ethylacetat, filtrierte den ungelösten Feststoff ab, trennte die Phasen des Filtrates, extrahierte die wässrige Phase zweimal mit Ethylacetat, wusch mit Kochsalzlösung, filtrierte über einen wasserabweisenden Filter, engte ein und reinigte säulenchromatographisch an Kieselgel (Hexan/Ethylacetat). Nach Entfernung der Lösungsmittel wurden 1.56 g der Titelverbindung als gelber Schaum erhalten.
UPLC-MS (Methode A1): Rt = 1.00 min (UV Detektor: TIC Smooth), gefundene Masse 354.00.
1H-NMR (500MHz,DMSO-d6): 6 [ppm] = 1.63 (s, 6H), 3.97 (s, 3H), 5.37(s,1H), 7.90 - 7.95 (m, 1H), 8.03-8.07 (m, 2H), 8.23(s, 1H),8.29 (s, 1H), 9.19 (s, 1H), 12.79 (s, 1H), 13.41 (br.s., 1H).

### Intermediat 4-1

### Methyl-2-(oxetan-3-ylmethyl)-5-({[6-(trifluormethyl)pyridin-2-yl]carbonyl}amino)-2H-indazol-6-carboxylat

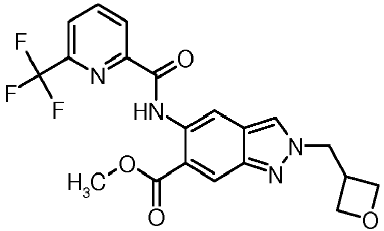

1.00 g (2.66 mmol) Methyl-5-({[6-(trifluormethyl)pyridin-2-yl]carbonyl}amino)-1H-indazol-6-carboxylat (Intermediat 3-1) wurden in 10 ml DMF gelöst und nach Zugabe von 1.10 g (7.99 mmol) Kaliumcarbonat und 221 mg (1.33 mmol) Kaliumiodid 30 min bei 25°C gerührt. Es wurden 603 mg (3.99 mmol) 3-Brommethyl-oxetan zugegeben und 24 h bei 25°C gerührt. Die Reaktionsmischung wurde zwischen Wasser und Ethylacetat verteilt. Es wurde zweimal mit Ethylacetat extrahiert, die vereinigten organischen Phasen über einen wasserabweisenden Filter filtriert und eingeengt. Der Rückstand wurde säulenchromatographisch an Kieselgel (Hexan/Ethylacetat) gereinigt. Es wurden 260 mg der Titelverbindung erhalten.
UPLC-MS (Methode A2): Rₜ = 1.24 min
MS (ESIpos): m/z = 435(M+H)⁺
¹H NMR (400 MHz, DMSO-d6): 6 [ppm] = 3.49 - 3.64 (m, 1 H), 3.95 (s, 3 H), 4.49 (t, 2 H), 4.68 (dd, 2 H), 4.81 (d, 2 H), 8.20 (dd, 1 H), 8.35 - 8.41 (m, 1 H), 8.43 - 8.49 (m, 2 H), 8.55 - 8.58 (m, 1 H), 9.06 (s, 1 H), 12.53 (s, 1 H).

### Intermediat 4-2

### Methyl-2-(2-methoxyethyl)-5-({[6-(trifluormethyl)pyridin-2-yl]carbonyl}amino)-2H-indazol-6-carboxylat

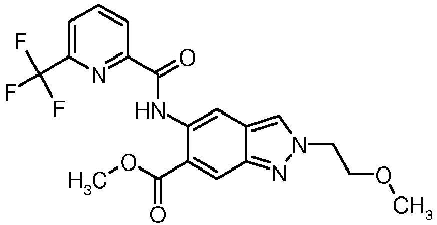

1.00 g (2.75 mmol) Methyl-5-({[6-(trifluormethyl)pyridin-2-yl]carbonyl}amino)-1H-indazol-6-carboxylat (Intermediat 3-1) wurden in 5 ml DMF gelöst und unter Rühren mit 387 µl (4.12 mmol) 2-Bromethyl-methylether, 1.14 g (8.23 mmol) Kaliumcarbonat und 228 mg (1.37 mmol) Kaliumiodid versetzt. Die Reaktionsmischung wurde 24 h bei 25°C gerührt, mit Wasser verdünnt und zweimal mit Ethylacetat extrahiert. Die vereinigten organischen Phasen wurden über einen wasserabweisenden Filter filtriert und eingeengt. Der Rückstand wurde säulenchromatographisch an Kieselgel (Hexan/Ethylacetat) gereinigt. Es wurden 12 mg der Titelverbindung erhalten.
UPLC-MS (Methode A1): Rₜ = 1.24 min
MS (ESIpos): m/z = 423 (M+H)⁺
¹H NMR (400 MHz, DMSO-d6): 6 [ppm] = 3.24 (s, 3 H), 3.86 (t, 2 H), 3.96 (s, 3 H), 4.65 (t, 2 H), 8.21 (dd, 1 H), 8.35 - 8.42 (m, 1 H), 8.43 - 8.51 (m, 2 H), 8.52 (d, 1 H), 9.06 (s, 1 H), 12.53 (s, 1 H).

### Intermediat 4-3

### Methyl-2-(3-methoxypropyl)-5-({[6-(trifluormethyl)pyridin-2-yl]carbonyl}amino)-2H-indazol-6-carboxylat

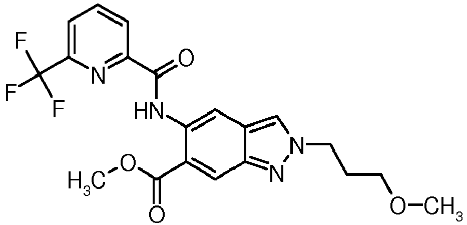

1.00 g (2.75 mmol) Methyl-5-({[6-(trifluormethyl)pyridin-2-yl]carbonyl}amino)-1H-indazol-6-carboxylat (Intermediat 3-1) wurden in 5 ml DMF gelöst und unter Rühren mit 460 µl (4.12 mmol) 1-Brom-3-methoxypropan, 1.14 g (8.23 mmol) Kaliumcarbonat und 228 mg (1.37 mmol) Kaliumiodid versetzt. Die Reaktionsmischung wurde 72 h bei 25°C gerührt, mit Wasser verdünnt und zweimal mit Ethylacetat extrahiert. Die vereinigten organischen Phasen wurden über einen wasserabweisenden Filter filtriert und eingeengt. Der Rückstand wurde säulenchromatographisch an Kieselgel (Hexan/Ethylacetat) gereinigt. Es wurden 28 mg der Titelverbindung erhalten.
UPLC-MS (Methode A1): Rₜ = 1.29 min
MS (ESIpos): m/z = 437 (M+H)⁺
¹H NMR (400 MHz, DMSO-d6): 6 [ppm] = 2.17 (quin, 2 H), 3.24 (s, 3 H), 3.33 - 3.36 (m, 2 H), 3.96 (s, 3 H), 4.53 (t, 2 H), 8.21 (dd, 1 H), 8.35 - 8.42 (m, 1 H), 8.45 - 8.49 (m, 2 H), 8.54 (d, 1 H), 9.06 (s, 1 H), 12.54 (s, 1 H).

### Intermediat 4-4

### Methyl-2-(3-hydroxy-3-methylbutyl)-5-({[6-(trifluormethyl)pyridin-2-yl]carbonyl}amino)-2H-indazol-6-carboxylat

### Herstellungsmethode 1

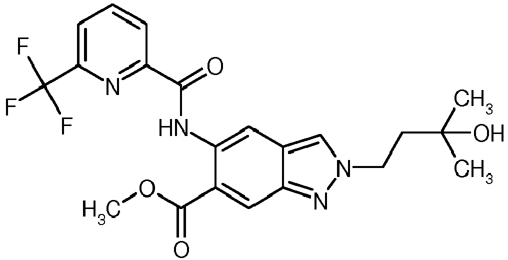

930 mg (2.55 mmol) Methyl-5-({[6-(trifluormethyl)pyridin-2-yl]carbonyl}amino)-1H-indazol-6-carboxylat (Intermediat 3-1), 1.06 g Kaliumcarbonat und 212 mg Kaliumiodid wurden in 9 ml DMF vorgelegt und die Mischung wurde 15 min gerührt. Danach addierte man 0.62 ml 4-Brom-2-methylbutan-2-ol und rührte bei 60°C über Nacht. Man versetzte mit Wasser, extrahierte zweimal mit Ethylacetat und wusch dreimal mit gesättigter Natriumchloridlösung, filtrierte und engte ein. Nach säulenchromatographischer Reinigung an Kieselgel (Hexan/Ethylacetat) wurden 424 mg der Titelverbindung erhalten. UPLC-MS (Methode A2): Rₜ = 1.21 min (UV Detector: TIC), gefundene Masse 450.00.
¹H-NMR (400MHz, DMSO-d₆): 6 [ppm]= 1.16 (s, 6 H) 2.02 - 2.11 (m, 2 H) 3.96 (s, 3 H) 4.51-4.60 (m, 3 H) 8.20 (dd, J=7.83, 1.01 Hz, 1 H) 8.39 (s, 1 H) 8.45 (s, 2 H) 8.55 (d, J=0.76 Hz, 1 H) 9.05 (s, 1 H) 12.52 (s, 1 H)

### Herstellungsmethode 2

1.95 g (7.03 mmol) Methyl-5-amino-2-(3-hydroxy-3-methylbutyl)-2H-indazol-6-carboxylat (Intermediat 7-1) wurden in 30 ml THF vorgelegt. Man addierte 1.45 g (7.73 mmol) 6-(Trifluormethyl)pyridin-2-carbonsäure, 2.71 g (8.44 mmol) O-(Benzotriazol-1-yl)-N,N,N',N'-tetramethyluroniumtetrafluoroborat und 1.47 ml (8.44 mmol) N-Ethyl-N-isopropylpropan-2-amin und rührte 20.5 h bei 25°C. Man versetzte mit Wasser, extrahierte dreimal mit Ethylacetat, wusch mit Kochsalzlösung, filtrierte über einen wasserabweisenden Filter und engte ein. Der Rückstand würde säulenchromatographisch an Kieselgel (Hexan/Ethylacetat-Gradient) getrennt. Es wurden 2.79 g der Titelverbindung erhalten.
UPLC-MS (Methode A1): Rt = 1.23 min (UV Detektor: TIC), gefundene Masse 450.00

### Intermediat 4-5

### Methyl-2-(2-{[tert-butyl(dimethyl)silyl]oxy}ethyl)-5-({[6-(trifluormethyl)pyridin-2-yl]carbonyl}amino)-2H-indazol-6-carboxylat

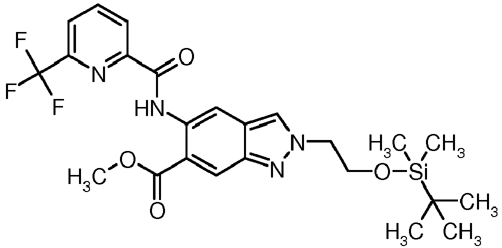

1.00 g (2.66 mmol, 97%) Methyl-5-({[6-(trifluormethyl)pyridin-2-yl]carbonyl}amino)-1H-indazol-6-carboxylat (Intermediat 3-1) wurden in 50 ml DMF vorgelegt, unter Rühren wurde mit 1.10 g (7.99 mmol) Kaliumcarbonat und 221 mg (1.33 mmol) Kaliumiodid versetzt und 30 min bei 25°C gerührt. Anschließend wurden 857 µl (3.99 mmol) (2-Bromethoxy)(tert-butyl)dimethylsilan zugegeben und 24 h bei 25°C gerührt. Die Reaktionsmischung wurde mit Wasser verdünnt und mit Ethylacetat extrahiert. Die vereinigten, organischen Phasen wurden über einen wasserabweisenden Filter filtriert und eingeengt. Der Rückstand wurde säulenchromatographisch an Kieselgel (Hexan/Ethylacetat) gereinigt. Es wurden 400 mg der Titelverbindung erhalten.
UPLC-MS (Methode A1): Rₜ = 1.58 min
MS (ESIpos): m/z = 523(M+H)⁺
¹H NMR (300 MHz, DMSO-d6): 6 [ppm] = -0.18 - -0.13 (m, 6 H), 0.74 (s, 9 H), 3.96 (s, 3 H), 4.08 (t, 2 H), 4.57 (t, 2 H), 8.15 - 8.25 (m, 1 H), 8.32 - 8.43 (m, 1 H), 8.43 - 8.52 (m, 3 H), 9.07 (s, 1 H), 12.53 (s, 1 H).

### Intermediat 4-6

### Methyl-2-(3-{[tert-butyl(dimethyl)silyl]oxy}propyl)-5-({[6-(trifluormethyl)pyridin-2-yl]carbonyl}amino)-2H-indazol-6-carboxylat

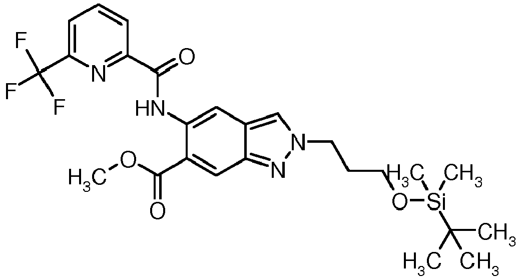

Analog zu Intermediat 4-5 wurden 1.00 g (2.75 mmol) Methyl-5-({[6-(trifluormethyl)pyridin-2-yl]carbonyl}amino)-1H-indazol-6-carboxylat (Intermediat 7-1) in 10 ml DMF gelöst, unter Rühren mit 1.14 g (8.24 mmol) Kaliumcarbonat und 228 mg (1.37 mmol) Kaliumiodid versetzt und 30 min bei 25°C gerührt. Anschließend wurden 1.04 g (4.12 mmol) (3-Brompropoxy)(tert-butyl)dimethylsilan zugegeben und 24 h bei 25°C gerührt. Die Reaktionsmischung wurde filtriert und der Filterkuchen mit Ethylacetat gewaschen. Es wurde zwischen Wasser und Ethylacetat verteilt und zweimal mit Ethylacetat extrahiert. Die vereinigten, organischen Phasen wurden über einen wasserabweisenden Filter filtriert und eingeengt. Nach Reinigung des Rückstandes durch präparative HPLC wurden 428 mg der Titelverbindung erhalten.
UPLC-MS (Methode A1): Rₜ = 1.63 min
MS (ESIpos): m/z = 537(M+H)⁺
¹H NMR (400 MHz, DMSO-d6): 6 [ppm] = -0.02 - 0.06 (m, 6 H), 0.87 (s, 9 H), 2.14 (quin, 2 H), 3.62 (t, 2 H), 3.96 (s, 3 H), 4.54 (t, 2 H), 8.20 (d, 1 H), 8.35 - 8.42 (m, 1 H), 8.43 - 8.48 (m, 2 H), 8.49 - 8.53 (m, 1 H), 9.06 (s, 1 H).

### Intermediat 4-7

### Methyl-5-({[6-(2-hydroxypropan-2-yl)pyridin-2-yl]carbonyl}amino)-2-(4,4,4-trifluorbutyl)-2H-indazol-6-carboxylat

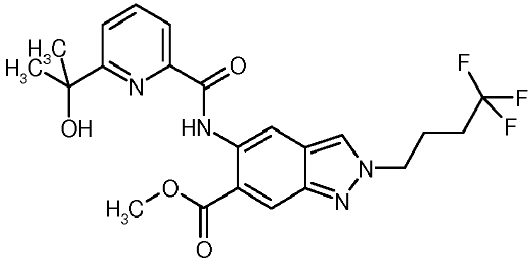

300 mg Methyl-5-({[6-(2-hydroxypropan-2-yl)pyridin-2-yl]carbonyl}amino)-1H-indazol-6-carboxylat (Intermediat 3-3) wurden in 4.5 ml DMF vorgelegt. Man addierte 287 mg 1,1,1-Trifluor-4-iodbutan und 333 mg Kaliumcarbonat und rührte bei 100°C 23 h. Man addierte Wasser und extrahierte dreimal mit Ethylacetat. Man engte ein und reinigte durch präparative HPLC. Man erhielt 72 mg der Titelverbindung.
UPLC-MS (Methode A1): Rt = 1.26 min (UV Detektor: TIC), gefundene Masse 464.17.

### Intermediat 4-8

### Methyl-5-{[(5-fluor-6-methylpyridin-2-yl)carbonyl]amino}-2-(3-hydroxy-3-methylbutyl)-2H-indazol-6-carboxylat

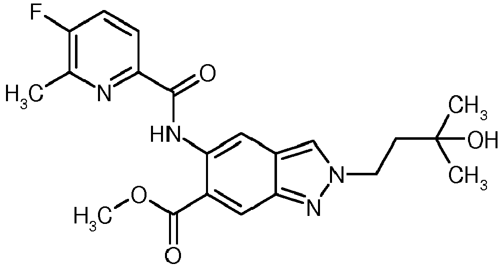

195 mg Methyl-5-amino-2-(3-hydroxy-3-methylbutyl)-2H-indazol-6-carboxylat (Intermediat 7-1) wurden mit 78 mg 5-Fluor-6-methylpyridin-2-carbonsäure analog zu Intermediat 4-4 (Herstellungsmethode 2) in 19.5 h umgesetzt. Es wurden 228 mg eines Rohproduktes nach analoger wässriger Aufarbeitung erhalten.
UPLC-MS (Methode A1): Rₜ = 1.20 min (UV Detektor: TIC), gefundene Masse 414.00.

### Intermediat 4-9

### Methyl-2-(3-hydroxy-3-methylbutyl)-5-{[(6-methylpyridin-2-yl)carbonyl]amino}-2H-indazol-6-carboxylat

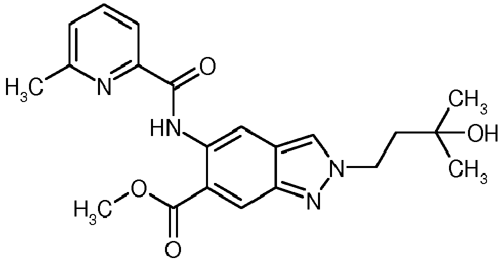

195 mg Methyl-5-amino-2-(3-hydroxy-3-methylbutyl)-2H-indazol-6-carboxylat (Intermediat 7-1) wurden mit 70 mg 6-Methylpyridin-2-carbonsäure analog zu Herstellung von Intermediat 4-4 (Herstellungsmethode 2) in 19.5 h umgesetzt. Es wurden 278 mg der Titelverbindung als Rohprodukt nach analoger wässriger Aufarbeitung erhalten.
UPLC-MS (Methode A1): Rt = 1.14 min (UV Detektor: TIC), gefundene Masse 396.00.

### Intermediat 4-10

### Methyl-2-[3-(2,2,2-trifluorethoxy)propyl]-5-({[6-(trifluormethyl)pyridin-2-yl]carbonyl}amino)-2H-indazol-6-carboxylat

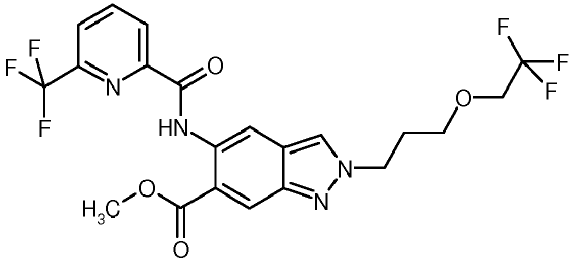

Eine Mischung aus 250 mg Methyl-5-({[6-(trifluormethyl)pyridin-2-yl]carbonyl}amino)-1H-indazol-6-carboxylat (Intermediat 7-1), 193 mg 3-Brompropyl-2,2,2-trifluorethylether, 242 mg Kaliumcarbonat und 145 mg Kaliumiodid in 3 ml DMF 20 h bei 100°C gerührt. Man versetzte mit Wasser, extrahierte mit Ethylacetat, wusch mit Kochsalzlösung und engte ein. Nach Reinigung durch präparative HPLC erhielt man 52 mg der Titelverbindung.
UPLC-MS (Methode A1): Rₜ = 1.39 min (UV Detektor: TIC), gefundene Masse 504.12.

### Intermediat 5-1

### N-[6-(2-Hydroxypropan-2-yl)-1H-indazol-5-yl]-6-(trifluormethyl)pyridin-2-carboxamid

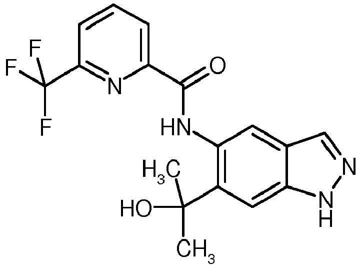

Zu einer im Eiswasser-Kältebad abgekühlten Lösung aus 1.50 g (4.12 mmol) Methyl-5-({[6-(trifluormethyl)pyridin-2-yl]carbonyl}amino)-1H-indazol-6-carboxylat (Intermediat 3-1) in 20 ml THF wurden vorsichtig 6.9 ml (5 Äquivalente) einer 3M MethylmagnesiumbromidLösung in Diethylether gegeben. Man rührte 1 h mit Eiswasser-Kältebadkühlung und 19.5 h bei Raumtemperatur. Man gab nochmals 2 Äquivalente der MethylmagnesiumbromidLösung zu und ließ weitere 24 h bei Raumtemperatur rühren. Man versetzte mit gesättigter wässriger Ammoniumchloridlösung, rührte die Mischung um und extrahierte dreimal mit Ethylacetat. Die vereinigten organischen Phasen wurden mit Kochsalzlösung gewaschen, über einen wasserabweisenden Filter filtriert und eingeengt. Der Rückstand wurde säulenchromatographisch an Kieselgel (Hexan/Ethylacetat) gereinigt. Es wurden 763 mg der Titelverbindung erhalten.
¹H-NMR (400MHz, DMSO-d₆): 6 [ppm]= 1.63 (s, 6H), 5.99 (s, 1H), 7.49 (s, 1H), 8.06 (s, 1H), 8.14 - 8.19 (m, 1H), 8.37 (t, 1H), 8.46 (d, 1H), 8.78 (s, 1H), 12.32 (s, 1H), 12.97 (s, 1H).

### Intermediat 5-2

### 6-(Difluormethyl)-N-[6-(2-hydroxypropan-2-yl)-1H-indazol-5-yl]pyridin-2-carboxamid

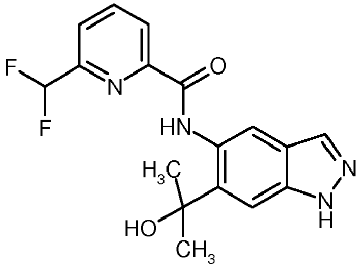

Analog zur Herstellung von Intermediat 5-1 wurden 2.40 g (6.93 mmol) Methyl-5-({[6-(difluormethyl)pyridin-2-yl]carbonyl}amino)-1H-indazol-6-carboxylat (Intermediat 3-2) in 10 ml THF mit drei Portionen 3M Methylmagnesiumbromidlösung in Diethylether (6.9 ml, dann 45 min Rühren bei Raumtemperatur; 11.6 ml, dann 2 h Rühren bei Raumtemperatur; 6.9 ml, dann 2 h Rühren bei Raumtemperatur) umgesetzt. Nach Aufarbeitung wie bei Intermediat 5-1 wurden 2.39 g eines Rohproduktes erhalten, welches ohne weitere Reinigung weiter eingesetzt wurde.

### Intermediat 6-1

### Methyl-2-(3-hydroxy-3-methylbutyl)-5-nitro-2H-indazol-6-carboxylat

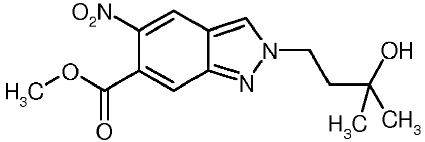

5.00 g (22.6 mmol) Methyl-5-nitro-1H-indazol-6-carboxylat (Intermediat 1-1) wurden in 40 ml DMF vorgelegt. Man addierte 5.65 g (33.9 mmol) 4-Brom-2-methylbutan-2-ol, 9.37 g (67.8 mmol) Kaliumcarbonat und 5.63 g (33.9 mmol) Kaliumiodid und die Mischung wurde 20 h bei 100°C gerührt. Man versetzte mit Wasser, extrahierte dreimal mit Ethylacetat, wusch mit Kochsalzlösung, filtrierte über einen wasserabweisenden Filter und engte ein. Der Rückstand wurde säulchenchromatographisch an Kieselgel (Hexan/Ethylacetat) gereinigt. Der erhaltene Feststoff wurde aus Diethylether ausgerührt, abgesaugt, mit Diethylether nachgewaschen und getrocknet. Es wurden 2.49 g der Titelverbindung erhalten.
UPLC-MS (Methode A1): Rₜ = 0.93 min (UV Detektor: TIC), gefundene Masse 307.00.
¹H-NMR (400MHz, DMSO-d₆): 6 [ppm]= 1.15 (s, 6H), 2.02 - 2.11 (m, 2H), 3.84 (s, 3H), 4.54 (s, 1H), 4.58 - 4.65 (m, 2H), 8.05 (s, 1H), 8.69 (s, 1H), 8.86 (s, 1H).

### Intermediat 7-1

### Methyl-5-amino-2-(3-hydroxy-3-methylbutyl)-2H-indazol-6-carboxylat

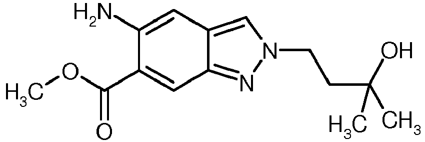

Man addierte 4.53 g Eisen und 217 mg Ammoniumchlorid zu 2.49 g (8.10 mmol) Methyl-2-(3-hydroxy-3-methylbutyl)-5-nitro-2H-indazol-6-carboxylat (Intermediat 6-1) in 30 ml Ethanol und 10 ml Wasser und rührte die Mischung 21.5 h bei 90°C. Man filtrierte über Celite, wusch dreimal mit Ethanol nach, engte das Filtrat ein und versetzte den Rückstand mit Wasser. Man extrahierte dreimal mit Ethylacetat (zur Verbesserung der Phasentrennung wurde mit Kochsalzlösung versetzt). Die vereinigten organischen Phasen wurden mit Kochsalzlösung gewaschen, über einen wasserabweisenden Filter filtriert und eingeengt. Es wurden 1.95 g (85% der Theorie) der Titelverbindung erhalten.
UPLC-MS (Methode A1): Rt = 0.67 min (UV Detektor: TIC), gefundene Masse 277.00.
¹H-NMR (400MHz, DMSO-d₆): 6 [ppm]= 1.14 (s, 6H), 1.96 - 2.08 (m, 2H), 3.85 (s, 3H), 4.39 -4.51 (m, 3H), 5.81 (s, 2H), 6.80 (s, 1H), 8.05 (s, 1H), 8.18 (s, 1H).

### Ausführungsbeispiele

### Beispiel 1

### N-[6-(2-Hydroxypropan-2-yl)-2-(2-methoxyethyl)-2H-indazol-5-yl]-6-(trifluormethyl)pyridin-2-carboxamid

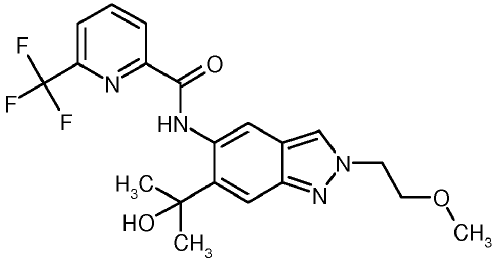

75 mg (0.18 mmol) Methyl-2-(2-methoxyethyl)-5-({[6-(trifluormethyl)pyridin-2-yl]carbonyl}amino)-2H-indazol-6-carboxylat (Intermediat 4-2) wurden in 500 µl THF gelöst und mit 887 µl (0.89 mmol) einer 1 M Methylmagnesiumbromidlösung in THF versetzt. Die Reaktionsmischung wurde 60 min bei 25°C gerührt. Anschließend wurde vorsichtig 1 ml einer gesättigten wässrigen Ammoniumchloridlösung hinzugegeben und filtriert. Die wässrige Phase wurde zweimal mit Ethylacetat extrahiert, die organischen Phasen vereint, über einen wasserabweisenden Filter filtriert und eingeengt. Der Rückstand wurde in 3 ml DMSO gelöst und durch präparative HPLC gereinigt. Die produkthaltigen Fraktionen wurden gefriergetrocknet. Es wurden 20 mg der Titelverbindung erhalten.
UPLC-MS (Methode A1): Rₜ = 1.08 min
MS (ESIpos): m/z = 423 (M+H)⁺
¹H NMR (300 MHz, DMSO-d6): 6 [ppm] = 1.62 (s, 6 H), 3.22 (s, 3 H), 3.82 (t, J=5.2 Hz, 2 H), 4.55 (t, J=5.2 Hz, 2 H), 5.96 (s, 1 H), 7.57 (s, 1 H), 8.16 (d, J=7.2 Hz, 1 H), 8.29 - 8.42 (m, 2 H), 8.42 - 8.50 (m, 1 H), 8.71 (s, 1 H), 12.36 (s, 1 H)

### Beispiel 2

### N-[6-(Hydroxymethyl)-2-(2-methoxyethyl)-2H-indazol-5-yl]-6-(trifluormethyl)pyridin-2-carboxamid

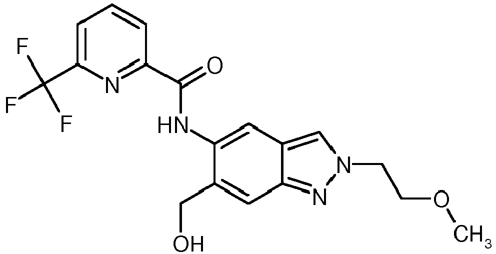

13 mg (0.36 mmol) Lithiumaluminiumhydrid wurden in 1 ml THF suspendiert und auf 0°C gekühlt. 75 mg (0.17 mmol) Methyl-2-(2-methoxyethyl)-5-({[6-(trifluormethyl)pyridin-2-yl]carbonyl}amino)-2H-indazol-6-carboxylat (Intermediat 4-2) gelöst in 500 µl THF wurden zugetropft und 60 min bei 25°C gerührt. Man verdünnte mit Wasser, extrahierte zweimal mit Ethylacetat und die vereinigten, organischen Phasen wurden Kochsalzlösung gewaschen, über einen wasserabweisenden Filter filtriert, eingeengt und im Vakuum getrocknet. Man erhielt 36 mg der Titelverbindung.
UPLC-MS (Methode A2): Rₜ = 0.97 min
MS (ESIpos): m/z = 409 (M+H)⁺
¹H NMR (400 MHz, DMSO-d6): 6 [ppm] = 1.62 (s, 6 H), 3.86 (q, 2 H), 4.43 (t, 2 H), 4.95 (t, 1 H), 5.94 (s, 1 H), 7.57 (s, 1 H), 8.16 (dd, 1 H), 8.30 (s, 1 H), 8.37 (t, 1 H), 8.45 (d, 1 H), 8.72 (s, 1 H), 12.36 (s, 1 H).

### Beispiel 3

### N-[6-(2-Hydroxypropan-2-yl)-2-(3-methoxypropyl)-2H-indazol-5-yl]-6-(trifluormethyl)pyridin-2-carboxamid

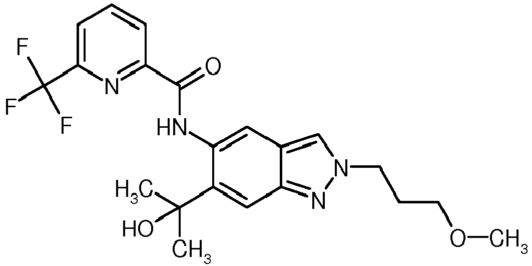

75 mg (0.17 mmol) Methyl-2-(3-methoxypropyl)-5-({[6-(trifluormethyl)pyridin-2-yl]carbonyl}amino)-2H-indazol-6-carboxylat (Intermediat 4-3) wurden in 500 µl THF gelöst und mit 859 µl (0.86 mmol) einer 1 M Methylmagnesiumbromidlösung in THF versetzt. Die Reaktionsmischung wurde 60 min bei 25°C gerührt. Anschließend wurde vorsichtig 1 ml einer gesättigten Ammoniumchloridlösung hinzugegeben und filtriert. Die wässrige Phase wurde zweimal mit Ethylacetat extrahiert, die organischen Phasen vereint, über einen wasserabweisenden Filter filtriert und eingeengt. Der Rückstand wurde in 3 ml DMSO gelöst und durch präparative HPLC gereinigt. Die produkthaltigen Fraktionen wurden gefriergetrocknet. Es wurden 25 mg der Titelverbindung erhalten.
UPLC-MS (Methode A1): Rₜ = 1.13 min
MS (ESIpos): m/z = 437 (M+H)⁺
¹H NMR (400 MHz, DMSO-d6): 6 [ppm] = 1.62 (s, 6 H), 2.14 (quin, 2 H), 3.23 (s, 3 H), 3.26-3.32 (m, 2 H), 4.44 (t, 2 H), 5.95 (s, 1 H), 7.58 (s, 1 H), 8.16 (d, 1 H), 8.31 - 8.40 (m, 2 H), 8.43 - 8.48 (m, 1 H), 8.72 (s, 1 H), 12.36 (s, 1 H).

### Beispiel 4

### N-[6-(Hydroxymethyl)-2-(3-methoxypropyl)-2H-indazol-5-yl]-6-(trifluormethyl)pyridin-2-carboxamid

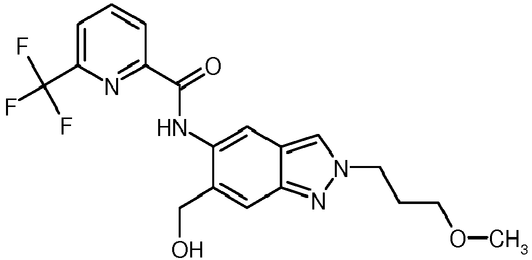

13 mg Lithiumaluminiumhydrid wurden in THF suspendiert und die Mischung auf 0°C abgekühlt. Man tropfte 75 mg (0.17 mmol) Methyl-2-(3-methoxypropyl)-5-({[6-(trifluormethyl)pyridin-2-yl]carbonyl}amino)-2H-indazol-6-carboxylat (Intermediat 4-3) in THF zu und ließ innerhalb von 30 min auf Raumtemperatur kommen. Man verdünnte mit Wasser, filtrierte, wusch den Rückstand mit Ethylacetat und extrahierte das Filtrat mit Ethylacetat. Die vereinigten Ethylacetatphasen wurden mit Kochsalzlösung gewaschen, über einen wasserabweisenden Filter filtriert und eingeengt. Man reinigte den Rückstand durch präparative HPLC.
¹H NMR (300 MHz, DMSO-*d*₆): 6 [ppm] = 2.14 (quin, 2 H), 3.23 (s, 3 H), 3.29 (t, 2 H), 4.45 (t, *J*=7.0 Hz, 2 H), 4.68 (d, 2 H), 5.77 (t, 1 H), 7.58 (s, 1 H), 8.18 (d, 1 H), 8.32 - 8.48 (m, 3 H), 8.51 (s, 1 H), 11.21 (s, 1 H).

### Beispiel 5

### N-[2-(2-Hydroxyethyl)-6-(2-hydroxypropan-2-yl)-2H-indazol-5-yl]-6-(trifluormethyl)pyridin-2-carboxamid

### Stufe A:

### Herstellung von N-[2-(2-{[tert-Butyl(dimethyl)silyl]oxy}ethyl)-6-(2-hydroxypropan-2-yl)-2H-indazol-5-yl]-6-(trifluormethyl)pyridin-2-carboxamid

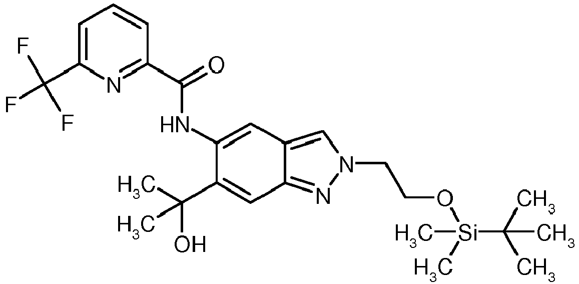

100 mg (0.19 mmol) Methyl-2-(2-{[tert-butyl(dimethyl)silyl]oxy}ethyl)-5-({[6-(trifluormethyl)pyridin-2-yl]carbonyl}amino)-2H-indazol-6-carboxylat (Intermediat 4-5) wurden in 1 ml THF gelöst und mit 669 µl (0.67 mmol) einer 1 M Methylmagnesiumbromidlösung in THF versetzt. Die Reaktionsmischung wurde 60 min bei 25°C gerührt. Es wurden nochmals 287 µl (0.29 mmol) einer 1 M Methylmagnesiumbromidlösung in THF hinzugegeben und 3 h bei 25°C gerührt.

Anschließend wurden vorsichtig 20 ml einer gesättigten Ammoniumchloridlösung hinzugegeben und filtriert. Die wässrige Phase wurde zweimal mit Ethylacetat extrahiert, die organischen Phasen vereint, über Magnesiumsulfat getrocknet, filtriert, eingeengt und im Vakuum getrocknet. Man erhielt 50 mg N-[2-(2-{[tert-Butyl(dimethyl)silyl]oxy}ethyl)-6-(2-hydroxypropan-2-yl)-2H-indazol-5-yl]-6-(trifluormethyl)pyridin-2-carboxamid.
UPLC-MS (Methode A2): Rₜ = 1.51 min
MS (ESIpos): m/z = 523(M+H)⁺
¹H NMR (300 MHz, DMSO-d6): 6 [ppm] = -0.17 - -0.09 (m, 6 H), 0.78 (s, 9 H), 1.62 (s, 6 H), 4.04 (t, 2 H), 4.47 (t, 2 H), 5.98 (s, 1 H), 7.57 (s, 1 H), 8.16 (d, 1 H), 8.29 (s, 1 H), 8.37 (t, 1 H), 8.45 (d, 1 H), 8.73 (s, 1 H), 12.38 (s, 1 H).

### Stufe B:

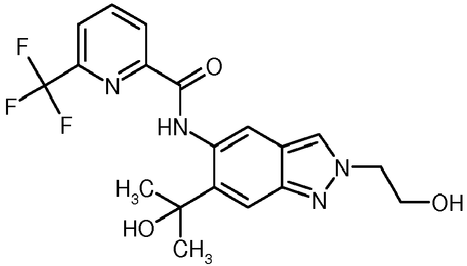

50 mg (96 µmol) N-[2-(2-{[tert-Butyl(dimethyl)silyl]oxy}ethyl)-6-(hydroxymethyl)-2H-indazol-5-yl]-6-(trifluormethyl)pyridin-2-carboxamid wurden in 1.0 ml THF gelöst und mit 144 µL (0.14 mmol) einer 1 M Lösung Tetrabutylammoniumfluorid in THF versetzt. Die Reaktionsmischung wurde 1 h bei Raumtemperatur gerührt. Man verdünnte mit Wasser, extrahierte zweimal mit Ethylacetat und die vereinigten organischen Phasen wurden mit gesättigter Natriumchloridlösung gewaschen, über einen wasserabweisenden Filter filtriert und eingeengt. Man erhielt 36 mg N-[2-(2-Hydroxyethyl)-6-(2-hydroxypropan-2-yl)-2H-indazol-5-yl]-6-(trifluormethyl)pyridin-2-carboxamid (Beispiel 5).
¹H-NMR (400MHz, DMSO-d₆): d [ppm]= 1.62 (s, 6H), 3.86 (q, 2H), 4.43 (t, 2H), 4.95 (t, 1H), 5.94 (s, 1H), 7.57 (s, 1H), 8.16 (dd, 1H), 8.30 (s, 1H), 8.37 (t, 1H), 8.45 (d, 1H), 8.72 (s, 1H), 12.36 (s, 1H).
UPLC-MS (Methode A2): Rₜ = 0.97 min (UV Detektor: TIC), gefundene Masse 408.00.

### Beispiel 6

### N-[6-(2-Hydroxypropan-2-yl)-2-(3-hydroxypropyl)-2H-indazol-5-yl]-6-(trifluormethyl)pyridin-2-carboxamid

### Stufe A:

### Herstellung von N-[2-(3-{[tert-Butyl(dimethyl)silyl]oxy}propyl)-6-(2-hydroxypropan-2-yl)-2H-indazol-5-yl]-6-(trifluormethyl)pyridin-2-carboxamid

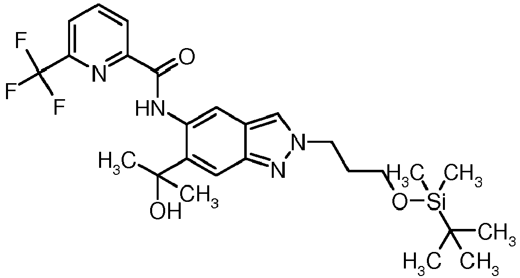

50 mg (0.09 mmol) Methyl-2-(3-{[tert-butyl(dimethyl)silyl]oxy}propyl)-5-({[6-(trifluormethyl)pyridin-2-yl]carbonyl}amino)-2H-indazol-6-carboxylat (Intermediat 4-6) wurden in 500 µl THF gelöst und mit 326 µl (0.33 mmol) einer 1 M Methylmagnesiumbromidlösung in THF versetzt. Die Reaktionsmischung wurde 60 min bei 25°C gerührt. Anschließend wurden vorsichtig 20 ml einer gesättigten Ammoniumchloridlösung hinzugegeben und zweimal mit Ethylacetat extrahiert. Die vereinigten organischen Phasen wurden über einen wasserabweisenden Filter filtriert eingeengt und im Vakuum getrocknet. Der Rückstand wurde durch präparative HPLC gereinigt. Es wurden 40 mg N-[2-(3-{[tert-Butyl(dimethyl)silyl]oxy}propyl)-6-(2-hydroxypropan-2-yl)-2H-indazol-5-yl]-6-(trifluormethyl)pyridin-2-carboxamid erhalten.
UPLC-MS (Methode A1): Rₜ = 1.58 min
MS (ESIpos): m/z = 537(M+H)⁺
¹H NMR (300 MHz, DMSO-d6): 6 [ppm] = 0.02 - 0.05 (m, 6 H), 0.84 - 0.91 (m, 9 H), 1.62 (s, 6 H), 2.02 - 2.18 (m, 2 H), 3.55 - 3.62 (m, 2 H), 4.45 (t, 2 H), 5.96 (s, 1 H), 7.57 (s, 1 H), 8.16 (d, 1 H), 8.31 (s, 1 H), 8.33 - 8.42 (m, 1 H), 8.45 (d, 1 H), 8.72 (s, 1 H), 12.37 (s, 1 H).

### Stufe B:

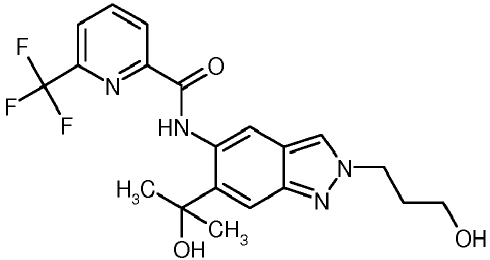

37 mg (0.07 mmol) N-[2-(3-{[tert-Butyl(dimethyl)silyl]oxy}propyl)-6-(2-hydroxypropan-2-yl)-2H-indazol-5-yl]-6-(trifluormethyl)pyridin-2-carboxamid wurden in 500 µl THF gelöst und mit 207 µL (0.21 mmol) einer 1 M Lösung Tetrabutylammoniumfluorid in THF versetzt. Die Reaktionsmischung wurde 2 h bei 25°C gerührt. Man verdünnte mit Wasser, extrahierte zweimal mit Ethylacetat und die vereinigten organischen Phasen wurden mit gesättigter Natriumchloridlösung gewaschen, filtriert und eingeengt. Nach Reinigung durch präparative HPLC wurden 10 mg N-[6-(2-Hydroxypropan-2-yl)-2-(3-hydroxypropyl)-2H-indazol-5-yl]-6-(trifluormethyl)pyridin-2-carboxamid (Beispiel 6) erhalten.
UPLC-MS (Methode A2): Rₜ = 1.00 min
MS (ESIpos): m/z = 423 (M+H)⁺
¹H NMR (400 MHz, DMSO-d6): 6 [ppm] = 1.62 (s, 6 H), 2.00 - 2.07 (m, 2 H), 3.07 - 3.22 (m, 1 H), 3.39 (t, 2 H), 4.45 (t, 2 H), 4.63 (br. s., 1 H), 5.94 (br. s., 1 H), 7.56 (s, 1 H), 8.14 (d, 1 H), 8.28 - 8.39 (m, 2 H), 8.41 - 8.47 (m, 1 H), 8.72 (s, 1 H), 12.31 (br. s., 1 H).

### Beispiel 7

### N-[2-(2-Hydroxyethyl)-6-(hydroxymethyl)-2H-indazol-5-yl]-6-(trifluormethyl)pyridin-2-carboxamid

### Stufe A:

### N-[2-(2-{[tert-Butyl(dimethyl)silyl]oxy}ethyl)-6-(hydroxymethyl)-2H-indazol-5-yl]-6-(trifluormethyl)pyridin-2-carboxamid

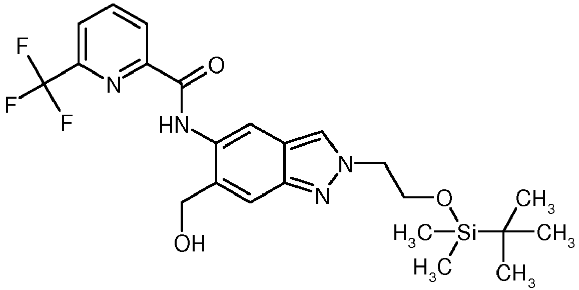

100 mg (0.19 mmol) Methyl-2-(2-{[tert-butyl(dimethyl)silyl]oxy}ethyl)-5-({[6-(trifluormethyl)pyridin-2-yl]carbonyl}amino)-2H-indazol-6-carboxylat (Intermediat 4-5) wurden in 1 ml THF gelöst und mit 191 µl (0.38 mmol) einer 2 M Lithiumborhydridlösung versetzt. Man ließ 24 h bei 25°C rühren. Es wurden 14 mg (0.38 mmol) Natriumborhydrid und 500 µl Methanol zugegeben und 4 h bei 25°C gerührt. Es wurden nochmals 14 mg (0.38 mmol) Natriumborhydrid zugegeben und 24 h bei 25°C gerührt. Die Reaktionsmischung wurde vorsichtig mit Wasser und eingeengt. Anschließend wurde zweimal mit Ethylacetat extrahiert und die vereinigten organischen Phasen mit gesättigter Natriumchloridlösung gewaschen, über einen wasserabweisenden Filter filtriert und eingeengt. Der Rückstand wurde in 2 ml DMSO aufgenommen und durch präparative HPLC gereinigt. Man erhielt 30 mg N-[2-(2-{[tert-Butyl(dimethyl)silyl]oxy}ethyl)-6-(hydroxymethyl)-2H-indazol-5-yl]-6-(trifluormethyl)pyridin-2-carboxamid.
UPLC-MS (Methode A2): Rₜ = 1.44 min
MS (ESIpos): m/z = 495(M+H)⁺
¹H NMR (300 MHz, DMSO-d6): 6 [ppm] = -0.16 - -0.12 (m, 6 H), 0.75 - 0.79 (m, 9 H), 4.05 (t, 2 H), 4.48 (t, 2 H), 4.69 (d, 2 H), 5.75 - 5.77 (m, 1 H), 7.57 (s, 1 H), 8.18 (dd, 1 H), 8.30-8.33 (m, 1 H), 8.38 (t, 1 H), 8.45 (d, 1 H), 8.51 (s, 1 H), 11.20 (s, 1 H).

### Stufe B:

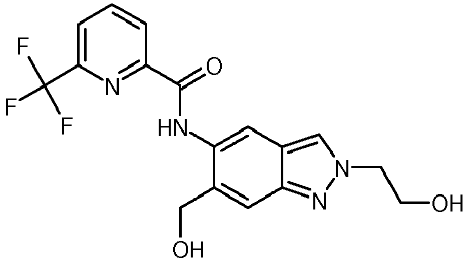

33 mg (0.07 mmol) N-[2-(2-{[tert-Butyl(dimethyl)silyl]oxy}ethyl)-6-(hydroxymethyl)-2H-indazol-5-yl]-6-(trifluormethyl)pyridin-2-carboxamid wurden in 1 ml THF gelöst und mit 100 µL (0.10 mmol) einer 1 M Lösung Tetrabutylammoniumfluorid in THF versetzt. Die Reaktionsmischung wurde 1 h bei 25°C gerührt. Man verdünnte mit Wasser, extrahierte zweimal mit Ethylacetat und die vereinigten organischen Phasen wurden mit gesättigter Natriumchloridlösung gewaschen, über einen wasserabweisenden Filter filtriert, eingeengt und im Vakuum getrocknet. Es wurden 25 mg N-[2-(2-Hydroxyethyl)-6-(hydroxymethyl)-2H-indazol-5-yl]-6-(trifluormethyl)pyridin-2-carboxamid (Beispiel 7) erhalten.
UPLC-MS (Methode A2): Rₜ = 0.87 min
MS (ESIpos): m/z = 381 (M+H)⁺
¹H NMR (300 MHz, DMSO-d6): 6 [ppm] = 3.87 (q, 2 H), 4.44 (t, 2 H), 4.69 (d, 2 H), 4.98 (t, 1 H), 5.70 - 5.81 (m, 1 H), 7.57 (s, 1 H), 8.11 - 8.23 (m, 1 H), 8.31 - 8.42 (m, 2 H), 8.43 - 8.49 (m, 1 H), 8.51 (s, 1 H), 11.20 (s, 1 H).

### Beispiel 8

### N-[6-(2-Hydroxypropan-2-yl)-2-(oxetan-3-ylmethyl)-2H-indazol-5-yl]-6-(trifluormethyl)pyridin-2-carboxamid

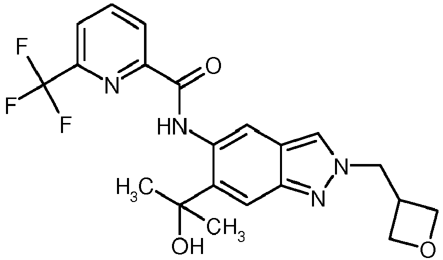

50 mg (0.12 mmol) Methyl-2-(oxetan-3-ylmethyl)-5-({[6-(trifluormethyl)pyridin-2-yl]carbonyl}amino)-2H-indazol-6-carboxylat (Intermediat 4-1) wurden in 500 µl THF gelöst und mit 576 µl (0.58 mmol) einer 1 M Methylmagnesiumbromidlösung in THF versetzt.. Die Reaktionsmischung wurde 60 min bei 25°C gerührt. Anschließend wurden vorsichtig 20 ml einer gesättigten wässrigen Ammoniumchloridlösung hinzugegeben und eingeengt. Die wässrige Phase wurde zweimal mit Ethylacetat extrahiert, die organischen Phasen vereint, über Magnesiumsulfat getrocknet, filtriert und eingeengt. Der Rückstand wurde in 2.0 ml DMSO gelöst und durch präparative HPLC gereinigt. Die produkthaltigen Fraktionen wurden gefriergetrocknet. Es wurden 30 mg der Titelverbindung erhalten.
UPLC-MS (Methode A2): Rₜ = 1.03 min
MS (ESIpos): m/z = 435 (M+H)⁺
¹H NMR (400 MHz, DMSO-d6): 6 [ppm] = 1.62 (s, 6 H), 3.45 - 3.61 (m, 1 H), 4.48 (t, 2 H), 4.66 (dd, 2 H), 4.72 (d, 2 H), 5.94 (s, 1 H), 7.57 (s, 1 H), 8.16 (d, 1 H), 8.33 - 8.42 (m, 2 H), 8.42 - 8.47 (m, 1 H), 8.72 (s, 1 H), 12.36 (s, 1 H).

### Beispiel 9

### N-[6-(Hydroxymethyl)-2-(oxetan-3-ylmethyl)-2H-indazol-5-yl]-6-(trifluormethyl)pyridin-2-carboxamid

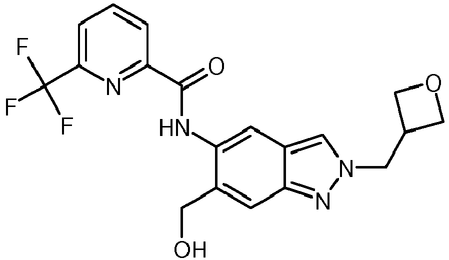

75 mg (0.17 mmol) Methyl-2-(oxetan-3-ylmethyl)-5-({[6-(trifluormethyl)pyridin-2-yl]carbonyl}amino)-2H-indazol-6-carboxylat (Intermediat 4-1) wurden in 1 ml einer Mischung aus THF/Methanol (1:1) gelöst und mit 8 mg (0.21 mmol) Natriumborhydrid versetzt. Man ließ 60 min bei 25°C rühren. Die Reaktionsmischung wurde eingeengt und der Rückstand mit Wasser versetzt. Die Suspension wurde 15 min kräftig gerührt, der Feststoff abgesaugt, zweimal mit Wasser und zweimal mit Diethylether gewaschen und im Vakuum getrocknet. Es wurden 48 mg der Titelverbindung erhalten.
UPLC-MS (Methode A2): Rₜ = 0.94 min
MS (ESIpos): m/z = 407 (M+H)⁺
¹H NMR (300 MHz, DMSO-d6): 6 [ppm] = 3.55 (s, 1 H), 4.48 (t, 2 H), 4.61 - 4.77 (m, 6 H), 7.57 (s, 1 H), 8.18 (dd, 1 H), 8.33 - 8.49 (m, 3 H), 8.51 (s, 1 H), 11.21 (s, 1 H).

### Beispiel 10

### N-{6-(2-Hydroxypropan-2-yl)-2-[3-(methylsulfonyl)propyl]-2H-indazol-5-yl}-6-(trifluormethyl)pyridin-2-carboxamid

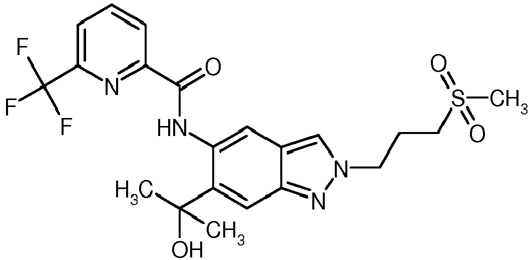

Eine Mischung aus 500 mg (1.32 mmol) N-[6-(2-Hydroxypropan-2-yl)-1H-indazol-5-yl]-6-(trifluormethyl)pyridin-2-carboxamid (Intermediat 5-1), 569 mg Kaliumcarbonat und 114 mg Kaliumiodid in 5.0 ml DMF wurde 15 min bei Raumtemperatur gerührt. Man addierte 414 mg 1-Brom-3-(methylsulfonyl)propan und rührte bei Raumtemperatur über Nacht. Man versetzte mit Wasser, extrahierte zweimal mit Ethylacetat, wusch mit Kochsalzlösung und engte ein. Der Rückstand wurde säulenchromatographisch gereinigt (Dichlormethan-Methanol-Gradient). Nach Ausrühren der Produktfraktion aus Diethylether wurden 59 mg der Titelverbindung erhalten.
UPLC-MS (Methode A2): Rₜ = 1.02 min
MS (ESIpos): m/z = 485 (M+H)+
¹H-NMR (300MHz, DMSO-d₆): 6 [ppm]= 1.63 (s, 6H), 2.26 - 2.42 (m, 2H), 2.99 (s, 3H), 3.06 - 3.16 (m, 2H), 4.55 (t, 2H), 5.96 (s, 1H), 7.60 (s, 1H), 8.16 (d, 1H), 8.33 - 8.48 (m, 3H), 8.73 (s, 1H), 12.37 (s, 1H).

### Beispiel 11

### N-[2-(3-Hydroxy-3-methylbutyl)-6-(2-hydroxypropan-2-yl)-2H-indazol-5-yl]-6-(trifluormethyl)pyridin-2-carboxamid

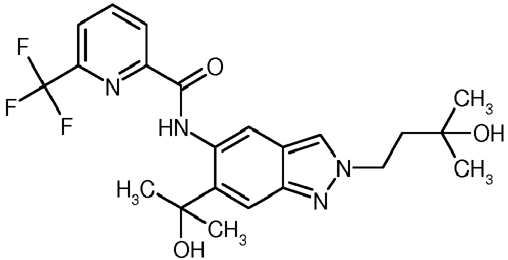

### Herstellungsmethode 1

705 mg (1.57 mmol) Methyl-2-(3-hydroxy-3-methylbutyl)-5-({[6-(trifluormethyl)pyridin-2-yl]carbonyl}amino)-2H-indazol-6-carboxylat (Intermediat 4-4) wurden in 10 ml THF vorgelegt und im Eiswasser-Kältebad gekühlt. Man addierte 2.6 ml (5.0 Äquivalente) 3M Methylmagnesiumbromidlösung (in Diethylether) und ließ 1 h unter Eisbadkühlung und 4.5 h bei Raumtemperatur rühren. Man addierte erneut 1 Äquivalent der Methylmagnesiumbromidlösung und ließ 20.5 h bei Raumtemperatur rühren. Erneut addierte man 1 Äquivalent der Methylmagnesiumbromidlösung und ließ 22 h bei Raumtemperatur rühren. Das Reaktionsgemisch wurde mit gesättigter wässriger Ammoniumchloridlösung versetzt, gerührt und dreimal mit Ethylacetat extrahiert. Die vereinigten organischen Phasen wurden mit Kochsalzlösung gewaschen, über einen wasserabweisenden Filter filtriert und eingeengt. Man erhielt 790 mg eines Rückstandes, der durch präparative HPLC gereinigt wurde. Man erhielt 234 mg der Titelverbindung und 164 mg einer Produktfraktion, die mit Diethylether ausgerührt wurde. Nach Absaugen und anschließender Trocknung wurden weitere 146 mg der Titelverbindung erhalten UPLC-MS (Methode A1): Rₜ = 1.10 min (UV Detektor: TIC), gefundene Masse 450.00. ¹H-NMR (400MHz, DMSO-d₆): 6 [ppm]= 1.14 (s, 6H), 1.61 (s, 6H), 1.99 - 2.08 (m, 2H), 4.42 - 4.55 (m, 3H), 5.93 (s, 1H), 7.56 (s, 1H), 8.15 (dd, 1H), 8.32 - 8.39 (m, 2H), 8.41 - 8.47 (m, 1H), 8.70 (s, 1H), 12.34 (s, 1H).

### Herstellungsmethode 2

Eine Mischung aus 500 mg (1.37 mmol) N-[6-(2-Hydroxypropan-2-yl)-1H-indazol-5-yl]-6-(trifluormethyl)pyridin-2-carboxamid (Intermediat 5-1), 569 mg Kaliumcarbonat und 114 mg Kaliumiodid in 5 ml DMF wurde 15 min bei Raumtemperatur gerührt. Man addierte 344 mg (1.5 Äquivalente) 4-Brom-2-methylbutan-2-ol und erwärmte 2 h auf 100°C. Man addierte erneut 0.5 Äquivalente 4-Brom-2-methylbutan-2-ol und rührte bei Raumtemperatur über Nacht. Die Mischung wurde mit Wasser versetzt, zweimal mit Ethylacetat extrahiert, die vereinigten organischen Phasen wurden mit gesättigter Kochsalzlösung gewaschen und über einen wasserabweisenden Filter filtriert und eingeengt. Der Rückstand wurde durch säulenchromatographische Reinigung an Kieselgel (Hexan/Ethylacetat) gereinigt. Man erhielt 100 mg einer Produktfraktion, die mit Diethylether ausgerührt wurde. Nach Trocknen wurden 60 mg der Titelverbindung erhalten.
¹H-NMR (400MHz, DMSO-d₆): 6 [ppm]= 1.14 (s, 6 H), 1.61 (s, 6H), 1.99 - 2.07 (m, 2 H), 4.43 - 4.52 (m, 3 H) 5.94 (s, 1 H) 7.57 (s, 1 H) 8.15 (dd, 1H) 8.33 - 8.40 (m, 2 H), 8.42 - 8.48 (m, 1 H), 8.71 (s, 1 H), 12.35 (s, 1 H)

### Beispiel 12

### N-{6-(2-Hydroxypropan-2-yl)-2-[2-(methylsulfonyl)ethyl]-2H-indazol-5-yl}-6-(trifluormethyl)pyridin-2-carboxamid

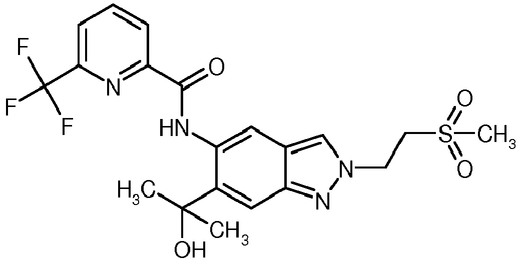

160 mg (0.44 mmol) N-[6-(2-Hydroxypropan-2-yl)-1H-indazol-5-yl]-6-(trifluormethyl)pyridin-2-carboxamid (Intermediat 5-1) wurden zusammen mit 182 mg Kaliumcarbonat und 36 mg Kaliumiodid in 1.0 ml DMF suspendiert und die Mischung wurde 15 min bei Raumtemperatur gerührt. Dann wurden 123 mg 2-Bromethyl-methylsulfon zugegeben und über Nacht bei Raumtemperatur gerührt. Man addierte Wasser, extrahierte zweimal mit Ethylacetat, wusch mit gesättigter wässriger Natriumchloridlösung, filtrierte über einen wasserabweisenden Filter und engte ein. Nach Reinigung des Rückstandes durch präparative HPLC wurden 20 mg der Titelverbindung erhalten.
UPLC (Methode A2): Rₜ = 1.01 min;
MS (ESIpos): m/z = 471 (M+H)+
¹H NMR (400 MHz, DMSO-*d*₆): δ [ppm]= 1.63 (s, 6 H), 2.90 (s, 3 H), 3.85 (t, 2 H), 4.86 (t, 2 H), 5.97 (s, 1 H), 7.59 (s, 1 H), 8.13 - 8.19 (m, 1 H), 8.37 (s, 1 H), 8.41 - 8.48 (m, 2 H), 8.74 (s, 1 H), 12.37 (s, 1 H).

### Beispiel 13

### 6-(Difluormethyl)-N-[2-(3-hydroxy-3-methylbutyl)-6-(2-hydroxypropan-2-yl)-2H-indazol-5-yl]pyridin-2-carboxamid

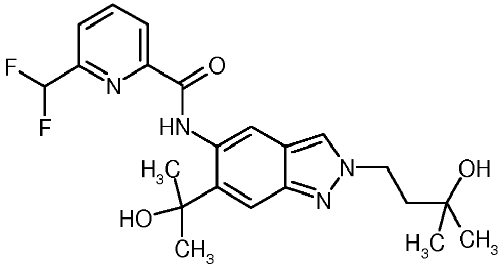

Ein Mischung aus 250 mg 6-(Difluormethyl)-N-[6-(2-hydroxypropan-2-yl)-1H-indazol-5-yl]pyridin-2-carboxamid (Rohprodukt Intermediat 5-2), 144 mg Kaliumiodid und 239 mg Kaliumcarbonat in 2.5 ml DMF wurde 15 min bei Raumtemperatur gerührt. Man addierte 145 mg (0.87 mmol) 4-Brom-2-methylbutan-2-ol, rührte bei 110°C 3 h, addierte erneut 96 mg 4-Brom-2-methylbutan-2-ol und rührte bei 110°C 4 h. Man versetzte mit Wasser, extrahierte zweimal mit Ethylacetat, wusch mit halbgesättigter wässriger Kochsalzlösung, filtrierte über einen wasserabweisenden Filter und engte ein. Man reinigte säulenchromatographisch an Kieselgel (Hexan/Ethylacetat). Es wurden 61 mg der Titelverbindung erhalten.

UPLC-MS (Methode A1): Rt = 1.00 min (UV Detektor: TIC), gefundene Masse 432.00. ¹H-NMR (300MHz, DMSO-d₆): 6 [ppm]= 1.14 (s, 6H), 1.63 (s, 6H), 1.97 - 2.08 (m, 2H), 4.41 - 4.55 (m, 3H), 5.99 (s, 1H), 7.03 (t, 1H), 7.56 (s, 1H), 7.94 - 8.00 (m, 1H), 8.24 - 8.38 (m, 3H), 8.71 (s, 1H), 12.49 (s, 1H).

### Beispiel 14

### 6-(Difluormethyl)-N-{6-(2-hydroxypropan-2-yl)-2-[2-(methylsulfonyl)ethyl]-2H-indazol-5-yl}pyridin-2-carboxamid

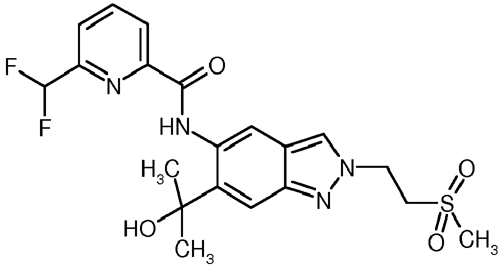

Ein Mischung aus 250 mg 6-(Difluormethyl)-N-[6-(2-hydroxypropan-2-yl)-1H-indazol-5-yl]pyridin-2-carboxamid (Rohprodukt Intermediat 5-2), 144 mg Kaliumiodid und 239 mg Kaliumcarbonat in 2.5 ml DMF wurde 15 min bei Raumtemperatur gerührt. Man addierte 162 mg 2-Bromethyl-methylsulfon und rührte bei 110°C 3 h. Man versetzte mit Wasser, extrahierte zweimal mit Ethylacetat, wusch mit halbgesättigter wässriger Kochsalzlösung, filtrierte über einen wasserabweisenden Filter und engte ein. Der Rückstand wurde durch präparative HPLC gereinigt und die Produktfraktionen zusätzlich noch durch säulenchromatographische Reinigung an Kieselgel (Hexan-Ethylacetat) gereinigt. Es wurden 40 mg der Titelverbindung erhalten.
¹H-NMR (400MHz, DMSO-d₆): 6 [ppm]= 1.65 (s, 6H), 2.90 (s, 3H), 3.85 (t, 2H), 4.85 (t, 2H), 6.03 (s, 1H), 7.04 (t, 1H), 7.59 (s, 1H), 7.98 (d, 1H), 8.25 - 8.36 (m, 2H), 8.43 (s, 1H), 8.75 (s, 1H), 12.52 (s, 1H).

### Beispiel 15

### 6-(Difluormethyl)-N-[6-(2-hydroxypropan-2-yl)-2-(3-hydroxypropyl)-2H-indazol-5-yl]pyridin-2-carboxamid

### Stufe A:

### Herstellung von N-[2-(3-{[tert-Butyl(dimethyl)silyl]oxy}propyl)-6-(2-hydroxypropan-2-yl)-2H-indazol-5-yl]-6-(difluormethyl)pyridin-2-carboxamid

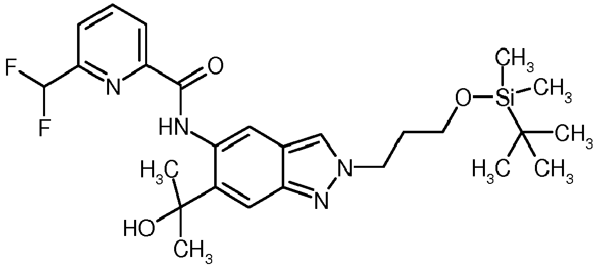

Ein Mischung aus 250 mg 6-(Difluormethyl)-N-[6-(2-hydroxypropan-2-yl)-1H-indazol-5-yl]pyridin-2-carboxamid (Intermediat 5-2), 48 mg Kaliumiodid und 239 mg Kaliumcarbonat in 2.5 ml DMF wurde 15 min bei Raumtemperatur gerührt. Man addierte 219 mg (0.87 mmol, 1.5 Äquivalente) (3-Brompropoxy)(tert-butyl)dimethylsilan und rührte 3 h bei 110°C. Man gab erneut 1 Äquivalent (3-Brompropoxy)(tert-butyl)dimethylsilan zu und rührte 4 h bei 100°C. Man versetzte mit Wasser, extrahiert mit Ethylacetat, wusch mit wässriger Kochsalzlösung, filtrierte durch einen wasserabweisenden Filter und engte ein. Der Rückstand wurde säulenchromatographisch gereinigt (Hexan/Ethylacetat). Es wurden 92 mg der Titelverbindung erhalten.

### Stufe B:

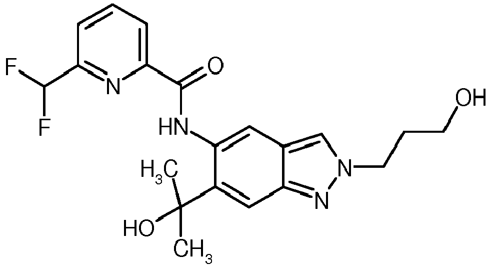

Analog zur Herstellung von Beispiel 6, Stufe B, wurden 92 mg N-[2-(3-{[tert-Butyl(dimethyl)silyl]oxy}propyl)-6-(2-hydroxypropan-2-yl)-2H-indazol-5-yl]-6-(difluormethyl)pyridin-2-carboxamid mit 0.53 ml einer 1 M Lösung Tetrabutylammoniumfluorid in THF innerhalb von 1 h umgesetzt. Nach wässriger Aufarbeitung wie bei Beispiel 6 und Reinigung durch präparative HPLC wurden 46 mg der Titelverbindung erhalten. UPLC-MS (Methode A1): Rt = 0.92 min (UV Detektor: TIC), gefundene Masse 404.00.
¹H-NMR (400MHz, DMSO-d₆): 6 [ppm]= 1.64 (s, 6H), 2.05 (quin, 2H), 3.35 - 3.46 (m), 4.45 (t, 2H), 4.64 (t, 1H), 5.99 (s, 1H), 7.04 (t, 1H), 7.57 (s, 1H), 7.95 - 7.99 (m, 1H), 8.25 - 8.36 (m, 3H), 8.73 (s, 1H), 12.50 (s, 1H).

### Beispiel 16

### N-[6-(2-Hydroxypropan-2-yl)-2-(4,4,4-trifluorbutyl)-2H-indazol-5-yl]-6-(trifluormethyl)pyridin-2-carboxamid

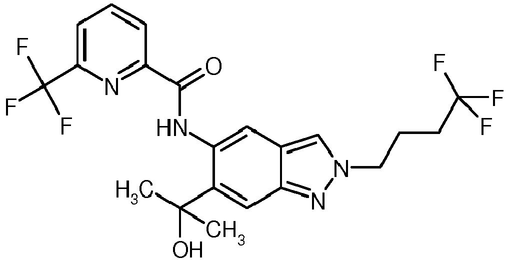

Eine Mischung aus 210 mg (0.58 mmol) N-[6-(2-Hydroxypropan-2-yl)-1H-indazol-5-yl]-6-(trifluormethyl)pyridin-2-carboxamid (Intermediat 5-1) in 3 ml DMF wurde mit 0.11 ml 1,1,1-Trifluor-4-iodbutan und 239 mg Kaliumcarbonat versetzt und die Mischung wurde 6 h bei 80°C gerührt. Nach Addition von Wasser wurde dreimal mit Ethylacetat extrahiert und die vereinigten organischen Phasen wurden mit gesättigter Kochsalzlösung, durch einen wasserabweisenden Filter filtriert und eingeengt. Das Rohprodukt wurde durch präparative HPLC gereinigt. Es wurden 19 mg der Titelverbindung erhalten.
UPLC-MS (Methode A1): Rₜ = 1.27 min (UV Detektor: TIC), gefundene Masse 474.15.
¹H-NMR (400MHz, DMSO-d₆): 6 [ppm]= 1.62 (s, 6H), 2.10 - 2.33 (m), 4.49 (t, 2H), 5.94 (s, 1H), 7.59 (s, 1H), 8.13 - 8.18 (m, 1H), 8.32 - 8.41 (m, 2H), 8.41 - 8.47 (m, 1H), 8.72 (s, 1H), 12.35 (s, 1H).

### Beispiel 17

### N-{6-(2-Hydroxypropan-2-yl)-2-[3-(trifluormethoxy)propyl]-2H-indazol-5-yl}-6-(trifluormethyl)pyridin-2-carboxamid

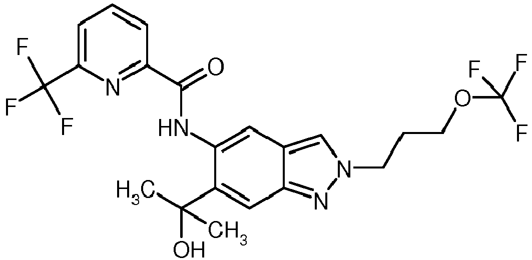

150 mg N-[6-(2-Hydroxypropan-2-yl)-1H-indazol-5-yl]-6-(trifluormethyl)pyridin-2-carboxamid (Intermediat 5-1) wurden in 2 ml THF vorgelegt. Man addierte 58 mg 3-(Trifluormethoxy)propan-1-ol, 131 mg Triphenylphosphin und 71 µl Diisopropylazodicarboxylat (DIAD, CAS 2446-83-5) und rührte 19 h bei Raumtemperatur. Man versetzte mit 0.83 ml Natronlauge (2M) und rührte 5 h bei 40°C. Man verdünnte mit Wasser, extrahierte dreimal mit Ethylacetat, engte die vereinigten organischen Phasen ein und reinigte durch präparative HPLC Es wurden 16 mg der Titelverbindung als Rohprodukt erhalten.

UPLC-MS (Methode A2): Rₜ = 1.26 min (UV Detektor: TIC), gefundene Masse 490.14. ¹H-NMR (400MHz, DMSO-d₆, ausgewählte Signale): 6 [ppm]= 1.61 (s, 6H), 1.84 (d, 1H), 2.32 (quint., 2H), 4.08 (t, 2H), 4.51 (t, 2H), 7.58 (s, 1H), 8.15 (d, 1H), 8.31 - 8.39 (m, 2H), 8.44 (d, 1H), 8.72 (s, 1H), 12.35 (s, 1H).

### Beispiel 18

### N-{6-(2-Hydroxypropan-2-yl)-2-[3-(2,2,2-trifluorethoxy)propyl]-2H-indazol-5-yl}-6-(trifluormethyl)pyridin-2-carboxamid

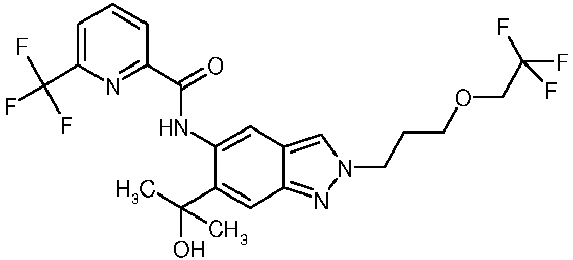

Analog zur Herstellung von Beispiel 11 (Herstellungsmethode 1) wurden 52 mg Methyl-2-[3-(2,2,2-trifluorethoxy)propyl]-5-({[6-(trifluormethyl)pyridin-2-yl]carbonyl}amino)-2H-indazol-6-carboxylat (Intermediat 4-10) in 3 ml THF mit 2 mal 171 Microliter 3M Magnesiumbromidlösung in Diethylether umgesetzt. Nach Reinigung durch präparative HPLC wurden 12 mg der Titelverbindung erhalten.

UPLC-MS (Methode A1): Rₜ = 1.25 min (UV Detektor: TIC), gefundene Masse 504.16. ¹H-NMR (500 MHz, DMSO-d₆): 6 [ppm]= 1.63 (s, 6H), 2.20(quin, 2H), 3.58(t, 2H),4.05(q, 2H), 4.47(t, 2H),5.94(s, 1H), 7.58 (s, 1H), 8.15 (dd, 1H), 8.32 (s, 1H), 8.36 (t, 1H), 8.45(d, 1H), 8.73 (s, 1H), 12.36 (s,1H).

### Beispiel 19

### 5-Fluor-N-[2-(3-hydroxy-3-methylbutyl)-6-(2-hydroxypropan-2-yl)-2H-indazol-5-yl]-6-methylpyridin-2-carboxamid

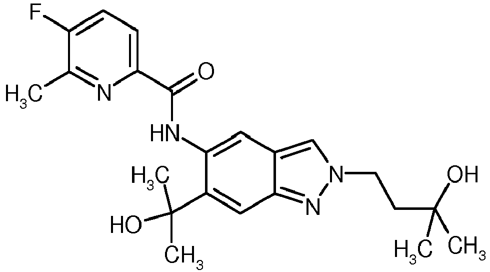

228 mg Methyl-5-{[(5-fluor-6-methylpyridin-2-yl)carbonyl]amino}-2-(3-hydroxy-3-methylbutyl)-2H-indazol-6-carboxylat (Intermediat 4-8) wurden in 4.5 ml THF vorgelegt und mit einem Eiskältebad abgekühlt. Man addierte 0.63 ml 3M Methylmagnesiumbromidlösung (in Diethylether) und ließ 2 h unter Eisbadkühlung und 21 h bei Raumtemperatur rühren. Das Reaktionsgemisch wurde mit gesättigter wässriger Ammoniumchloridlösung versetzt und dreimal mit Ethylacetat extrahiert. Die vereinigten organischen Phasen wurden eingeengt. Der Rückstand wurde durch präparative HPLC gereinigt. Es wurden 82 mg der Titelverbindung erhalten.

UPLC-MS (Methode A2): Rt = 1.03 min (UV Detektor: TIC), gefundene Masse 414.21. ¹H-NMR (400MHz, DMSO-d₆): 6 [ppm]= 1.13 (s, 6H), 1.63 (s, 6H), 1.99 - 2.05 (m, 2H), 2.55 - 2.59 (m, 3H), 4.42 - 4.50 (m, 3H), 5.95 (s, 1H), 7.54 (s, 1H), 7.83 (t, 1H), 8.05 (dd, 1H), 8.31 (s, 1H), 8.68 (s, 1H), 12.33 (s, 1H).

### Beispiel 20

### N-[2-(3-Hydroxy-3-methylbutyl)-6-(2-hydroxypropan-2-yl)-2H-indazol-5-yl]-6-methylpyridin-2-carboxamid

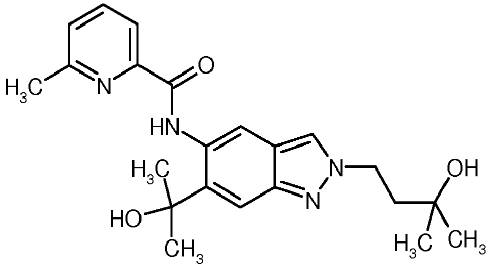

278 mg Methyl-2-(3-hydroxy-3-methylbutyl)-5-{[(6-methylpyridin-2-yl)carbonyl]amino}-2H-indazol-6-carboxylat (Intermediat 4-9) wurden in 5.0 ml THF vorgelegt und mit einem Eiskältebad abgekühlt. Man addierte 0.97 ml 3M Methylmagnesiumbromidlösung (in Diethylether) und ließ 2 h unter Eisbadkühlung und 20.5 h bei Raumtemperatur rühren. Man addierte erneut 0.48 ml 3M Methylmagnesiumbromidlösung und ließ 67 h bei Raumtemperatur rühren. Man versetzte mit gesättigter wässriger Ammoniumchloridlösung, extrahierte dreimal mit Ethylacetat, wusch mit Kochsalzlösung, filtrierte über einen wasserabweisenden Filter und engte ein. Der Rückstand wurde durch präparative HPLC gereinigt. Es wurden 111 mg der Titelverbindung erhalten.
UPLC-MS (Methode A2): Rₜ = 0.97 min (UV Detektor: TIC), gefundene Masse 396.22.
¹H-NMR (400MHz, DMSO-d₆): 6 [ppm]= 1.15 (s, 6H), 1.64 (s, 6H), 2.00 - 2.08 (m, 2H), 2.61 (s, 3H), 4.41 - 4.59 (m, 3H), 5.92 (s, 1H), 7.50 (dd, 1H), 7.56 (s, 1H), 7.90 - 7.99 (m, 2H), 8.33 (s, 1H), 8.70 (s, 1H), 12.39 (s, 1H).

### Beispiel 21

### 6-(2-Hydroxypropan-2-yl)-N-[6-(2-hydroxypropan-2-yl)-2-(4,4,4-trifluorbutyl)-2H-indazol-5-yl]pyridin-2-carboxamid

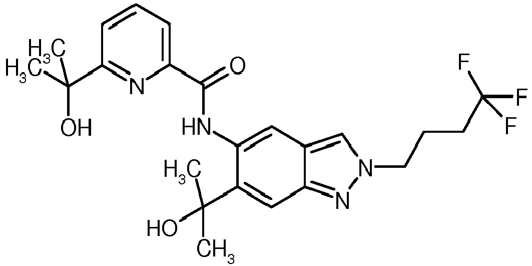

Eine Lösung aus 72 mg (0.155 mmol) Methyl-5-({[6-(2-hydroxypropan-2-yl)pyridin-2-yl]carbonyl}amino)-2-(4,4,4-trifluorbutyl)-2H-indazol-6-carboxylat (Intermediat 4-7) in 10 ml THF wurde in einem Eiswasser-Kältebad gekühlt. Man addierte 0.26 ml einer 3M Methylmagnesiumbromidlösung in Diethylether, rührte 2 h und danach 20 h bei Raumtemperatur. Man addierte erneut 1 Äquivalent der 3M Methylmagnesiumbromidlösung und rührte 24 h bei Raumtemperatur. Man addierte gesättigte wässrige Ammoniumchloridlösung, extrahierte dreimal mit Ethylacetat, wusch mit Kochsalzlösung und engte ein. Nach präparativer HPLC wurden 22 mg (31% d. Th.) der Titelverbindung erhalten.

UPLC-MS (Methode A2): Rₜ = 1.15 min (UV Detektor: TIC), gefundene Masse 464.20. ¹H-NMR (400MHz, DMSO-d₆): 6 [ppm]= 1.56 (s, 6H), 1.64 (s, 6H), 2.07 - 2.34 (m, 4H), 4.49 (t, 2H), 5.32 (s, 1H), 6.05 (s, 1H), 7.60 (s, 1H), 7.87 (dd, 1H), 7.99 - 8.05 (m, 2H), 8.35 (s, 1H), 8.79 (s, 1H), 12.45 (s, 1H).

### Bewertung der physiologischen Wirksamkeit

### IRAK4-Kinaseassay

Die IRAK4-inhibitorische Aktivität der erfindungsgemäßen Substanzen der Formel (I) als Bestandteile der erfindungsgemäßen Kombinationen wurde in dem nachfolgend beschriebenen Irak4-TR-FRET-Assay gemessen (TR-FRET = Time Resolved Fluorescence Resonance Energy Transfer).

Rekombinantes Fusionsprotein aus N-terminalem GST (Glutathion-S-Transferase) und humanem Irak4, exprimiert in Bakulovirus-infizierten Insektenzellen (Hi5, BTI-TN-5B1-4, Zelllinie gekauft von Invitrogen, Katalog-Nr. B855-02) und gereinigt via Affinitätschromatographie, wurde als Enzym verwendet. Als Substrat für die Kinasereaktion wurde das biotinylierte Peptid Biotin-Ahx-KKARFSRFAGSSPSQASFAEPG (C-Terminus in Amid-Form) verwendet, das z.B. bei der Firma Biosyntan GmbH (Berlin-Buch) gekauft werden kann.
Für den Assay wurden 11 verschiedene Konzentrationen im Bereich von 20 µM bis 0,073 nM aus einer 2 mM Lösung der Testsubstanz in DMSO hergestellt. 50 nl der jeweiligen Lösung wurden in eine schwarze low-volume 384well-Mikrotiterplatte (Greiner Bio-One, Frickenhausen, Deutschland) pipettiert, 2 µl einer Lösung von Irak4 in Assaypuffer [50 mM HEPES pH 7.5, 5 mM MgCl2, 1.0 mM Dithiothreitol, 30 µM aktiviertes Natriumorthovanadat, 0,1 % (w/v) bovines gamma-Globulin (BGG) 0,04% (v/v) Nonidet-P40 (Sigma)] hinzugegeben und die Mischung für 15 min inkubiert, um eine Vorbindung der Substanzen an das Enzym vor der Kinasereaktion zu ermöglichen. Dann wurde die Kinasereaktion gestartet durch Zugabe von 3 µl einer Lösung von Adenosine-tri-phosphat (ATP, 1,67 mM =Endkonzentration in 5 µl Assayvolumen ist 1 mM) und Peptidsubstrat (0,83 µM =Endkonzentration in 5 µl Assayvolumen ist 0,5 µM) in Assaypuffer und die resultierende Mischung für die Reaktionszeit von 45 min bei 22°C inkubiert. Die Konzentration des Irak4 wurde an die jeweilige Aktivität des Enzyms angepasst und so eingestellt, dass der Assay im linearen Bereich arbeitete. Typische Konzentrationen lagen in der Größenordnung von etwa 0,2 nM. Die Reaktion wurde gestoppt durch Zugabe von 5 µl einer Lösung von TR-FRET-Detektionsreagentien [0,1 µM Streptavidin-XL665 (Cisbio Bioassays; Frankreich, Katalog-Nr. 610SAXLG) und 1,5 nM Anti-phosho-Serin Antikörper [Merck Millipore, "STK Antibody", Katalog-Nr. 35-002] und 0,6 nM LANCE EU-W1024-markierter anti-Maus-IgG-Antikörper (Perkin-Elmer, Produkt-Nr. AD0077, alternativ kann ein Terbium-Kryptat-markierter anti-Maus-IgG-Antikörper von Cisbio Bioassays verwendet werden) in wässriger EDTA-Lösung (100 mM EDTA, 0.4 % [w/v] bovines Serumalbumin [BSA] in 25 mM HEPES pH 7,5].

Die resultierende Mischung wurde 1 h bei 22°C inkubiert, um die Bildung eines Komplexes aus dem biotinylierten phosphorylierten Substrat und den Detektionsreagentien zu ermöglichen. Anschließend wurde die Menge des phosphorylierten Substrates ausgewertet durch eine Messung des Resonanz-Energietransfers vom Europium-Chelat markierten anti-Maus-IgG-Antikörper zum Streptavidin-XL665. Hierzu wurden in einem TR-FRET-Meßgerät, z.B. einem Rubystar (BMG Labtechnologies, Offenburg, Germany) oder einem Viewlux (Perkin-Elmer), die Fluoreszenz-Emissionen bei 620 nm and 665 nm nach Anregung bei 350 nm gemessen. Das Verhältnis der Emissionen bei 665 nm und 622 nm wurde als Maß für die Menge des phosphorylierten Substrates genommen. Die Daten wurden normalisiert (Enzymreaktion ohne Testsubstanz = 0 % Inhibition, alle anderen Assaykomponenten aber kein Enzym = 100 % Inhibition). Üblicherweise wurden die Testsubstanzen auf derselben Mikrotiterplatten bei 11 verschiedenen Konzentrationen im Bereich von 20 µM bis 0,073 nM getestet (20 µM, 5,7 µM, 1,6 µM, 0,47 µM, 0,13 µM, 38 nM, 11 nM, 3,1 nM, 0,89 nM, 0,25 nM und 0,073 nM). Die Verdünnungsreihen wurden vor dem Assay hergestellt (2 mM bis 7,3 nM in 100 % DMSO) durch serielle Verdünnungen. Die IC50-Werte wurden kalkuliert mit einem 4-Parameter-Fit.

**Tabelle 1: IC₅₀-Werte der Beispielverbindungen der Formel (I) als Bestandteile der erfindungsgemäßen Kombinationen im IRAK4 Kinase Assay**

| Beispiel | IC₅₀ [nM] |
|---|---|
| 1 | 30.6 |
| 2 | 135.6 |
| 3 | 7.2 |
| 4 | 52.7 |
| 5 | 264.5 |
| 6 | 35.7 |
| 7 | 867.3 |
| 8 | 15.0 |
| 9 | 103.8 |
| 10 | 18.5 |
| 11 | 3.4 |
| 12 | 10.7 |
| 13 | 1.3 |
| 14 | 10.8 |
| 15 | 12.3 |
| 16 | 21.5 |
| 17 | 36.0 |
| 18 | 47.5 |
| 19 | 8.9 |
| 20 | 13.3 |
| 21 | 117.2 |

### TNF-α Ausschüttung in THP-1 Zellen

Mithilfe dieses Tests können Substanzen auf ihre Fähigkeit hin getestet werden, TNF-α (Tumornekrosefaktor-alpha) Ausschüttung in THP-1 Zellen (humane monozytische akute Leukämie-Zellinie) zu inhibieren. TNF-α ist ein Zytokin, welches in inflammatorischen Prozessen beteiligt ist. Die TNF-α Ausschüttung wird in diesem Test ausgelöst durch Inkubation mit bakteriellem Lipopolysaccharid (LPS).

THP-1 Zellen werden in kontinuierlicher Suspensions-Zellkultur [RPMI 1460 Medium mit L-Glutamax (Gibco, Kat-Nr. 61870-044) supplementiert mit foetalem Kälberserum (FCS) 10% (Invitrogen, Kat-Nr. 10082-147), 1% Penicillin/Streptomycin (Gibco BRL, Kat-Nr. 15140-114)] gehalten und sollten eine Zellkonzentration von 1x10⁶ Zellen/ml nicht überschreiten. Der Assay erfolgte im Zellkulturmedium (RPMI 1460 Medium mit L-Glutamax supplemetiert mit FCS 10%).

Jeweils 2-2.5 µl der Zellsuspension (entspricht 4000 Zellen) wurden pro well in eine 384-well Testplatte Greiner Kat-Nr. 784076) dispensiert, in der sich jeweils 40-50 nl Substanz in 100% DMSO gelöst befanden. Hierbei wurden jeweils 10 verschiedene Konzentrationen im Bereich von 20 µM bis 0,073 nM je Substanz eingesetzt. Die Zellen wurden 15 min bei Raumtemperatur inkubiert. Anschließend wurden 2-2.5 µl pro well 0.1 µg/ml LPS (Sigma, *Escherichia coli* 055:B5, Kat-Nr. L5418) gelöst in Zellkulturmedium dispensiert (finale Konzentration 0.05 µg/ml). Als Neutralkontrolle wurden Zellen mit 0.05 µg/ml LPS und 1% DMSO und als Inhibitorkontrolle nur mit 1% DMSO behandelt.

Die Platten wird 30 sec bei 80g zentrifugiert und 17 h bei 37°C, 5% CO₂ und 95% Luftfeuchtigkeit inkubiert. Zur Bestimmung der Menge an TNF-α wurde der TNF-alpha HTRF Detection Kit (Cisbio, Kat-Nr. 62TNFPEB/C) verwendet. Hierzu wurden jeweils 2 µl der Detektionslösung, bestehend aus Anti-TNF-α-XL665 Konjugat und Anti-TNF-α-Kryptat Konjugat gelöst nach Anweisung des Herstellers im Rekonstitutionspuffer, für den HTRF (Homogeneous Time-Resolved Fluorescence) Test zugegeben. Nach Zugabe wurde entweder 3 h bei Raumtemperatur oder über Nacht bei 4°C inkubiert. Anschließend wurden die Signale mit einem HTRF-fähigen wie dem BMG PheraStar bei 620/665nm Messgerät ausgelesen.

Die Wirkung der Substanzen wird als Verhältnis zwischen Neutral- und Inhibitorkontrolle in Prozent ausgedrückt. Die IC₅₀-Werte wurden mit einem 4-Parameter-Fit kalkuliert.

**Tabelle 2: IC₅₀-Werte der Beispielverbindungen bezüglich der TNF-α Ausschüttung in THP-1 Zellen**

| Beispiel | IC₅₀ [µM] |
|---|---|
| 1 | 1.0 |
| 2 | 15.1 |
| 3 | 0.7 |
| 4 | 5.6 |
| 5 | 5.4 |
| 6 | 0.9 |
| 7 | 16.4 |
| 8 | 1.0 |
| 9 | 6.5 |
| 10 | 1.0 |
| 11 | 0.2 |
| 12 | 0.3 |
| 13 | 0.1 |
| 14 | 0.2 |
| 15 | 0.2 |
| 16 | 0.2 |
| 17 | 0.5 |
| 18 | 0.3 |
| 19 | 0.1 |
| 20 | 0.2 |
| 21 | 1.8 |

### In Vitro LPS (Lipopolysaccharide)-induzierte Zytokinproduktion in humanen PBMCs (peripheral blood mononuclear cells)

Die Wirkung der Verbindungen der allgemeinen Formel (I) als Bestandteile der erfindungsgemäßen Kombinationen auf die induzierte Zytokinproduktion in humanen PBMCs wurde untersucht. Hierbei erfolgte die Induktion der Zytokinproduktion durch LPS, einen TLR4 Liganden, welcher zur Aktivierung des IRAK4-vermittelte Signalwege führt.

Die Gewinnung der humanen PBMCs erfolgte aus anti-koaguliertem humanem Vollbut. Hierzu wurde in Leucosep-Röhrchen 15 ml Ficoll-Paque (Biochrom, Kat-Nr. L6115) vorgelegt und 20 ml Humanblut hinzugefügt. Nach Zentrifugation des Blutes bei 800g für 15 min bei Raumtemperatur wurde Plasma inklusive der Thrombozyten abgenommen und verworfen. Die PBMCs wurden in Zentrifugenröhrchen überführt und mit PBS (phosphatgepufferte Salzlösung) (Gibco, Kat-Nr. 14190) aufgefüllt. Die Zellsuspension wurde bei 250g für 10 min bei Raumtemperatur zentrifugiert und der Überstand verworfen. Die Resuspension der PBMCs erfolgte in Komplettmedium (RPMI 1640, ohne L-Glutamin (PAA, Kat-Nr. E15-039), 10% FCS; 50 U/ml Penicillin, 50 µg/ml Streptomycin (PAA, Kat-Nr. P11-010) und 1% L-Glutamin (Sigma, Kat-Nr. G7513)).

Auch der Assay erfolgte in Komplettmedium. Die PBMCs wurden in 96-well Platten mit einer Zelldichte von 2.5x10⁵ Zellen/well ausgesät. Die Verbindungen wurden in einem konstanten Volumen von 100% DMSO seriell verdünnt und in dem Assay mit 8 verschiedenen Konzentrationen im Bereich von 10 µM bis 3 nM so eingesetzt, so dass die finale DMSO Konzentration 0.4% DMSO betrug. Die Zellen wurden damit für 30 min vor der eigentlichen Stimulation vorinkubiert. Zur Induktion der Zytokinsekretion erfolgte eine Stimulation mit 0.1 µg/ml LPS (Sigma, *Escherichia coli* 0128:B12, Kat-Nr. L2887) für 24 Stunden. Die Bestimmung der Zellviabilität erfolgte unter Verwendung des CellTiter-Glo Luminescent Assay (Promega, Kat-Nr. G7571 (G755/G756A)) nach Anweisung des Herstellers. Die Bestimmung der Menge an sekretiertem TNF- 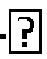 im Zellkulturüberstand erfolgte mittels Human Prolnflammatory 9-Plex Tissue Culture Kit (MSD, Kat.-Nr. K15007B) nach Anweisung des Herstellers. Exemplarisch sind die Beispielverbindung 11 und die Beispielverbindung 12 mit einer Aktivität ≤ 1µM genannt.

### In vivo B-Zell-Lymphom-assoziierte Xenotransplantationsmodelle

Die Anti-Tumoraktivität der Verbindungen der allgemeinen Formel (I) als Bestandteile der erfindungsgemäßen Kombinationen wurde in murinen Xenotransplantationsmodellen untersucht. Hierzu wurde weiblichen C.B-17 SCID Mäusen subkutan Tumorzelllinien von humanen B-Zell-Lymphomen, z. B. TMD-8, implantiert. Bei einer mittleren Tumorgröße von 20-30 mm² begann die monotherapeutische Behandlung oder die Behandlung in Kombination mit der Standardtherapie Ibrutinib, welche jeweils oral verabreicht wurden. Zuvor erfolgte eine Randominisierung der Tiere. Die Behandlung wurde beendet, sobald die unbehandelte Kontrollgruppe Tumoren einer Fläche ≤ 150 mm² aufwies. Die Tumorgröße als auch das Körpergewicht wurden drei Mal wöchentlich bestimmt. Änderungen des Körpergewichts stellten ein Maß für die behandlungsbedingte Toxizität dar (>10% = kritisch, Behandlungsstopp bis zur Genesung, >20% toxisch, Beendigung). Die Tumorfläche wurde mittels elektronischem Messschieber erfasst [Länge (mm) x Breite (mm)]. Die Anti-Tumor-Wirksamkeit definierte das Verhältnis der Tumorfläche von Behandlung vs. Kontrolle [Tumorfläche der Behandlungsgruppe am Tag X/Tumorfläche der Kontrollgruppe am Tag X]. Eine Verbindung mit einem T/C größer gleich 0.5 wurde als aktiv (wirksam) definiert. Die statistische Analyse erfolgte unter Verwendung der einfaktoriellen ANOVA und dem Vergleich zur Kontrollgruppe mittels paarweiser Vergleichsanalyse (Dunnett-Test).

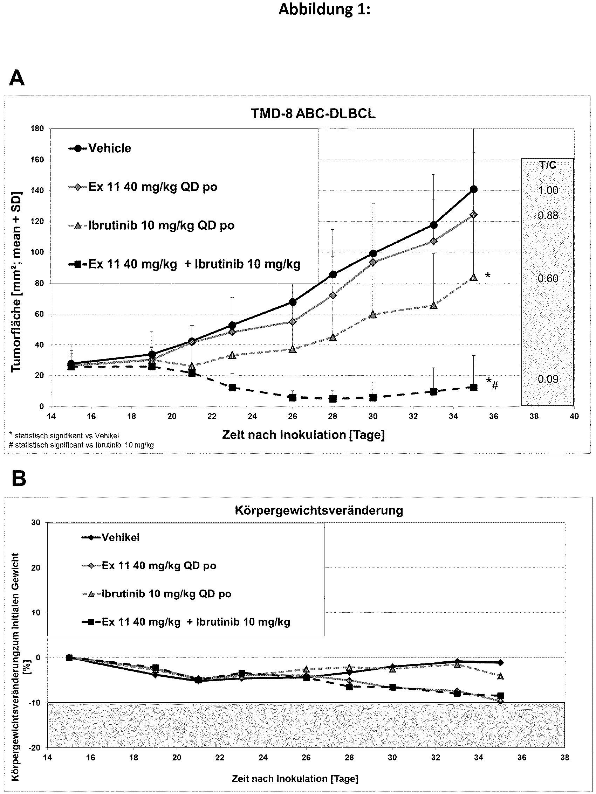

Die Abbildung 1 zeigt die Wirksamkeit von Beispielverbindung 11 in Monotherapie und in Kombination mit Ibrutinib in der Behandlung von humanen TMD-8 ABC-DLBCL Tumoren. TMD-8 Tumorzellen wurden subkutan am Tag 0 in weibliche C.B-17 SCID Mäuse implantiert. Die Behandlung wurde an Tag 15 bei einer Tumorfläche von etwa 26 mm² gestartet. Beispielverbindung 11 wurde oral bei einer Dosierung von 40 mg/kg täglich verabreicht. Ibrutinib wurde ebenfalls oral bei einer Dosierung von 10 mg/kg täglich verabreicht. Das Tumorwachstum wurde durch Bestimmung der Tumorfläche (siehe Abbildung 1, oberer Teil A), das Befinden der Tiere durch Bestimmung des Körpergewichtes (siehe Abbildung 1, unterer Teil B) beurteilt.

Die Beispielverbindung 11 zeigte keine inhibitorische Wirkung auf das Tumorwachstum von TMD-8, wenn sie als Monotherapie verabreicht wurde. Ibrutinib zeigte in Monotherapie eine moderate, im Vergleich zur Kontrollgruppe jedoch statistisch signifikante, inhibitorische Wirkung auf das Tumorwachstum von TMD-8, mit einem T/C-Wert von 0.60. In der Kombinationsbehandlung mit der Beispielverbindung 11 und Ibrutinib konnte eine deutliche Steigerung der Anti-Tumor-Wirkung verzeichnet werden, was sich im T/C-Wert von 0.09 sowie einer statistisch signifikant reduzierten Tumorfläche im Vergleich zu Kontrolle und Ibrutinib widerspiegelt.

Die Behandlungen wurden sehr gut vertragen, es war kein kritischer Gewichtsverlust zu verzeichnen.

Zusammenfassend konnte in dieser Studie gezeigt werden, dass durch die Kombination der Beispielverbindung 11 mit dem BTK Inhibitor Ibrutinib eine deutliche Steigerung der anti-tumoralen Wirkung der jeweiligen Monotherapien im Modell eines ABC-DLBCL erzielt werden kann.

### Abbildung 1

Anti-Tumor Aktivität der Beispielverbindung 11 in Monotherapie und in Kombination mit Ibrutinib in TMD-8 C.B-17 SCID Mäusen.

| TMD-8: humanes ABC-DLBCL Xenograft Modell | | | | |
|---|---|---|---|---|
| Studien-Nr. | ONC20114.00714 | | | |
| Su bstanz | Dosierung | T/C^{a} Fläche | Max. Gewichtsverlust^{b} (%) | Toxizität |
| Vehikel | 10 ml/kg QD p.o. + 10 ml/kg QD p.o. | 1.00 | - | 0/8 |
| Beispiel 11 | 40 mg/kg QD p.o. | 0.88 | -9 | 0/8 |
| Ibrutinib | 10 mg/kg QD p.o. | 0.60# | -5 | 0/8 |
| Beispiel 11 + Ibrutinib | 40 mg/kg QD p.o. + 10 mg/kg QD p.o. | 0.09*# | -8 | 0/8 |

| | | | | |
|---|---|---|---|---|
| * P<0.05 (verglichen mit Vehikelkontrolle) # P<0.05 (verglichen mit Ibrutinib Monotherapie) a) T/C=Verhältnis der Tumorfläche von Behandlung vs. Kontrolle [Tumorfläche der Behandlungsgruppe am Tag X/Tumorfläche der Kontrollgruppe am Tag X]. b) Körpergewichtsverlust: Änderungen des Körpergewichts im Vergleich zum initialen Körpergewicht bei Behandlungsstart (>10% = kritisch, Behandlungsstopp bis zur Genesung, >20% toxisch, Beendigung). | | | | |

Die Abbildung 1 zeigt die anti-Tumor Aktivität der Beispielverbindung 11 in Monotherapie und in Kombination mit Ibrutinib in TMD-8 C.B-17 SCID Mäusen. Legende Abbildung 1: Die Abkürzung Ex bedeutet Beispiel, QD bedeutet einmal pro Tag und po bedeutet peroral.

### Zellproliferations-Messung

Die antiproliferative Aktivität der Verbindungen der allgemeinen Formel (I) als Bestandteile der erfindungsgemäßen Kombinationen wurde in humanen ABC-DLBCL Zellen in vitro untersucht. Hierzu wurden 4000 TMD-8 oder HBL-1 Zellen (beide von ATCC) oder OCI-YL10 in 30 µpL/Kavität in Wachstumsmedium ((RPMI (Biochrom: FG 1215), 20% FCS (Biochrom: S 0615)) in eine 384- Kavitäten Platte (Perkin Elmer, weiß) überführt und über Nacht bei 37°C inkubiert. Nach 24h wurden Zellen einer Platte (0h Platte) mit 30 µL/Kavität CTG Lösung behandelt (Promega Cell Titer Glo (Katalog # G755B und G756B)), für 10 min bei Raumtemperatur inkubiert und die Lumineszenz mittels eines VICTOR V (Perkin Elmer) gemessen um die Zellviablilität zu Beginn der Behandlung zu bestimmen. Die Zellen der Testplatte wurden mit den Verbindungen der allgemeinen Formel (I) als Bestandteile der erfindungsgemäßen Kombinationen behandelt und für 72 Stunden bei 37°C inkubiert. Die Verbindungen wurden in einer 7-fachen Verdünnungsreihe-entweder alleine oder als Kombination zweier Verbindungen von verschiedenen Konzentrationen (Ratios Substanz 1 (Beispielverbindung der allgemeinen Formel (I) als Bestandteil der erfindungsgemäßen Kombinationen) und Substanz 2 (BTK Inhibitoren Ibrutinib oder RN486 oder AVL-292 oder CGI-1746 als Bestandteil der erfindungsgemäßen Kombinationen) : 1:0; 0,85:0,15; 0,7:0,3; 0,5:0,5; 0,3:0,7; 0,15:0,85; 0:1) mittels eines HP D300 Digital Dispensers zu den Zellen gegeben. Als Kontrolle wurden Zellen mit Vehikel (DMSO) behandelt. Nach 72h wurden die Zellen mit 30 µL/Kavität CTG Lösung behandelt (Promega Cell Titer Glo (Katalog # G755B und G756B)), für 10 min bei Raumtemperatur inkubiert und die Lumineszenz mittels eines VICTOR V (Perkin Elmer) gemessen um die Zellviablilität zum Ende der Behandlung zu bestimmen. Die prozentuale Beeinflussung des Zellwachstums und die daraus abgeleitete IC50 wurden unter Zuhilfenahme der Werte der 0h Platte (=maximale Inhibition) und der DMSO Kontrolle (= minimale Inhibition) für jede Testsubstanz bestimmt. Die IC50-Werte wurden mit einem 4-Parameter-Fit kalkuliert. Der kombinatorische Effekt von zwei Testsubstanzen wurde basierend auf der oben beschriebenen IC50- Bestimmung ermittelt. Der Kombinations-Index (Cl) wurde basierend auf der Formel von Chou, (Chou TC et al., Pharmacological Reviews September 2006), berechnet. Dieser Index erlaubt eine quantitative Bestimmung von Substanz-Interaktionen. Ein Cl < 1, =1, und > 1 beschreiben jeweils synergistische, additive oder antagonistische Effekt. Die Visualisierung erfolgte durch Isobologramme.

In der Kombinationsbehandlung mit der Beispielverbindung 3 oder 11 oder 12 oder 13 oder 19 und BTK Inhibitoren Ibrutinib oder RN486 oder AVL-292 oder CGI-1746konnte im Vergleich zu Einfachbehandlungen fast immer eine deutliche Steigerung der Anti-Tumor-Wirkung verzeichnet werden.

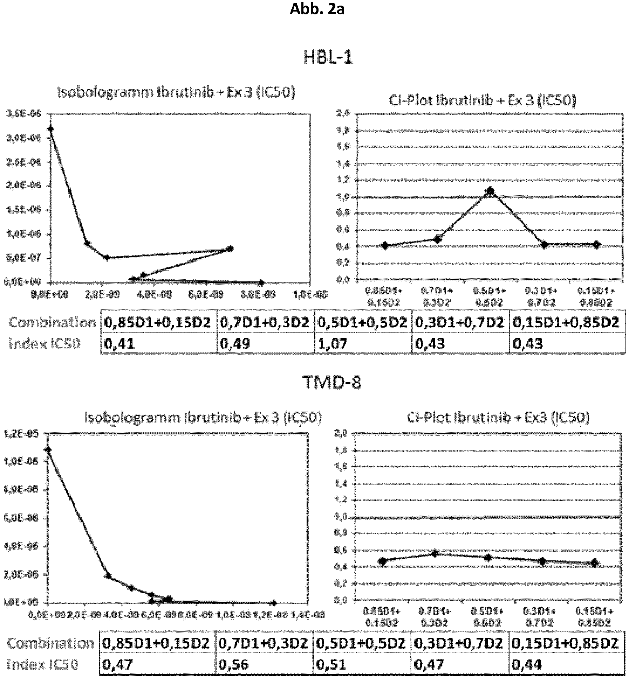

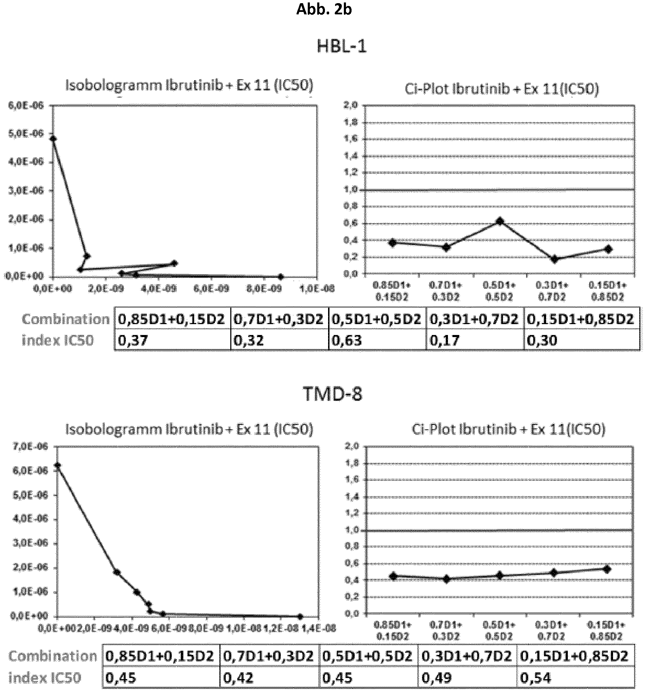

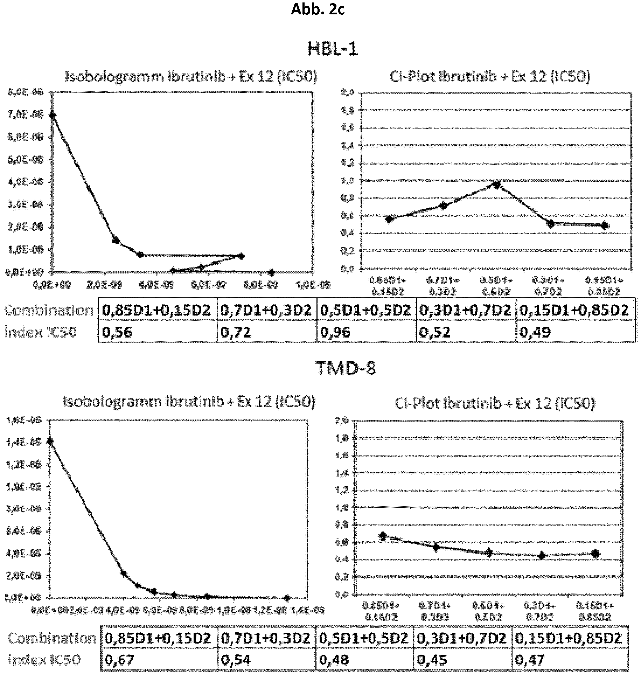

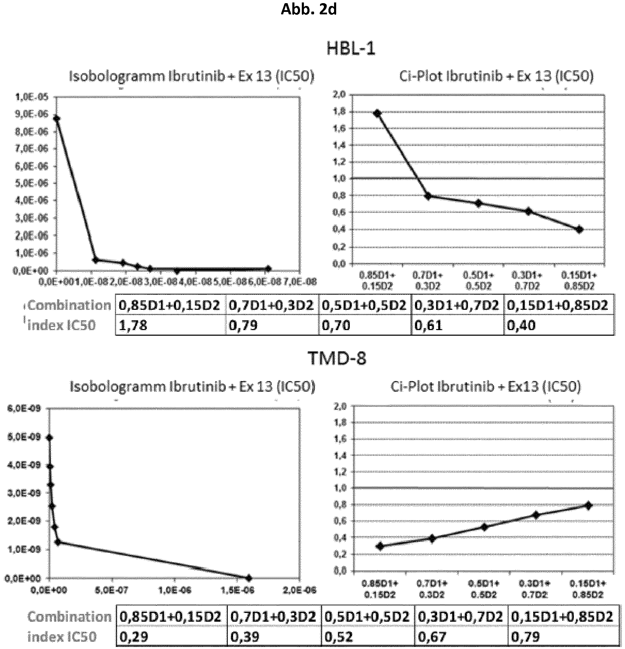

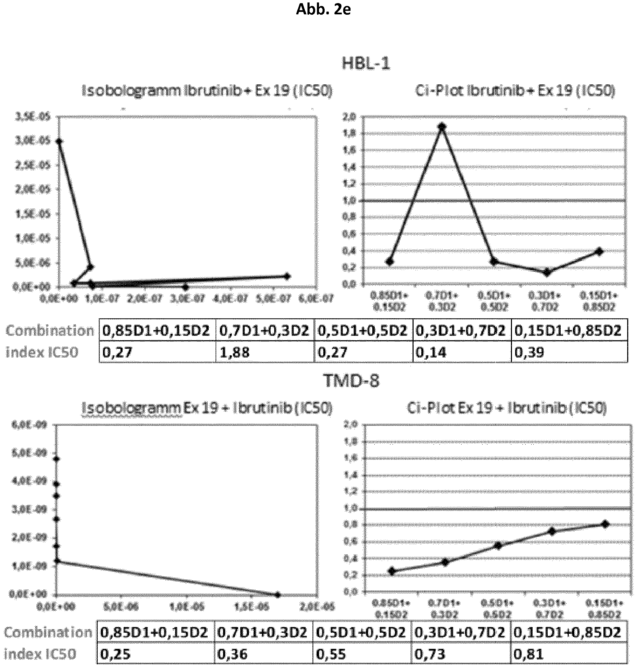

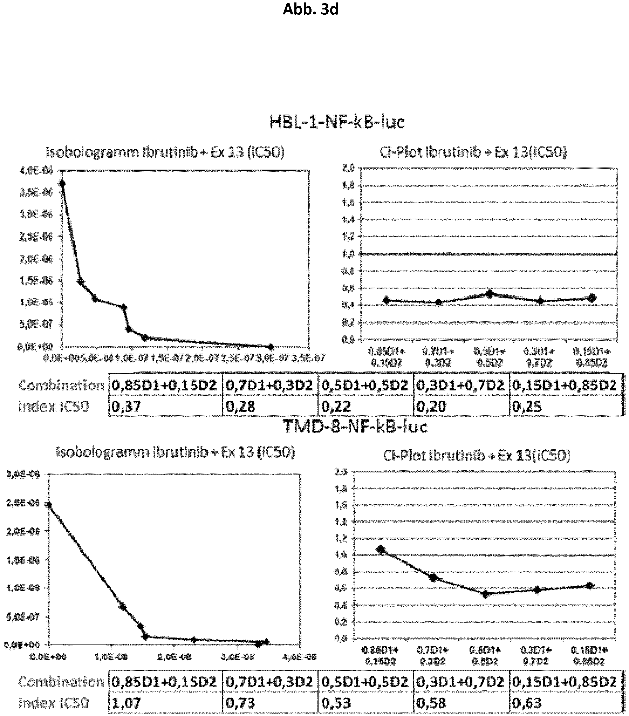

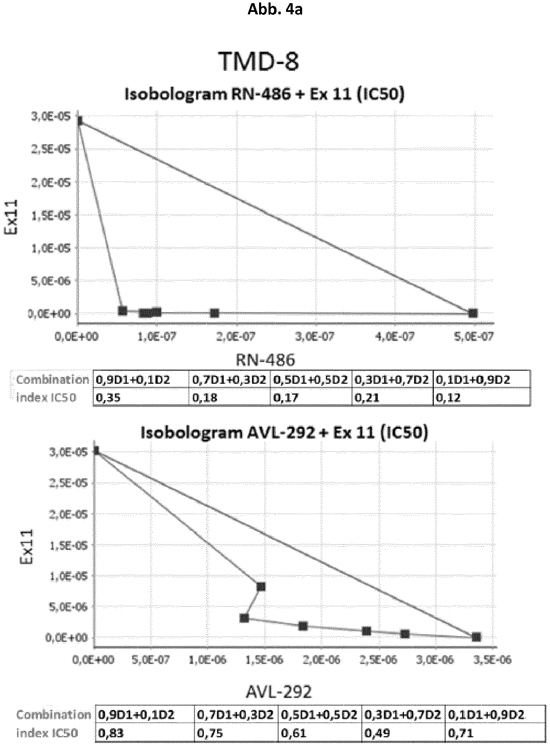

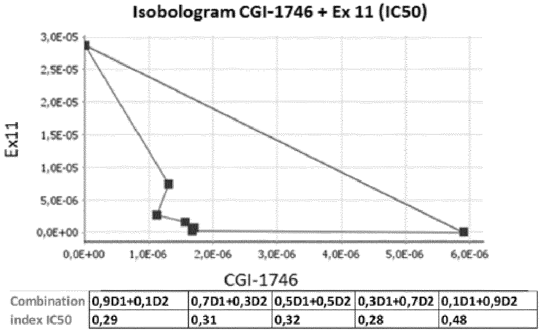

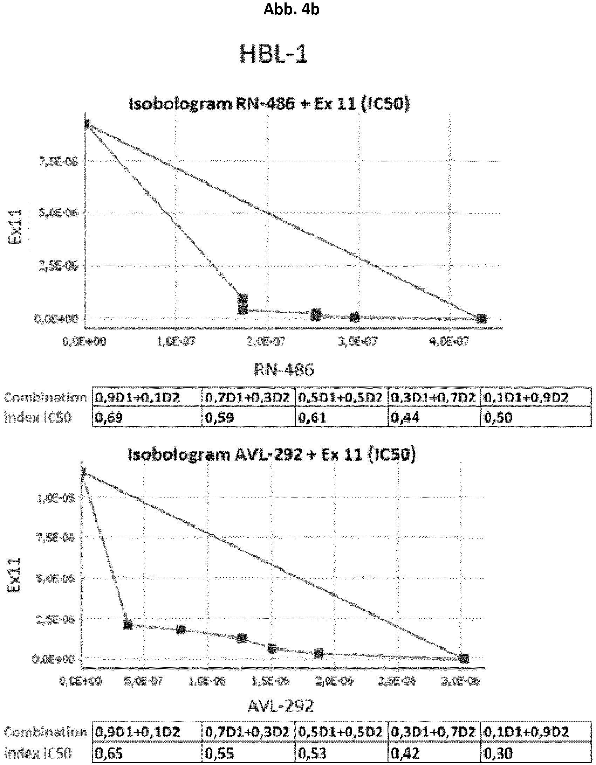

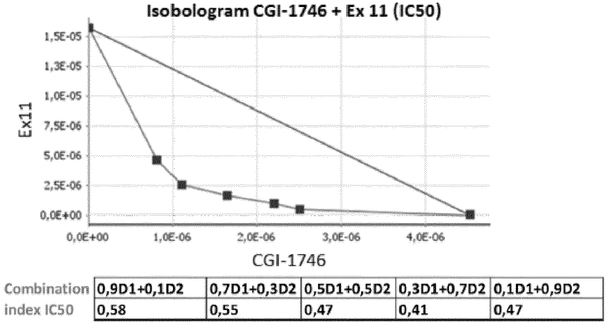

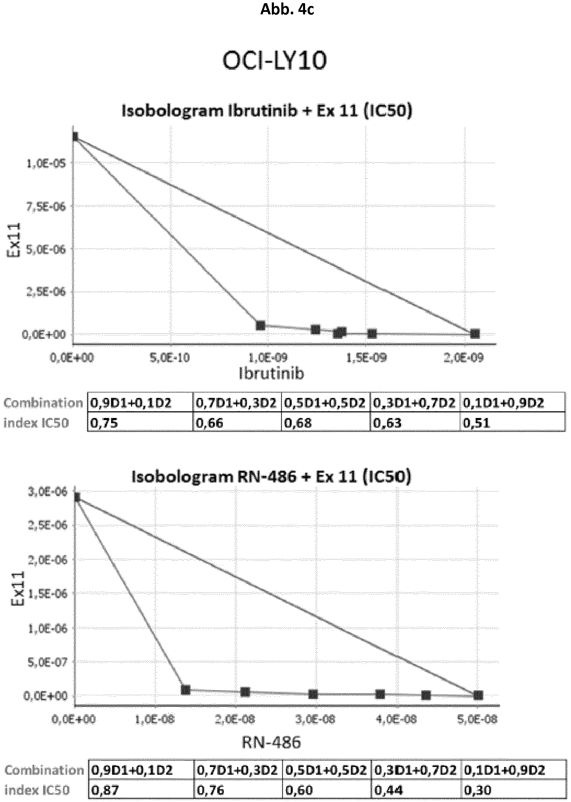

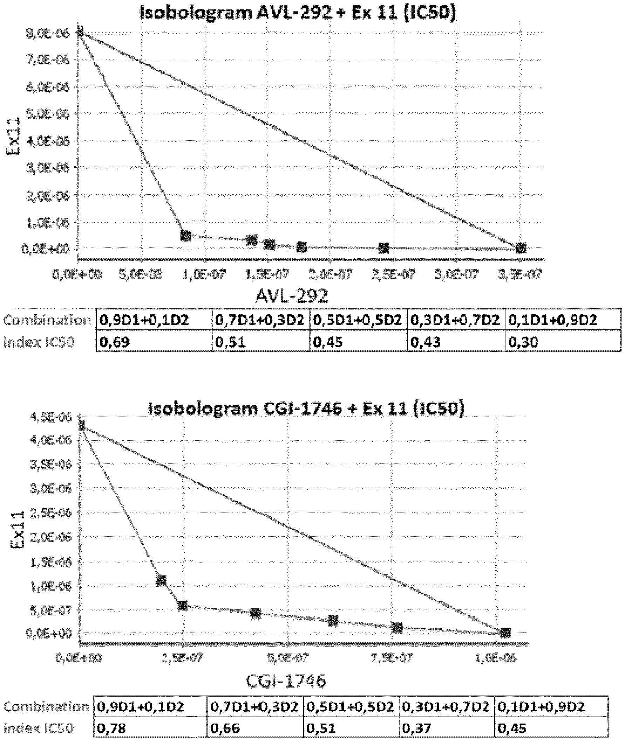

Die Abbildungen 2a bis 2e und 4a bis c (Ex bedeutet Beispielverbindung) zeigen die Ergebnisse einer kombinatorischen Zellproliferations-Messung in ABC-DLBCL Zell-Linien TMD-8 und HBL-1 und OCI-LY10 bei Kombination von BTK Inhibitoren Ibrutinib oder RN486 oder AVL-292 oder CGI-1746mit den Verbindungen der allgemeinen Formel (I) (Abbildung 2a: Ex 03; Abbildung 2b: Ex 11; Abbildung 2c: Ex 12; Abbildung 3d: Ex 13, Abbildung 2e: Ex 19). IC50 Isobologramme für die verschiedenen Kombinationen mit den jeweiligen Konzentrationen der Substanz 1 (D1) (Beispielverbindung der allgemeinen Formel (I) als Bestandteil der erfindungsgemäßen Kombinationen) auf der y- und Substanz 2 (D2) (BTK Inhibitoren Ibrutinib oder RN486 oder AVL-292 oder CGI-1746als Bestandteil der erfindungsgemäßen Kombinationen) auf der x-Achse sind dargestellt. Datenpunkte, die unter, auf oder über der Hypotenuse liegen indizieren jeweils synergistische, additive oder antagonistische Wirkung auf Zellproliferation.
Legende Abb. 2a: Effekt der Kombination von Ibrutinib mit Beispiel 03 auf Zellviabilität von TMD-8 und HBL-1 Zellen.
Legende Abb. 2b: Effekt der Kombination von Ibrutinib mit Beispiel 11 auf Zellviabilität von TMD-8 und HBL-1 Zellen.
Legende Abb. 2c: Effekt der Kombination von Ibrutinib mit Beispiel 12 auf Zellviabilität von TMD-8 und HBL-1 Zellen.
Legende Abb. 2d: Effekt der Kombination von Ibrutinib mit Beispiel 13 auf Zellviabilität von TMD-8 und HBL-1 Zellen.
Legende Abb. 2e: Effekt der Kombination von Ibrutinib mit Beispiel 19 auf Zellviabilität von TMD-8 und HBL-1 Zellen.
Legende Abb. 4a: Effekt der Kombination von BTK Inhibitoren RN486 oder AVL-292 oder CGI-1746 mit Beispiel 11 auf Zellviabilität von TMD-8 Zellen.
Legende Abb. 4b: Effekt der Kombination von BTK Inhibitoren RN486 oder AVL-292 oder CGI-1746 mit Beispiel 11 auf Zellviabilität von HBL-1 Zellen.
Legende Abb. 4c: Effekt der Kombination von BTK Inhibitoren Ibrutinib oder RN486 oder AVL-292 oder CGI-1746 mit Beispiel 11 auf Zellviabilität von OCI-LY10 Zellen.

### NF-kB-Reporter Messung

Die Wirkung der Verbindungen der allgemeinen Formel (I) als Bestandteile der erfindungsgemäßen Kombinationen auf den NF-kB Signalweg wurde in humanen DLBCL Zellen in vitro untersucht. 10,000 TMD-8-NF-kB-luc Zellen oder 10,000 HBL-1-NF-kB-luc Reporter-Zellen wurden in 30 µpL/Kavität in Wachstumsmedium ((RPMI (Biochrom: FG 1215), 20% FCS (Biochrom: S 0615)) in einer 384- Kavitäten Platte (Perkin Elmer, weiß) überführt und über Nacht bei 37°C inkubiert. Nach 24h wurden die Zellen mit den Testsubstanzen behandelt und für 6h bei 37°C inkubiert. Die Verbindungen wurden in einer 7-fachen Verdünnungsreihe - entweder alleine oder als Kombination zweier Verbindungen von verschiedenen Konzentrationen (Ratios Substanz 1 (Beispielverbindung der allgemeinen Formel (I) als Bestandteil der erfindungsgemäßen Kombinationen) und Substanz 2 (BTK Inhibitor Ibrutinib als Bestandteil der erfindungsgemäßen Kombinationen) : 1:0; 0,85:0,15; 0,7:0,3; 0,5:0,5; 0,3:0,7; 0,15:0,85; 0:1) mittels eines HP D300 Digital Dispensers zu den Zellen gegeben. Als Kontrolle wurden Zellen mit dem Vehikel (DMSO) behandelt. Nach 6h wurden die Zellen mit 30 µpL/Kavität One-Glo Lösung (Promega E6110) behandelt, für 10 min bei Raumtemperatur inkubiert und die Lumineszenz mittels eines VICTOR V (Perkin Elmer) gemessen, um die NF-KB Reporter Aktivität am Ende der Behandlung zu bestimmen. Die prozentuale Beeinflussung der NF-KB Reporter Aktivität und die daraus abgeleitete IC50 wurden unter Zuhilfenahme der Werte eines bekannten NF-KB Inhibitors (= maximale Inhibition) sowie der DMSO Kontrolle (= minimale Inhibition) für jede Testsubstanz bestimmt. Die IC₅₀-Werte wurden mit einem 4-Parameter-Fit kalkuliert. Der kombinatorische Effekt von zwei Testsubstanzen wurde basierend auf der oben beschriebenen IC50-Bestimmung ermittelt. Der Kombinations-Index (CI) wurde basierend auf der Formel von Chou (Chou TC et al., Pharmacological Reviews September 2006) berechnet. Dieser Index erlaubt eine quantitative Bestimmung von Substanz-Interaktionen. Ein CI < 1, =1, und > 1 beschreibt jeweils einen synergistischen, additiven oder antagonistischen Effekt. Die Visualisierung erfolgte durch Isobologramme.

In der Kombinationsbehandlung mit der Beispielverbindung 3 oder 11 oder 12 oder 13 oder 19 und Ibrutinib konnte im Vergleich zu Einfachbehandlungen eine deutliche Steigerung der NF-kB Signalwegsinhibition verzeichnet werden.

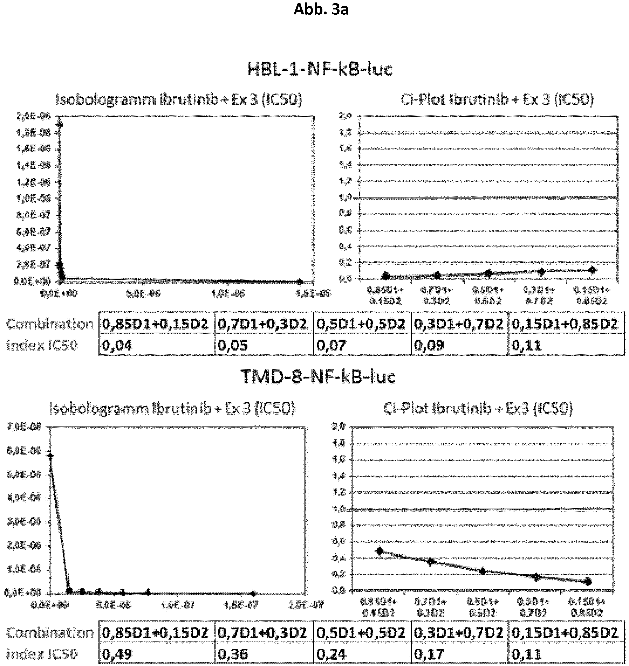

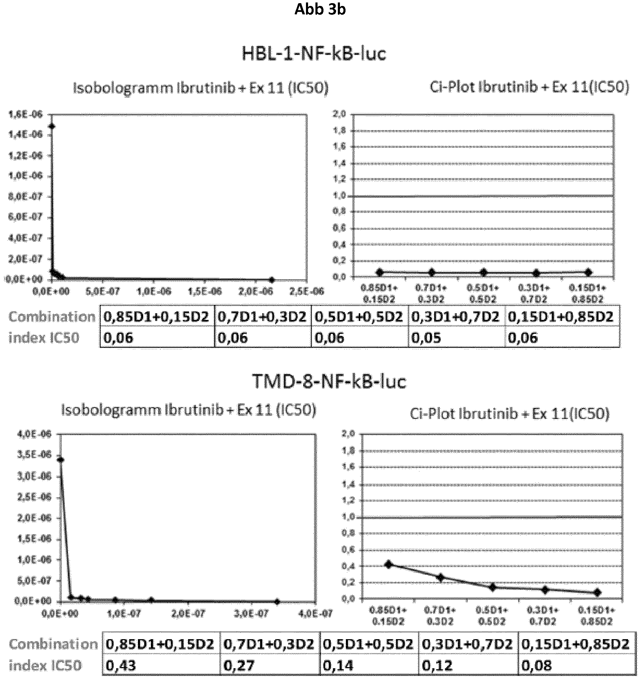

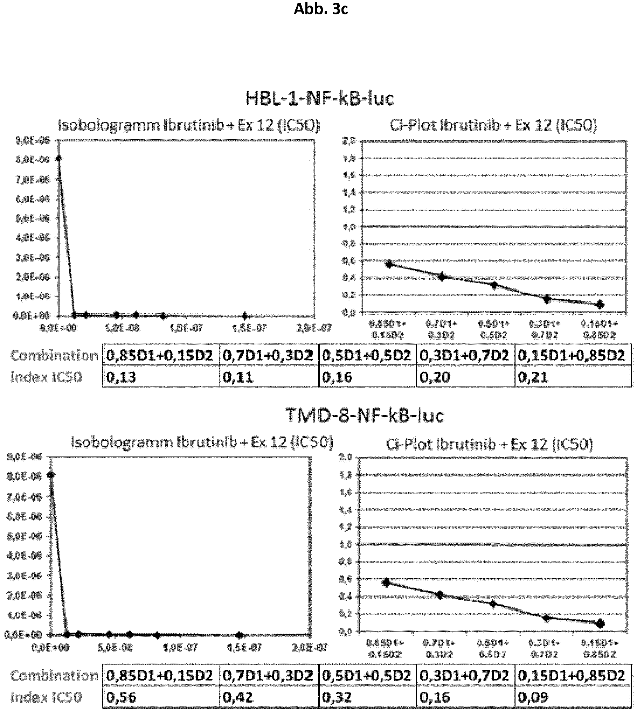

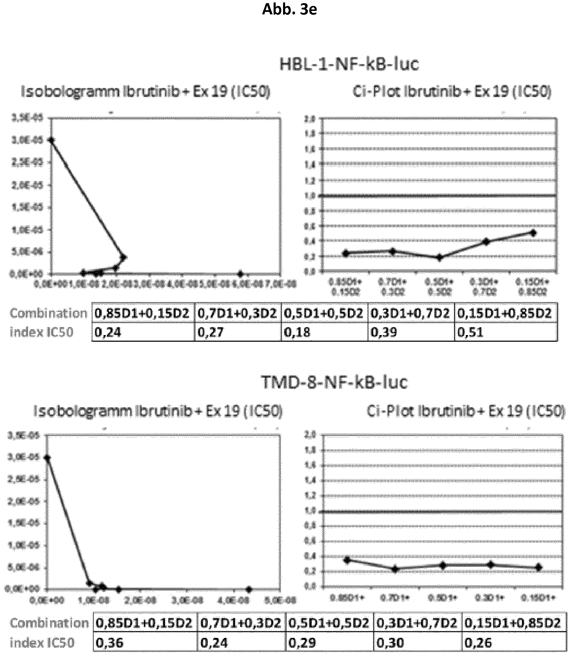

Abbildungen 3a bis 3e (Ex bedeutet Beispielverbindung): Ergebnisse einer NF-kB-Reporter Messung in ABC-DLBCL Zell-Linien TMD-8-NF-kB-luc und HBL-1-NF-kB-luc bei Kombination von Ibrutinib mit den Verbindungen der allgemeinen Formel (I) (Abb. 3a: Ex 03; Abb. 3b: Ex 11; Abb. 3c: Ex 12; Abb. 3d: Ex 13, Abb. 3e: Ex 19). IC50 Isobologramme für die verschiedenen Kombinationen mit den jeweiligen Konzentrationen der Substanz 1 (D1) (Beispielverbindung der allgemeinen Formel (I) als Bestandteil der erfindungsgemäßen Kombinationen) auf der y- und Substanz 2 (D2) (BTK Inhibitor Ibrutinib als Bestandteil der erfindungsgemäßen Kombinationen) auf der x-Achse sind dargestellt. Datenpunkte, die unter, auf oder über der Hypotenuse liegen indizieren jeweils synergistische, additive oder antagonistische Wirkung auf den NF-kB Signalweg.
Legende Abb. 3a: Effekt der Kombination von Ibrutinib mit Beispiel 03 auf NF-kB-Signalwegs-Aktivität in TMD-8 und HBL-1 Zellen.
Abb. 3b: Effekt der Kombination von Ibrutinib mit Beispiel 11 auf NF-kB-Signalwegs-Aktivität in TMD-8 und HBL-1 Zellen.
Abb. 3c: Effekt der Kombination von Ibrutinib mit Beispiel 12 auf NF-kB-Signalwegs-Aktivität in TMD-8 und HBL-1 Zellen.
Abb. 3d: Effekt der Kombination von Ibrutinib mit Beispiel 13 auf NF-kB-Signalwegs-Aktivität in TMD-8 und HBL-1 Zellen.
Abb. 3e: Effekt der Kombination von Ibrutinib mit Beispiel 19 auf NF-kB-Signalwegs-Aktivität in TMD-8 und HBL-1 Zellen.

### Ausführungsbeispiele für pharmazeutische Zusammensetzungen

Die Verbindungen der Formel (I) als Bestandteile der erfindungsgemäßen Kombinationen können folgendermaßen in pharmazeutische Zubereitungen überführt werden:

### Tablette:

### Zusammensetzung:

100 mg der Verbindung von Beispiel 11 oder der Verbindung von Beispiel 12, 50 mg Lactose (Monohydrat), 50 mg Maisstärke (nativ), 10 mg Polyvinylpyrolidon (PVP 25) (Fa. BASF, Ludwigshafen, Deutschland) und 2 mg Magnesiumstearat.

Tablettengewicht 212 mg. Durchmesser 8 mm, Wölbungsradius 12 mm.

### Herstellung:

Die Mischung aus einer Verbindung der Formel (I) als Bestandteil der erfindungsgemäßen Kombinationen, Lactose und Stärke wird mit einer 5%-igen Lösung (m/m) des PVPs in Wasser granuliert. Das Granulat wird nach dem Trocknen mit dem Magnesiumstearat 5 Minuten gemischt. Diese Mischung wird mit einer üblichen Tablettenpresse verpresst (Format der Tablette siehe oben). Als Richtwert für die Verpressung wird eine Presskraft von 15 kN verwendet.

### Oral applizierbare Suspension

### Zusammensetzung:

1000 mg der Verbindung von Beispiel 11 oder der Verbindung von Beispiel 12, 1000 mg Ethanol (96%), 400 mg Rhodigel^{®} (Xanthan gum der Firma FMC, Pennsylvania, USA) und 99 g Wasser.

Einer Einzeldosis von 100 mg der Verbindung entsprechen 10 ml orale Suspension.

### Herstellung:

Das Rhodigel wird in Ethanol suspendiert, die Verbindung wird der Suspension zugefügt. Unter Rühren erfolgt die Zugabe des Wassers. Bis zum Abschluß der Quellung des Rhodigels wird ca. 6h gerührt.

### Oral applizierbare Lösung:

### Zusammensetzung

500 mg der Verbindung von Beispiel 11 oder der Verbindung von Beispiel 12, 2.5 g Polysorbat und 97 g Polyethylenglycol 400. Einer Einzeldosis von 100 mg der Verbindung entsprechen 20 g orale Lösung.

### Herstellung

Die Verbindung wird in der Mischung aus Polyethylenglycol und Polysorbat unter Rühren suspendiert. Der Rührvorgang wird bis zur vollständigen Auflösung der Verbindung fortgesetzt.

## Patentansprüche

1. Eine pharmazeutische Kombination von :
• einer Komponente A, die IRAK4-inhibierende Verbindungen der allgemeinen Formel (I) sind: 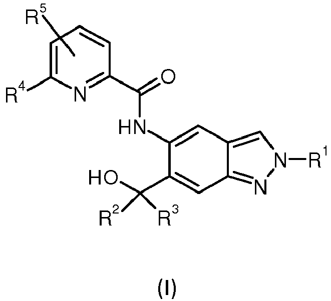 in der:
R¹ für C₁-C₆-Alkyl steht, wobei der C₁-C₆-Alkylrest unsubstituiert oder ein- oder mehrfach, gleich oder verschieden substituiert ist mit Halogen, Hydroxy, einem unsubstituierten oder ein- oder mehrfach mit Halogen substituierten C₃-C₆-Cycloalkyl, einem Rest R⁶, R⁷SO₂, R⁷SO oder R⁸O
oder für eine Gruppe steht, ausgewählt aus: 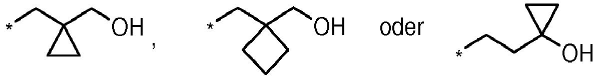
wobei * für die Verknüpfungsstelle der Gruppe mit dem Rest des Moleküls steht;
R² und R³ immer dieselbe Bedeutung haben und gleichzeitig entweder für Wasserstoff oder C₁-C₆-Alkyl stehen;
R⁴ für Halogen, Cyano, ein unsubstituiertes oder ein- oder mehrfach, gleich oder verschieden voneinander substituiertes C₁-C₆-Alkyl oder ein unsubstituiertes oder ein- oder mehrfach, gleich oder verschieden voneinander substituiertes C₃-C₆-Cycloalkyl steht, und die Substituenten ausgewählt sind aus der Gruppe Halogen und Hydroxy;
R⁵ für Wasserstoff, Halogen oder ein unsubstituiertes oder ein- oder mehrfach mit Halogen substituiertes C₁-C₆-Alkyl steht;
R⁶ für einen unsubstituierten oder ein- oder zweifach mit Methyl substituierten monocyclischen, gesättigten Heterocyclus mit 4 bis 6 Ringatomen steht, der ein Heteroatom oder eine Heterogruppe aus der Reihe O, S, SO oder SO₂ enthält;
R⁷ für C₁-C₆-Alkyl steht, wobei der C₁-C₆-Alkylrest unsubstituiert oder ein- oder mehrfach, gleich oder verschieden mit Halogen, Hydroxy oder C₃-C₆-Cycloalkyl substituiert ist; oder R⁷ steht für C₃-C₆-Cycloalkyl;
R⁸ für C₁-C₆-Alkyl steht, wobei der C₁-C₆-Alkylrest unsubstituiert oder ein- oder mehrfach, gleich oder verschieden mit Halogen substituiert ist;
oder ihre Diastereomere, Enantiomere, Metabolite, Salze, Solvate oder die Solvate ihrer Salze;
• einer Komponente B, die eine BTK-inhibierende Verbindung ist;
und, wahlweise,
• einer oder mehreren Komponenten C, die pharmazeutische Mittel sind;
in der gegebenenfalls eine oder beide der oben definierten Komponenten A und B in pharmazeutischen Formulierungen vorliegen, die zur simultanen, separaten oder sequenziellen Verabreichung bereit sind.

2. Die Kombination gemäß Anspruch 1, in der die Komponente A eine Verbindung der allgemeinen Formel (I) ist, wobei:
R¹ für C₁-C₆-Alkyl steht, wobei der C₁-C₆-Alkylrest unsubstituiert oder ein- oder mehrfach, gleich oder verschieden substituiert ist mit Fluor, Hydroxy, einem Rest R⁶**,** R⁷SO₂, R⁷SO oder R⁸O,
R² und R³ immer dieselbe Bedeutung haben und gleichzeitig entweder für Wasserstoff oder C₁-C₃-Alkyl stehen;
R⁴ für Halogen, Cyano oder C₁-C₃-Alkyl steht, wobei der C₁-C₃-Alkylrest unsubstituiert oder ein- oder mehrfach, gleich oder verschieden substituiert ist mit Halogen oder Hydroxy;
R⁵ für Wasserstoff, Fluor, Chlor oder C₁-C₃ Alkyl steht;
R⁶ für Oxetanyl oder Tetrahydrofuranyl steht;
R⁷ für C₁-C₄-Alkyl steht, wobei der C₁-C₄-Alkylrest unsubstituiert oder einfach mit Hydroxy oder mit Cyclopropyl oder mit drei Fluoratomen substituiert ist;
R⁸ für einen unsubstituierten C₁-C₄-Alkylrest oder einen dreifach mit Fluor substituierten C₁-C₄-Alkylrest steht.

3. Die Kombination gemäß Anspruch 1 oder 2, in der die Komponente A eine Verbindung der allgemeinen Formel (I) ist, wobei R⁴ für Difluormethyl, Trifluormethyl oder Methyl steht.

4. Die Kombination gemäß Anspruch 1, 2 oder 3, in der die Komponente A eine Verbindung der allgemeinen Formel (I) ist, wobei R⁵ Wasserstoff oder Fluor ist.

5. Die Kombination gemäß Anspruch 1, 2, 3 oder 4, in der die Komponente A eine Verbindung der allgemeinen Formel (I) ist, wobei R² und R³ gleichzeitig entweder Wasserstoff oder Methyl bedeuten.

6. Die Kombinationgemäß Anspruch 2, in der die Komponente A eine Verbindung der allgemeinen Formel (I) ist, wobei:
R¹ für C₂-C₆-Alkyl steht, wobei der C₂-C₆-Alkylrest unsubstituiert ist, oder der C₂-C₆-Alkylrest ein-, zwei- oder dreifach mit Fluor substituiert ist oder der C₂-C₆-Alkylrest einfach mit Hydroxy, R⁶, R⁷SO₂, oder R⁸O substituiert ist, oder R¹ für einen mit Oxetanyl subsituierter C₁-C₃ Alkylrest steht;
R² und R³ immer dieselbe Bedeutung haben und gleichzeitig entweder für Wasserstoff oder Methyl stehen;
R⁴ für einen unsubstituierten oder einen ein- oder mehrfach mit Halogen substituierten C₁-C₃-Alkylrest oder einen mit einer Hydroxygruppe substituierten C₁-C₃-Alkylrest oder einen mit einer Hydroxygruppe und drei Fluoratomen substituierten C₁-C₃-Alkylrest steht
R⁵ für Wasserstoff, Fluor oder C₁-C₃ Alkyl steht;
R⁷ für C₁-C₃-Alkyl steht;
R⁸ für C₁-C₄-Alkyl steht, wobei der C₁-C₄-Alkylrest unsubstituiert oder ein-, zwei- oder dreifach mit Fluor substituiert ist.

7. Die Kombination gemäß Anspruch 6, in der die Komponente A eine Verbindung der allgemeinen Formel (I) ist, worin :
R¹ für einen C₂-C₅-Alkylrest, der mit Hydroxy oder C₁-C₃-Alkoxy oder Trifluormethoxy oder 2,2,2-Trifluorethoxy oder Trifluormethyl substitutiert
ist oder
für einen mit Methyl-SO₂ substituierten C₂-C₄-Alkylrest oder für einen mit Oxetan-3-yl substituierter C₁-C₂ Alkylrest steht;
R² und R³ immer dieselbe Bedeutung haben und gleichzeitig für Wasserstoff oder Methyl stehen;
R⁴ für Methyl, Ethyl, Trifluor-C₁-C₃-alkyl, Difluor-C₁-C₃-Alkyl, Hydroxymethyl, 1-Hydroxyethyl, 2-Hydroxypropan-2-yl und 2,2,2-Trifluor-1-hydroxyethyl steht;
R⁵ für Wasserstoff, Fluor oder Methyl steht.

8. Die Kombination gemäß Anspruch 7, in der die Komponente A eine Verbindung der allgemeinen Formel (I) ist, worin :
R¹ für 4,4,4-Trifluorbutyl, 3-Hydroxy-3-methylbutyl, 3-Hydroxybutyl, 3-Methoxypropyl, 3-Hydroxypropyl, 3-Hydroxy-2-methylpropyl, 3-Hydroxy-2,2-dimethylpropyl, 3-Trifluormethoxypropyl, 2-Methoxyethyl, 2-Hydroxyethyl, 2-(Methylsulfonyl)ethyl oder 3-(Methylsulfonyl)propyl steht;
R² und R³ gleichzeitig für Methyl oder Wasserstoff stehen;
R⁴ für Difluormethyl, Trifluormethyl oder Methyl steht;
R⁵ für Wasserstoff oder Fluor steht.

9. Die Kombination gemäß Anspruch 8, in der die Komponente A eine Verbindung der allgemeinen Formel (I) ist, worin :
R¹ für 3-Hydroxy-3-methylbutyl, 3-Hydroxybutyl, 3-Hydroxy-2-methylpropyl, 3-Hydroxy-2,2-dimethylpropyl, 3-(Methylsulfonyl)propyl oder 2-(Methyl-sulfonyl)ethyl steht;
R² und R³ gleichzeitig für Methyl stehen;
R⁴ für Difluormethyl oder Trifluormethyl steht;
R⁵ für Wasserstoff steht.

10. Die Kombination gemäß Anspruch 8, in der die Komponente A eine Verbindung der allgemeinen Formel (I) ist, worin :
R¹ für 3-Hydroxy-3-methylbutyl, 3-Hydroxybutyl, 3-Hydroxy-2-methylpropyl, 3-Hydroxy-2,2-dimethylpropyl, 3-(Methylsulfonyl)propyl oder 2-(Methyl-sulfonyl)ethyl steht;
R² und R³ gleichzeitig für Methyl stehen;
R⁴ für Methyl steht;
R⁵ für Fluor steht, wobei sich R⁵ in ortho-Position zu R⁴ befindet.

11. Die Kombination gemäß einem der Ansprüche 1 bis 10, in der die Komponente A eine Verbindung der allgemeinen Formel (I) ist, nämlich:
1) N-[6-(2-Hydroxypropan-2-yl)-2-(2-methoxyethyl)-2H-indazol-5-yl]-6-(trifluormethyl)pyridin-2-carboxamid
2) N-[6-(Hydroxymethyl)-2-(2-methoxyethyl)-2H-indazol-5-yl]-6-(trifluormethyl)pyridin-2-carboxamid
3) N-[6-(2-Hydroxypropan-2-yl)-2-(3-methoxypropyl)-2H-indazol-5-yl]-6-(trifluormethyl)pyridin-2-carboxamid
4) N-[6-(Hydroxymethyl)-2-(3-methoxypropyl)-2H-indazol-5-yl]-6-(trifluormethyl)pyridin-2-carboxamid
5) N-[2-(2-Hydroxyethyl)-6-(2-hydroxypropan-2-yl)-2H-indazol-5-yl]-6-(trifluormethyl)pyridin-2-carboxamid
6) N-[6-(2-Hydroxypropan-2-yl)-2-(3-hydroxypropyl)-2H-indazol-5-yl]-6-(trifluormethyl)pyridin-2-carboxamid
7) N-[2-(2-Hydroxyethyl)-6-(hydroxymethyl)-2H-indazol-5-yl]-6-(trifluormethyl)pyridin-2-carboxamid
8) N-[6-(2-Hydroxypropan-2-yl)-2-(oxetan-3-ylmethyl)-2H-indazol-5-yl]-6-(trifluormethyl)pyridin-2-carboxamid
9) N-[6-(Hydroxymethyl)-2-(oxetan-3-ylmethyl)-2H-indazol-5-yl]-6-(trifluormethyl)pyridin-2-carboxamid
10) N-{6-(2-Hydroxypropan-2-yl)-2-[3-(methylsulfonyl)propyl]-2H-indazol-5-yl}-6-(trifluormethyl)pyridin-2-carboxamid
11) N-[2-(3-Hydroxy-3-methylbutyl)-6-(2-hydroxypropan-2-yl)-2H-indazol-5-yl]-6-(trifluormethyl)pyridin-2-carboxamid
12) N-{6-(2-Hydroxypropan-2-yl)-2-[2-(methylsulfonyl)ethyl]-2H-indazol-5-yl}-6-(trifluormethyl)pyridin-2-carboxamid
13) 6-(Difluormethyl)-N-[2-(3-hydroxy-3-methylbutyl)-6-(2-hydroxypropan-2-yl)-2H-indazol-5-yl]pyridin-2-carboxamid
14) 6-(Difluormethyl)-N-{6-(2-hydroxypropan-2-yl)-2-[2-(methylsulfonyl)ethyl]-2H-indazol-5-yl}pyridin-2-carboxamid
15) 6-(Difluormethyl)-N-[6-(2-hydroxypropan-2-yl)-2-(3-hydroxypropyl)-2H-indazol-5-yl]pyridin-2-carboxamid
16) N-[6-(2-Hydroxypropan-2-yl)-2-(4,4,4-trifluorbutyl)-2H-indazol-5-yl]-6-(trifluormethyl)pyridin-2-carboxamid
17) N-{6-(2-Hydroxypropan-2-yl)-2-[3-(trifluormethoxy)propyl]-2H-indazol-5-yl}-6-(trifluormethyl)pyridin-2-carboxamid
18) N-{6-(2-Hydroxypropan-2-yl)-2-[3-(2,2,2-trifluorethoxy)propyl]-2H-indazol-5-yl}-6-(trifluormethyl)pyridin-2-carboxamid
19) 5-Fluor-N-[2-(3-hydroxy-3-methylbutyl)-6-(2-hydroxypropan-2-yl)-2H-indazol-5-yl]-6-methylpyridin-2-carboxamid
20) N-[2-(3-Hydroxy-3-methylbutyl)-6-(2-hydroxypropan-2-yl)-2H-indazol-5-yl]-6-methylpyridin-2-carboxamid
21) 6-(2-Hydroxypropan-2-yl)-N-[6-(2-hydroxypropan-2-yl)-2-(4,4,4-trifluorbutyl)-2H-indazol-5-yl]pyridin-2-carboxamid.

12. Die Kombination gemäß einem der Ansprüche 1 bis 11, in der die Komponente B eine BTK-inhibierende Verbindung aus der Liste ausgewählt ist:
• Ibrutinib, oder ein pharmazeutisch verträgliches Salz davon;
• 4-tert-Butyl-N-[2-methyl-3-(4-methyl-6-{[4-(morpholin-4-ylcarbonyl)phenyl]amino}-5-oxo-4,5-dihydropyrazin-2-yl)phenyl]benzamid (CGI-1746, CAS 910232-84-7);
• N-{3-[(5-Fluor-2-{[4-(2-methoxyethoxy)phenyl]amino}pyrimidin-4-yl)amino]phenyl}acrylamid (AVL-292, CAS 1202757-89-8) ;
• 6-Cyclopropyl-8-fluor-2-[2-(hydroxymethyl)-3-(1-methyl-5-{[5-(4-methylpiperazin-1-yl)pyridin-2-yl]amino}-6-oxo-1,6-dihydropyridin-3-yl)phenyl]isochinolin-1(2H)-on (RN486, CAS 1242156-23-5);
• HM71224;
• N-{3-[6-({4-[(2R)-1,4-Dimethyl-3-oxopiperazin-2-yl]phenyl}amino)-4-methyl-5-oxo-4,5-dihydropyrazin-2-yl]-2-methylphenyl}-4,5,6,7-tetrahydro-1-benzothiophen-2-carboxamid (GDC-0834, CAS 1133432-50-4);
• 5-Amino-1-[(3R)-1-cyanpiperidin-3-yl]-3-[4-(2,4-difluorphenoxy)phenyl]-1H-pyrazol-4-carboxamid (PF-06250112, *J Immunol* 2013; 191:4540-4550) ;
• (2E)-4-(Dimethylamino)-N-{7-fluor-4-[(2-methylphenyl)amino]imidazo[1,5-a]chinoxalin-8-yl}-N-methylbut-2-enamid (CAS 1345250-62-5, *Bioorg. Med. Chem. Lett.* 21 (2011) 6258-6262) ;
• N-[3-(8-Anilinoimidazo[1,2-a]pyrazin-6-yl)phenyl]-4-tert-butylbenzamid (CGI-560, CAS 845269-74-1);
• 4-{4-[(4-{[3-(Acryloylamino)phenyl]amino}-5-fluorpyrimidin-2-yl)amino]phenoxy}-N-methylpyridin-2-carboxamid (CNX-774, CAS1202759-32-7);
• ONO-4059 (Athritis and rheumatism 2012, 64 Suppl 10:1660).

13. Die Kombination gemäß einem der Ansprüche 1 bis 12, in der die Komponente B, die ein BTK-Inhibitor ist, Ibrutinib ist, oder ein pharmazeutisch verträgliches Salz davon.

14. Die Kombination gemäß einem der Ansprüche 1 bis 13, in der die Komponente C ein pharmazeutisches Mittel aus der Liste ausgewählt ist:
131l-chTNT, Abarelix, Abirateron, Aclarubicin, Ado-Trastuzumab Emtansin, Afatinib, Aflibercept, Aldesleukin, Alemtuzumab, Alendronsäure, Alitretinoin, Altretamin, Amifostine, Aminoglutethimid, Hexyl-5-Aminolevulinat, Amrubicin, Amsacrin, Anastrozol, Ancestim, Anethole dithiolethione, Angiotensin II, Antithrombin III, Aprepitant, Arcitumomab, Arglabin, Arsentrioxid, Asparaginase, Axitinib, Azacitidin, Belotecan, Bendamustin, Belinostat, Bevacizumab, Bexaroten, Bicalutamid, Bisantren, Bleomycin, Bortezomib, Buserelin, Bosutinib, Brentuximab Vedotin, Busulfan, Cabazitaxel, Cabozantinib, Calciumfolinat, Calciumlevofolinat, Capecitabin, Capromab, Carboplatin, Carfilzomib, Carmofur, Carmustin, Catumaxomab, Celecoxib, Celmoleukin, Ceritinib, Cetuximab, Chlorambucil, Chlormadinon, Chlormethin, Cidofovir, Cinacalcet, Cisplatin, Cladribin, Clodronsäure, Clofarabin, Copanlisib(BAY 80-6946), Crisantaspase, Crizotinib, Cyclophosphamid, Cyproteron, Cytarabin, Dacarbazin, Dactinomycin, Dabrafenib, Dasatinib, Daunorubicin, Decitabin, Degarelix, Denileukin-Diftitox, Denosumab, Depreotid, Deslorelin, Dexrazoxane, Dibrospidiumchlorid, Dianhydrogalactitol, Diclofenac, Docetaxel, Dolasetron, Doxifluridin, Doxorubicin, Doxorubicin + Estron, Dronabinol, Edrecolomab, Elliptiniumacetat, Endostatin, Enocitabin, Enzalutamid, Epirubicin, Epitiostanol, Epoetin-alfa, Epoetin-beta, Epoetin-zeta, Eptaplatin, Eribulin, Erlotinib, Esomeprazol, Estramustin, Etoposid, Everolimus, Exemestan, Fadrozol, Fentanyl, Fluoxymesteron, Floxuridin, Fludarabin, Fluoruracil, Flutamid, Folinsäure, Formestan, Fosaprepitant, Fotemustin, Fulvestrant, Gadobutrol, Gadoteridol, Gadotersäure-Megluminsalz, Gadoversetamid, Gadoxetsäure Dinatriumsalz (Gd-EOB-DTPA Dinatriumsalz), Galliumnitrat, Ganirelix, Gefitinib, Gemcitabin, Gemtuzumab, Glucarpidase, Glutoxim, Goserelin, Granisetron, Granulocyten Kolonie stimulierender Factor (G-CSF), Granulocyten Macrophagen Kolonie stimulierender Factor (GM-CSF), Histamindihydrochlorid, Histrelin, Hydroxycarbamid, , I-125-Seeds, Ibandronsäure, Ibritumomab-Tiuxetan, Idarubicin, Ifosfamid, Imatinib, Imiquimod, Improsulfan, Indisetron, Incadronic acid, Ingenolmebutat, Interferon-alfa, Interferon-beta, Interferon-gamma, lobitridol, lobenguane (1231), lomeprol, Ipilimumab, Irinotecan, Itraconazole, Ixabepilon, Lanreotid, Lansoprazole, Lapatinib, Lasocholine, Lenalidomid, Lentinan, Letrozol, Leuprorelin, Levamisol, Levonorgestrel, Levothyroxin-Natrium, Lipegfilgrastim, Lisurid, Lobaplatin, Lomustin, Lonidamin, Masoprocol, Medroxyprogesteron, Megestrol, Melarsoprol, Melphalan, Mepitiostan, Mercaptopurin, Mesna, Methadon, Methotrexat, Methoxsalen, Methylaminolevulinat, Methylprednisolon, Methyltestosteron, Metirosin, Mifamurtid, Miltefosin, Miriplatin, Mitobronitol, Mitoguazon, Mitolactol, Mitomycin, Mitotan, Mitoxantron, Mogamulizumab, Molgramostim, Mopidamol, Morphinhydrochlorid, Morphinsulfat, Nabilon, Nabiximols, Nafarelin, Naloxon + Pentazocin, Naltrexon, Nartograstim, Nedaplatin, Nelarabin, Neridronsäure, Nivolumabpentetreotid, Nilotinib, Nilutamid, Nimorazol, Nimotuzumab, Nimustin, Nitracrin, Nivolumab, Obinutuzumab, Octreotid, Ofatumumab, Omacetaxin-Mepesuccinat, Omeprazol, Ondansetron, Orgotein, Orilotimod, Oxaliplatin, Oxycodon, Oxymetholon, Ozogamicin, p53-Gentherapie, Paclitaxel, Palladium-103-Seed, Palonosetron, Pamidronsäure, Panitumumab, Pantoprazol, Pazopanib, Pegaspargase, Pembrolizumab, Peg-interferon-alfa-2b, Pemetrexed, Pentostatin, Peplomycin, Perflubutane, Perfosfamid, Pertuzumab, Picibanil, Pilocarpin, Pirarubicin, Pixantron, Plerixafor, Plicamycin, Poliglusam, Polyestradiolphosphat, Polyvinylpyrrolidone + Natriumhyaluronat, Polysaccharid-K, Pomalidomid, Ponatinib, Porfimer-Natrium, Pralatrexat, Prednimustin, Prednison, Procarbazin, Procodazole, Propranolol, Quinagolid, Rabeprazol, Racotumomab, Radium-223-chlorid, Radotinib, Raloxifen, Raltitrexed, Ramosetron, Ramucirumab, Ranimustin, Rasburicase, Razoxan, RefametinibBAY 86-9766 (RDEA 119), Regorafenib, Risedronsäure, Rhenium-186 Etidronat, Rituximab, Romidepsin, Romurtid, Roniciclib (BAY 1000394), Samarium (153Sm) lexidronam, Satumomab, Secretin, Sipuleucel-T, Sizofiran, Sobuzoxan, Natriumglycididazol, Sorafenib, Stanozolol, Streptozocin, Sunitinib, Talaporfin, Tamibaroten, Tamoxifen, Tapentadol, Tasonermin, Teceleukin, Technetium (99mTc) Nofetumomab Merpentan, 99mTc-HYNIC-[Tyr3]-octreotid, Tegafur, Tegafur + Gimeracil + Oteracil, Temoporfin, Temozolomid, Temsirolimus, Teniposid, Testosteron, Tetrofosmin, Thalidomid, Thiotepa, Thymalfasin, Thyrotropin alfa, Tioguanin, Tocilizumab, Topotecan, Toremifen, Tositumomab, Trabectedin, Tramadol, Trastuzumab, Treosulfan, Tretinoin, Trifluridine + Tipiracil, Trametinib, Trilostan, Triptorelin, Trofosfamid, Thrombopoietin, Ubenimex, Valrubicin, Vandetanib, Vapreotid, Vatalanib (PTK/ZK), Vemurafenib, Vinblastin, Vincristin, Vindesin, Vinflunin, Vinorelbin, Vismodegib, Vorinostat, Yttrium-90-Glasmikrokugeln, Zinostatin, Zinostatin-Stimalamer, Zoledronsäure, Zorubicin.

15. Ein Kit bestehend aus einer Kombination von:
• einer Komponente A, die eine IRAK4-inhibierende Verbindung der allgemeinen Formel (I) gemäß einem der Ansprüche 1 bis 14 sind, oder ein Diastereomer, ein Enantiomer, ein Tautomer, ein N-Oxid, ein Metabolit, ein Salz, ein Solvat oder ein Solvat eines Salzes von dieser;
• einer Komponente B, die eine BTK-inhibierende Verbindung gemäß einem der Ansprüche 1 bis 14 ist;
und, wahlweise,
• einer oder mehreren Komponenten C, die ein pharmazeutisches Mittel gemäß einem der Ansprüche 1 bis 14 sind;
in der gegebenenfalls eine oder beide der oben definierten Komponenten A und B in pharmazeutischen Formulierungen vorliegen, die zur simultanen, separaten oder sequenziellen Verabreichung bereit sind.

16. Eine Kombination, wie in einem der Ansprüche 1 bis 14 definiert, zur Behandlung und/oder Prophylaxe von Krankheiten.

17. Eine Kombination, wie in einem der Ansprüche 1 bis 14 definiert, zur Verwendung in einem Verfahren zur Behandlung und/oder Prophylaxe von Tumorerkrankungen.

18. Eine Kombination, wie in einem der Ansprüche 1 bis 14 definiert, zur Verwendung in einem Verfahren zur Behandlung und/oder Prophylaxe von Non-Hodgkin-Lymphomen (abgekürzt als "NHL"), insbesondere Erstlinientherapie oder Zweitlinientherapie rezidiver oder refraktärer indolenter oder aggressiver Non-Hodgkin-Lymphome (NHL), insbesondere des Follikulären Lymphoms (abgekürzt als "FL"), der chronischen lymphatischen Leukämie (abgekürzt als "CLL"), des Marginalzonen-Lymphoms (abgekürzt als "MZL"), des Diffusen großzelligen B-Zell-Lymphoms (abgekürzt als "DLBCL"), insbesondere des aktivierten B-Zellen ähnlichen Diffusen großzelligen B-Zell-Lymphoms (abgekürzt als "ABC-DLBCL"), des Mantelzelllymphoms (abgekürzt als "MCL"), des transformierten Lymphoms (abgekürzt als "TL"), peripherer T-Zell-Lymphome (abgekürzt als "PTCL") oder der lymphoplasmazytischen Lymphome (Waldenströms Makroglobulinämie (abgekürzt als "WM")).

19. Eine Kombination, wie in einem der Ansprüche 1 bis 14 definiert, zur Verwendung in einem Verfahren zur Behandlung und/oder Prophylaxe von Lymphomen.

20. Verwendung einer Kombination, wie in einem der Ansprüche 1 bis 14 definiert, zur Herstellung eines Arzneimittels.

21. Verwendung gemäß Anspruch 20, wobei das Arzneimittel zur Behandlung und/oder Prophylaxe von Tumorerkrankungen verwendet wird.

22. Eine Kombination, wie in einem der Ansprüche 1 bis 14 definiert, zur Verwendung bei der Behandlung und/oder Prophylaxe von Lymphomen.

23. Arzneimittel enthaltend eine Kombination, wie in einem der Ansprüche 1 bis 14 definiert, in Kombination mit einem inerten, nicht-toxischen, pharmazeutisch geeigneten Hilfsstoff.

24. Verwendung gemäß Anspruch 20, wobei das Arzneimittel zur Behandlung und/oder Prophylaxe von Verwendung in einem Verfahren zur Behandlung und/oder Prophylaxe von Non-Hodgkin-Lymphomen (abgekürzt als "NHL"), insbesondere Erstlinientherapie oder Zweitlinientherapie rezidiver oder refraktärer indolenter oder aggressiver Non-Hodgkin-Lymphome (NHL), insbesondere des Follikulären Lymphoms (abgekürzt als "FL"), der chronischen lymphatischen Leukämie (abgekürzt als "CLL"), des Marginalzonen-Lymphoms (abgekürzt als "MZL"), des Diffusen großzelligen B-Zell-Lymphoms (abgekürzt als "DLBCL"), insbesondere des aktivierten B-Zellen ähnlichen Diffusen großzelligen B-Zell-Lymphoms (abgekürzt als "ABC-DLBCL"), des Mantelzelllymphoms (abgekürzt als "MCL"), des transformierten Lymphoms (abgekürzt als "TL"), peripherer T-Zell-Lymphome (abgekürzt als "PTCL") oder der lymphoplasmazytischen Lymphome (Waldenströms Makroglobulinämie (abgekürzt als "WM")) verwendet wird.

25. Verwendung gemäß Anspruch 20, wobei das Arzneimittel zur Behandlung und/oder Prophylaxe von Krankheiten verwendet wird, die durch unkontrolliertes Zellwachstum, Zellvermehrung und/oder Zellüberleben, eine unangemessene zelluläre Immunantwort oder eine unangemessene zelluläre inflammatorische Reaktion verursacht werden, insbesondere wenn die Krankheiten, die durch unkontrolliertes Zellwachstum, Zellvermehrung und/oder Zellüberleben, eine unangemessene zelluläre Immunantwort oder eine unangemessene zelluläre inflammatorische Reaktion verursacht werden, hämatologische Tumore, ein solider Tumor und/oder Metastasen davon, z.B. Leukämien und myelodysplastische Syndrome, maligne Lymphome, Kopf- und Genicktumore inklusive Gehirntumore und -metastasen, Tumore des Thorax inklusive nicht-kleinzellige und kleinzellige Lungentumore, Gastrointestinaltumore, endokrine Tumore, Mamma- und andere gynäkologische Tumore, urologische Tumore inklusive Nieren-, Blasen- und Prostatatumore, Hauttumore und Sarkome und/oder deren Metastasen sind.
